# EUROPEAN PATENT SPECIFICATION

(11) **EP 1 520 030 B1**
(45) Date of publication and mention of the grant of the patent: **26.10.2011**
(21) Application number: 02746888.3
(22) Date of filing: 03.07.2002
(51) Int. Cl.: C12N 9/10, C12N 9/04, C12P 13/02, C12N 15/03, C07C 235/12

(54) **MICROORGANISMS AND PROCESSES FOR ENHANCED PRODUCTION OF PANTOTHENATE**
MIKROORGANISMEN UND VERFAHREN ZUR VERBESSERTEN PRODUKTION VON PANTHOTHENAT
MICRO-ORGANISMES ET PROCEDES DE PRODUCTION AMELIOREE DE PANTOTHENATE

(43) Date of publication of application: 06.04.2005
(73) Proprietor: BASF SE, 67056 Ludwigshafen (DE)
(72) Inventor: YOCUM, R., Rogers, Lexington, MA 02420 (US); PATTERSON, Thomas, A., Attleboro, MA 02760 (US); PERO, Janice, G., Lexington, MA 02420 (US); HERMANN, Theron, Kinnelon, NJ 07405 (US)
(86) International application number: PCT/US2002/021336
(87) International publication number: WO 2004/005527

(56) References cited:
- EP-A- 1 390 519
- WO-A-02/057474
- WO-A-03/004672
- WO-A2-01/21772

### SEQUENCE LISTING

<110> OmniGene BioProducts, Inc. et al.
<120> MICROORGANISMS AND PROCESSES FOR ENHANCED PRODUCTION OF PANTOTHENATE
<130> BGI-154PC
<160> 31
<170> PatentIn Ver. 2.0
<210> 1
   <211> 194
   <212> DNA
   <213> Artificial Sequence
<220>
   <223> Description of Artificial Sequence:promoter sequence
<220>
   <221> -35_signal
   <222> (136)..(141)
<220>
   <221> -10_signal
   <222> (159)..(164)
<400> 1
   gctattgacg acagctatgg ttcactgtcc accaaccaaa actgtgctca gtaccgccaa 60
   tatttctccc ttgaggggta caaagaggtg tccctagaag agatccacgc tgtgtaaaaa 120
   ttttacaaaa aggtattgac tttccctaca gggtgtgtaa taatttaatt acaggcgggg 180
   gcaaccccgc ctgt                                                   194
<210> 2
   <211> 163
   <212> DNA
   <213> Artificial Sequence
<220>
   <223> Description of Artificial Sequence:promoter sequence
<220>
   <221> -35_signal
   <222> (113)..(118)
<220>
   <221> -10_signal
   <222> (136)..(141)
<400> 2
   gcctacctag cttccaagaa agatatccta acagcacaag agcggaaaga tgttttgttc 60
   tacatccaga acaacctctg ctaaaattcc tgaaaaattt tgcaaaaagt tgttgacttt 120
   atctacaagg tgtggtataa taatcttaac aacagcagga cgc                   163
<210> 3
   <211> 127
   <212> DNA
   <213> Artificial Sequence
<220>
   <223> Description of Artificial Sequence:promoter sequence
<220>
   <221> -35_signal
   <222> (34)..(39)
<220>
   <221> -10_signal
   <222> (58)..(63)
<220>
   <221> -35_signal
   <222> (75)..(80)
<220>
   <221> -10_signal
   <222> (98)..(103)
<400> 3
   gaggaatcat agaattttgt caaaataatt ttattgacaa cgtcttatta acgttgatat 60
   aatttaaatt ttatttgaca aaaatgggct cgtgttgtac aataaatgta gtgaggtgga 120
   tgcaatg                                                           127
<210> 4
   <211> 24
   <212> DNA
   <213> Artificial Sequence
<220>
   <223> Description of Artificial Sequence:ribosome binding site
<400> 4
   taaacatgag gaggagaaaa catg 24
<210> 5
   <211> 28
   <212> DNA
   <213> Artificial Sequence
<220>
   <223> Description of Artificial Sequence:ribosome binding site
<400> 5
   attcgagaaa tggagagaat ataatatg 28
<210> 6
   <211> 13
   <212> DNA
   <213> Artificial Sequence
<220>
   <223> Description of Artificial Sequence:ribosome binding site
<400> 6
   agaaaggagg tga 13
<210> 7
   <211> 23
   <212> DNA
   <213> Artificial Sequence
<220>
   <223> Description of Artificial Sequence:ribosome binding site
<220>
   <221> misc_feature
   <222> 17, 18, 19, 20
   <223> n = a, t, c, or g
<400> 7
   ttaagaaagg aggtgannnn atg 23
<210> 8
   <211> 23
   <212> DNA
   <213> Artificial Sequence
<220>
   <223> Description of Artificial Sequence:ribosome binding site
<220>
   <221> misc_feature
   <222> 16, 17, 18, 19, 20
   <223> n = a, c, t, or g
<400> 8
   ttagaaagga ggtgannnnn atg 23
<210> 9
   <211> 23
   <212> DNA
   <213> Artificial Sequence
<220>
   <223> Description of Artificial Sequence:ribosome binding site
<220>
   <221> misc_feature
   <222> 14, 15, 16, 17, 18, 19, 20
   <223> n = a, c, t, or g
<400> 9
   agaaaggagg tgannnnnnn atg 23
<210> 10
   <211> 22
   <212> DNA
   <213> Artificial Sequence
<220>
   <223> Description of Artificial Sequence:ribosome binding site
<220>
   <221> misc_feature
   <222> 14, 15, 16, 17, 18, 19
   <223> n = a, c, t, or g
<400> 10
   agaaaggagg tgannnnnna tg 22
<210> 11
   <211> 25
   <212> DNA
   <213> Artificial Sequence
<220>
   <223> Description of Artificial Sequence:ribosome binding site
<400> 11
   ccctctagaa ggaggagaaa acatg 25
<210> 12
   <211> 24
   <212> DNA
   <213> Artificial Sequence
<220>
   <223> Description of Artificial Sequence:ribosome binding site
<400> 12
   ccctctagag gaggagaaaa catg 24
<210> 13
   <211> 23
   <212> DNA
   <213> Artificial Sequence
<220>
   <223> Description of Artificial Sequence:ribosome binding site
<400> 13
   ttagaaagga ggatttaaat atg 23
<210> 14
   <211> 23
   <212> DNA
   <213> Artificial Sequence
<220>
   <223> Description of Artificial Sequence:ribosome binding site
<400> 14
   ttagaaagga ggtttaatta atg 23
<210> 15
   <211> 23
   <212> DNA
   <213> Artificial Sequence
<220>
   <223> Description of Artificial Sequence:ribosome binding site
<400> 15
   ttagaaagga ggtgatttaa atg 23
<210> 16
   <211> 23
   <212> DNA
   <213> Artificial Sequence
<220>
   <223> Description of Artificial Sequence:ribosome binding site
<400> 16
   ttagaaagga ggtgtttaaa atg 23
<210> 17
   <211> 28
   <212> DNA
   <213> Artificial Sequence
<220>
   <223> Description of Artificial Sequence:ribosome binding site
<400> 17
   attcgagaaa ggaggtgaat ataatatg 28
<210> 18
   <211> 27
   <212> DNA
   <213> Artificial Sequence
<220>
   <223> Description of Artificial Sequence:ribosome binding site
<400> 18
   attcgagaaa ggaggtgaat aataatg 27
<210> 19
   <211> 28
   <212> DNA
   <213> Artificial Sequence
<220>
   <223> Description of Artificial Sequence:ribosome binding site
<400> 19
   attcgtagaa aggaggtgaa ttaatatg 28
<210> 20
   <211> 51
   <212> DNA
   <213> Artificial Sequence
<220>
   <223> Description of Artificial Sequence:5' PCR primer
   <223> for serA gene
<400> 20
   ccctctagag gaggagaaaa catgtttcga gtattggtct cagacaaaat g 51
<210> 21
   <211> 43
   <212> DNA
   <213> Artificial Sequence
<220>
   <223> Description of Artificial Sequence:3' PCR primer
   <223> for serA gene
<400> 21
   cccggatcca attatggcag atcaatgagc ttcacagaca caa 43
<210> 22
   <211> 48
   <212> DNA
   <213> Artificial Sequence
<220>
   <223> Description of Artificial Sequence:5' PCR primer
   <223> for glyA gene
<400> 22
   ggatctagag gaggtgtaaa catgaaacat ttacctgcgc aagacgaa 48
<210> 23
   <211> 43
   <212> DNA
   <213> Artificial Sequence
<220>
   <223> Description of Artificial Sequence:3' PCR primer
   <223> for glyA gene
<400> 23
   cggggatccc ccatcaacaa ttacacactt ctattgattc tac 43
<210> 24
   <211> 7926
   <212> DNA
   <213> Artificial Sequence
<220>
   <223> Description of Artificial Sequence:serA overexpression
   <223> plasmid
<400> 24
   gaattttgcg gccgcttcga aagctgtaat ataaaaacct tcttcaacta acggggcagg 60
   ttagtgacat tagaaaaccg actgtaaaaa gtacagtcgg cattatctca tattataaaa 120
   gccagtcatt aggcctatct gacaattcct gaatagagtt cataaacaat cctgcatgat 180
   aaccatcaca aacagaatga tgtacctgta aagatagcgg taaatatatt gaattacctt 240
   tattaatgaa ttttcctgct gtaataatgg gtagaaggta attactatta ttattgatat 300
   ttaagttaaa cccagtaaat gaagtccatg gaataataga aagagaaaaa gcattttcag 360
   gtataggtgt tttgggaaac aatttccccg aaccattata tttctctaca tcagaaaggt 420
   ataaatcata aaactctttg aagtcattct ttacaggagt ccaaatacca gagaatgttt 480
   tagatacacc atcaaaaatt gtataaagtg gctctaactt atcccaataa cctaactctc 540
   cgtcgctatt gtaaccagtt ctaaaagctg tatttgagtt tatcaccctt gtcactaaga 600
   aaataaatgc agggtaaaat ttatatcctt cttgttttat gtttcggtat aaaacactaa 660
   tatcaatttc tgtggttata ctaaaagtcg tttgttggtt caaataatga ttaaatatct 720
   cttttctctt ccaattgtct aaatcaattt tattaaagtt catttgatat gcctcctaaa 780
   tttttatcta aagtgaattt aggaggctta cttgtctgct ttcttcatta gaatcaatcc 840
   ttttttaaaa gtcaatatta ctgtaacata aatatatatt ttaaaaatat cccactttat 900
   ccaattttcg tttgttgaac taatgggtgc tttagttgaa gaataaagac cacattaaaa 960
   aatgtggtct tttgtgtttt tttaaaggat ttgagcgtag cgaaaaatcc ttttctttct 1020
   tatcttgata ataagggtaa ctattgaatt cggtaccaag agtttgtaga aacgcaaaaa 1080
   ggccatccgt caggatggcc ttctgcttaa tttgatgcct ggcagtttat ggcgggcgtc 1140
   ctgcccgcca ccctccgggc cgttgcttcg caacgttcaa atccgctccc ggcggatttg 1200
   tcctactcag gagagcgttc accgacaaac aacagataaa acgaaaggcc cagtctttcg 1260
   actgagcctt tcgttttatt tgatgcctgg cagttcccta ctctcgcatg gggagacccc 1320
   acactaccat cggcgctacg gcgtttcact tctgagttcg gcatggggtc aggtgggacc 1380
   accgcgctac tgccgccagg caaattctgt tttatcagac cgcttctgcg ttctgattta 1440
   atctgtatca ggctgaaaat cttctctcat ccgccaaaac aggatccaat tatggcagat 1500
   caatgagctt cacagacaca atatcaggga catttgttag ttctttcaca attttatctt 1560
   ccagatgtct gtcaaaggaa agcatcatga tggcttctcc gcctttttcc ttacggccaa 1620
   cctgcatagt tgcaatgtta atatcattat ctccgagaat acgtcctact cggccgatga 1680
   cacctgttgt atcttgatgc tggatataca ccaagtgacc agtcggataa aaatcaatat 1740
   taaatccatt gatctcgaca attcgttctc cgaaatgagg aatatacgta gccgttacag 1800
   taaaggtgct gcggtctcct gtcactttta cgctgatgca gttatcgtat ccagattcag 1860
   aagaggaaat tttttcactg aagctaatgc cgcgttcttt tgcgacaccc ccggcattga 1920
   cctcattaac agtagagtct acgcgcggtt ttaaaaagcc tgacagaagg gcttttgtaa 1980
   tgaacgatgt ttcaagttta gcaattgtgc cttcatattg aatggcaaca tcctgtactg 2040
   gttctttcat gcactgtgat acaaggctgc caatttttcc tgcaatttga tggtaaggct 2100
   taattttagc aaattcatct tttgtcatgg caggcaggtt gatagctgac atgacaggca 2160
   ggccttttgc gaactgcaga acttcttctg acacttgggc ggcgacattg agctgtgctt 2220
   ctttcgttga tgctcccaag tgaggagtgg caatgactaa tggatgatca acaagtttgt 2280
   tgtcaactgg cggttcgact tcgaaaacgt caagcgctgc tcccgcaaca tgcccgtttt 2340
   ccaaagcttc gagaagtgct gcttcatcga taattccgcc tcgcgcacag ttaattaagc 2400
   gaacgccttt tttcgttttt gcaatcgttt ctttattcaa taagcctttt gtttcttttg 2460
   ttaaaggcgt gtgaacggta atgatatccg cactttcaag cacttcttca aatgtacggc 2520
   tgtttacgcc gatttttttc gctctttctt ccgttaagaa aggatcaaaa acgtgcacag 2580
   tcataccgaa cgctcctcga cgctgtgcaa tttcacttcc gattcggcct aatcctacaa 2640
   taccaagcgt ttttccataa agctctgaac cgacataagc tgtgcggttc cactctctgg 2700
   atttcactga gatattagcc tgcggaatgt gtctcattaa agaagagatc attgcaaatg 2760
   tatgctcagc tgtcgaaatg gtgttgccgt tcggagcatt gatcacgatt accccgtgtt 2820
   tcgtagcctc atcaatatcg atattatcga caccgacacc ggctcttccg acaattttta 2880
   aagaagtcat tttgttgaaa aggtcttctg ttacttttgt cgcgcttcgc accaaaagag 2940
   catcaaaagt atgtaattca tcttctgcat ctgctacgtt tttttgaacg atttcaataa 3000
   agtctgattc aataagtggc tgtaaaccgt cgttgctcat tttgtctgag accaatactc 3060
   gaaacatgtt ttctcctcct ctagagcgtc ctgctgttgt taagattatt ataccacacc 3120
   ttgtagataa agtcaacaac tttttgcaaa atttttcagg aattttagca gaggttgttc 3180
   tggatgtaga acaaaacatc tttccgctct tgtgctgtta ggatatcttt cttggaagct 3240
   aggtaggcct cgagttatgg cagttggtta aaaggaaaca aaaagaccgt tttcacacaa 3300
   aacggtcttt ttcgatttct ttttacagtc acagccactt ttgcaaaaac cggacagctt 3360
   catgccttat aactgctgtt tcggtcgaca agcttcgcga agcggccgca aaattcactg 3420
   gccgtcgttt tacaacgtcg tgactgggaa aaccctggcg ttacccaact taatcgcctt 3480
   gcagcacatc ccccctttcg cagctggcgt aatagcgaag aggcccgcac cgatcgccct 3540
   tcccaacagt tgcgcagcct gaatggcgaa tggcgcctga tgcggtattt tctccttacg 3600
   catctgtgcg gtatttcaca ccgcatatgg tgcactctca gtacaatctg ctctgatgcc 3660
   gcatagttaa gccagccccg acacccgcca acacccgctg actatgcttg taaaccgttt 3720
   tgtgaaaaaa tttttaaaat aaaaaagggg acctctaggg tccccaatta attagtaata 3780
   taatctatta aaggtcattc aaaaggtcat ccaccggatc agcttagtaa agccctcgct 3840
   agattttaat gcggatgttg cgattacttc gccaactatt gcgataacaa gaaaaagcca 3900
   gcctttcatg atatatctcc caatttgtgt agggcttatt atgcacgctt aaaaataata 3960
   aaagcagact tgacctgata gtttggctgt gagcaattat gtgcttagtg catctaacgc 4020
   ttgagttaag ccgcgccgcg aagcggcgtc ggcttgaacg aattgttaga cattatttgc 4080
   cgactacctt ggtgatctcg cctttcacgt agtggacaaa ttcttccaac tgatctgcgc 4140
   gcgaggccaa gcgatcttct tcttgtccaa gataagcctg tctagcttca agtatgacgg 4200
   gctgatactg ggccggcagg cgctccattg cccagtcggc agcgacatcc ttcggcgcga 4260
   ttttgccggt tactgcgctg taccaaatgc gggacaacgt aagcactaca tttcgctcat 4320
   cgccagccca gtcgggcggc gagttccata gcgttaaggt ttcatttagc gcctcaaata 4380
   gatcctgttc aggaaccgga tcaaagagtt cctccgccgc tggacctacc aaggcaacgc 4440
   tatgttctct tgcttttgtc agcaagatag ccagatcaat gtcgatcgtg gctggctcga 4500
   agatacctgc aagaatgtca ttgcgctgcc attctccaaa ttgcagttcg cgcttagctg 4560
   gataacgcca cggaatgatg tcgtcgtgca caacaatggt gacttctaca gcgcggagaa 4620
   tctcgctctc tccaggggaa gccgaagttt ccaaaaggtc gttgatcaaa gctcgccgcg 4680
   ttgtttcatc aagccttacg gtcaccgtaa ccagcaaatc aatatcactg tgtggcttca 4740
   ggccgccatc cactgcggag ccgtacaaat gtacggccag caacgtcggt tcgagatggc 4800
   gctcgatgac gccaactacc tctgatagtt gagtcgatac ttcggcgatc accgcttccc 4860
   tcatgatgtt taactttgtt ttagggcgac tgccctgctg cgtaacatcg ttgctgctcc 4920
   ataacatcaa acatcgaccc acggcgtaac gcgcttgctg cttggatgcc cgaggcatag 4980
   actgtacccc aaaaaaacag tcataacaag ccatgaaaac cgccactgcg ccgttaccac 5040
   cgctgcgttc ggtcaaggtt ctggaccagt tgcgtgagcg catacgctac ttgcattaca 5100
   gcttacgaac cgaacaggct tatgtccact gggttcgtgc cttcatccgt ttccacggtg 5160
   tgcgtcaccc ggcaaccttg ggcagcagcg aagtcgaggc atttctgtcc tggctggcga 5220
   acgagcgcaa ggtttcggtc tccacgcatc gtcaggcatt ggcggccttg ctgttcttct 5280
   acggcaaggt gctgtgcacg gatctgccct ggcttcagga gatcggaaga cctcggccgt 5340
   cgcggcgctt gccggtggtg ctgaccccgg atgaagtggt tcgcatcctc ggttttctgg 5400
   aaggcgagca tcgtttgttc gcccagcttc tgtatggaac gggcatgcgg atcagtgagg 5460
   gtttgcaact gcgggtcaag gatctggatt tcgatcacgg cacgatcatc gtgcgggagg 5520
   gcaagggctc caaggatcgg gccttgatgt tacccgagag cttggcaccc agcctgcgcg 5580
   agcaggggaa ttgatccggt ggatgacctt ttgaatgacc tttaatagat tatattacta 5640
   attaattggg gaccctagag gtcccctttt ttattttaaa aattttttca caaaacggtt 5700
   tacaagcata acgggttttg ctgcccgcaa acgggctgtt ctggtgttgc tagtttgtta 5760
   tcagaatcgc agatccggct tcaggtttgc cggctgaaag cgctatttct tccagaattg 5820
   ccatgatttt ttccccacgg gaggcgtcac tggctcccgt gttgtcggca gctttgattc 5880
   gataagcagc atcgcctgtt tcaggctgtc tatgtgtgac tgttgagctg taacaagttg 5940
   tctcaggtgt tcaatttcat gttctagttg ctttgtttta ctggtttcac ctgttctatt 6000
   aggtgttaca tgctgttcat ctgttacatt gtcgatctgt tcatggtgaa cagctttaaa 6060
   tgcaccaaaa actcgtaaaa gctctgatgt atctatcttt tttacaccgt tttcatctgt 6120
   gcatatggac agttttccct ttgatatcta acggtgaaca gttgttctac ttttgtttgt 6180
   tagtcttgat gcttcactga tagatacaag agccataaga acctcagatc cttccgtatt 6240
   tagccagtat gttctctagt gtggttcgtt gtttttgcgt gagccatgag aacgaaccat 6300
   tgagatcatg cttactttgc atgtcactca aaaattttgc ctcaaaactg gtgagctgaa 6360
   tttttgcagt taaagcatcg tgtagtgttt ttcttagtcc gttacgtagg taggaatctg 6420
   atgtaatggt tgttggtatt ttgtcaccat tcatttttat ctggttgttc tcaagttcgg 6480
   ttacgagatc catttgtcta tctagttcaa cttggaaaat caacgtatca gtcgggcggc 6540
   ctcgcttatc aaccaccaat ttcatattgc tgtaagtgtt taaatcttta cttattggtt 6600
   tcaaaaccca ttggttaagc cttttaaact catggtagtt attttcaagc attaacatga 6660
   acttaaattc atcaaggcta atctctatat ttgccttgtg agttttcttt tgtgttagtt 6720
   cttttaataa ccactcataa atcctcatag agtatttgtt ttcaaaagac ttaacatgtt 6780
   ccagattata ttttatgaat ttttttaact ggaaaagata aggcaatatc tcttcactaa 6840
   aaactaattc taatttttcg cttgagaact tggcatagtt tgtccactgg aaaatctcaa 6900
   agcctttaac caaaggattc ctgatttcca cagttctcgt catcagctct ctggttgctt 6960
   tagctaatac accataagca ttttccctac tgatgttcat catctgagcg tattggttat 7020
   aagtgaacga taccgtccgt tctttccttg tagggttttc aatcgtgggg ttgagtagtg 7080
   ccacacagca taaaattagc ttggtttcat gctccgttaa gtcatagcga ctaatcgcta 7140
   gttcatttgc tttgaaaaca actaattcag acatacatct caattggtct aggtgatttt 7200
   aatcactata ccaattgaga tgggctagtc aatgataatt actagtcctt ttcctttgag 7260
   ttgtgggtat ctgtaaattc tgctagacct ttgctggaaa acttgtaaat tctgctagac 7320
   cctctgtaaa ttccgctaga cctttgtgtg ttttttttgt ttatattcaa gtggttataa 7380
   tttatagaat aaagaaagaa taaaaaaaga taaaaagaat agatcccagc cctgtgtata 7440
   actcactact ttagtcagtt ccgcagtatt acaaaaggat gtcgcaaacg ctgtttgctc 7500
   ctctacaaaa cagaccttaa aaccctaaag gcttaagtag caccctcgca agctcgggca 7560
   aatcgctgaa tattcctttt gtctccgacc atcaggcacc tgagtcgctg tctttttcgt 7620
   gacattcagt tcgctgcgct cacggctctg gcagtgaatg ggggtaaatg gcactacagg 7680
   cgccttttat ggattcatgc aaggaaacta cccataatac aagaaaagcc cgtcacgggc 7740
   ttctcagggc gttttatggc gggtctgcta tgtggtgcta tctgactttt tgctgttcag 7800
   cagttcctgc cctctgattt tccagtctga ccacttcgga ttatcccgtg acaggtcatt 7860
   cagactggct aatgcaccca gtaaggcagc ggtatcatca acaggcttac ccgtcttact 7920
   gtcaac                                                            7926
<210> 25
   <211> 7701
   <212> DNA
   <213> Artificial Sequence
<220>
   <223> Description of Artificial Sequence:glyA overexpression
   <223> plasmid
<400> 25
   gaattttgcg gccgcttcga aagctgtaat ataaaaacct tcttcaacta acggggcagg 60
   ttagtgacat tagaaaaccg actgtaaaaa gtacagtcgg cattatctca tattataaaa 120
   gccagtcatt aggcctatct gacaattcct gaatagagtt cataaacaat cctgcatgat 180
   aaccatcaca aacagaatga tgtacctgta aagatagcgg taaatatatt gaattacctt 240
   tattaatgaa ttttcctgct gtaataatgg gtagaaggta attactatta ttattgatat 300
   ttaagttaaa cccagtaaat gaagtccatg gaataataga aagagaaaaa gcattttcag 360
   gtataggtgt tttgggaaac aatttccccg aaccattata tttctctaca tcagaaaggt 420
   ataaatcata aaactctttg aagtcattct ttacaggagt ccaaatacca gagaatgttt 480
   tagatacacc atcaaaaatt gtataaagtg gctctaactt atcccaataa cctaactctc 540
   cgtcgctatt gtaaccagtt ctaaaagctg tatttgagtt tatcaccctt gtcactaaga 600
   aaataaatgc agggtaaaat ttatatcctt cttgttttat gtttcggtat aaaacactaa 660
   tatcaatttc tgtggttata ctaaaagtcg tttgttggtt caaataatga ttaaatatct 720
   cttttctctt ccaattgtct aaatcaattt tattaaagtt catttgatat gcctcctaaa 780
   tttttatcta aagtgaattt aggaggctta cttgtctgct ttcttcatta gaatcaatcc 840
   ttttttaaaa gtcaatatta ctgtaacata aatatatatt ttaaaaatat cccactttat 900
   ccaattttcg tttgttgaac taatgggtgc tttagttgaa gaataaagac cacattaaaa 960
   aatgtggtct tttgtgtttt tttaaaggat ttgagcgtag cgaaaaatcc ttttctttct 1020
   tatcttgata ataagggtaa ctattgaatt cggtaccaag agtttgtaga aacgcaaaaa 1080
   ggccatccgt caggatggcc ttctgcttaa tttgatgcct ggcagtttat ggcgggcgtc 1140
   ctgcccgcca ccctccgggc cgttgcttcg caacgttcaa atccgctccc ggcggatttg 1200
   tcctactcag gagagcgttc accgacaaac aacagataaa acgaaaggcc cagtctttcg 1260
   actgagcctt tcgttttatt tgatgcctgg cagttcccta ctctcgcatg gggagacccc 1320
   acactaccat cggcgctacg gcgtttcact tctgagttcg gcatggggtc aggtgggacc 1380
   accgcgctac tgccgccagg caaattctgt tttatcagac cgcttctgcg ttctgattta 1440
   atctgtatca ggctgaaaat cttctctcat ccgccaaaac aggatccccc atcaacaatt 1500
   acacacttct attgattcta caaaaaaaga cattgagttt caagaacatc gtcaaaaaac 1560
   ccgccgggca taagcccaag cgggttttag gatcttaata atctaattct ttatataaag 1620
   gaaatttatc agtcagagca gctacacgct gtcttgcttc ttcaagtttt ccttcatctt 1680
   cgtggttttt caatgcaagc gcaatgatag caccgacttc ttctaatgcg tctccgtcaa 1740
   aaccgcggct ggttacagca gctgtaccaa gacggatgcc gcttgttacg aaaggttttt 1800
   caggatcata tggaatcgcg tttttgttag acgtaatacc aatttcatca agtacatgct 1860
   ccgcaacctt accagtcagt ccgagcgaac gaaggtcaac aaggataagg tggttgtctg 1920
   ttccgcctga aacgagctgg atgccctctt tcgttaaggc ttcagccaga cgtttcgcgt 1980
   ttgaaatgac gttttgtgca tatgttttga aatcgtcctg caatacttca ccgaatgaaa 2040
   cagcttttgc ggcaataacg tgcatcagag ggccgccttg aattccaggg aagatcgatt 2100
   tatcaatttt cttgccaaac tcttcacggc aaaggatcat accgccgcga ggaccgcgaa 2160
   gtgttttatg tgttgttgtt gtaacgaaat cagcgtaagg aaccgggttt ggatgaaggc 2220
   ctgccgcaac aagtcctgcg atatgtgcca tatccaccat gaagtaagcg ccgacttcat 2280
   cagcaatttc acggaatttc ttaaagtcga ttgtacgagg atacgcactt gctcctgcta 2340
   cgataagctt cggtttatga gcgagggctt tttcacgcac gtcatcgtaa tcaatatatt 2400
   gagtttcttt atctacgccg tactcaacaa agttatattg aacaccgctg aagttgactg 2460
   ggcttccgtg tgttaaatgg ccgccgtggg agaggttcat cccaagtaca gtatcgcctt 2520
   gctccaaaat cgtgaagtac actgccatgt ttgcttgtgc gcctgaatga ggctgaacgt 2580
   ttacatgctc cgctccaaag atttccttcg cgcggtcacg ggcgatatct tcaacgacat 2640
   cgacgtgctc gcatccgccg tagtagcgtt tgcccggata tccttctgcg tacttatttg 2700
   tcaaaacaga tccttgtgct tccataaccg cttcacttac aaagttctca gaagcaatca 2760
   attcgatctt agtctgttgg cgttcacgct catttttaat ggcgttaaac acttgttcgt 2820
   cttgcgcagg taaatgtttc atgtttacac ctcctctaga gcgtcctgct gttgttaaga 2880
   ttattatacc acaccttgta gataaagtca acaacttttt gcaaaatttt tcaggaattt 2940
   tagcagaggt tgttctggat gtagaacaaa acatctttcc gctcttgtgc tgttaggata 3000
   tctttcttgg aagctaggta ggcctcgagt tatggcagtt ggttaaaagg aaacaaaaag 3060
   accgttttca cacaaaacgg tctttttcga tttcttttta cagtcacagc cacttttgca 3120
   aaaaccggac agcttcatgc cttataactg ctgtttcggt cgacaagctt cgcgaagcgg 3180
   ccgcaaaatt cactggccgt cgttttacaa cgtcgtgact gggaaaaccc tggcgttacc 3240
   caacttaatc gccttgcagc acatccccct ttcgccagct ggcgtaatag cgaagaggcc 3300
   cgcaccgatc gcccttccca acagttgcgc agcctgaatg gcgaatggcg cctgatgcgg 3360
   tattttctcc ttacgcatct gtgcggtatt tcacaccgca tatggtgcac tctcagtaca 3420
   atctgctctg atgccgcata gttaagccag ccccgacacc cgccaacacc cgctgactat 3480
   gcttgtaaac cgttttgtga aaaaattttt aaaataaaaa aggggacctc tagggtcccc 3540
   aattaattag taatataatc tattaaaggt cattcaaaag gtcatccacc ggatcagctt 3600
   agtaaagccc tcgctagatt ttaatgcgga tgttgcgatt acttcgccaa ctattgcgat 3660
   aacaagaaaa agccagcctt tcatgatata tctcccaatt tgtgtagggc ttattatgca 3720
   cgcttaaaaa taataaaagc agacttgacc tgatagtttg gctgtgagca attatgtgct 3780
   tagtgcatct aacgcttgag ttaagccgcg ccgcgaagcg gcgtcggctt gaacgaattg 3840
   ttagacatta tttgccgact accttggtga tctcgccttt cacgtagtgg acaaattctt 3900
   ccaactgatc tgcgcgcgag gccaagcgat cttcttcttg tccaagataa gcctgtctag 3960
   cttcaagtat gacgggctga tactgggccg gcaggcgctc cattgcccag tcggcagcga 4020
   catccttcgg cgcgattttg ccggttactg cgctgtacca aatgcgggac aacgtaagca 4080
   ctacatttcg ctcatcgcca gcccagtcgg gcggcgagtt ccatagcgtt aaggtttcat 4140
   ttagcgcctc aaatagatcc tgttcaggaa ccggatcaaa gagttcctcc gccgctggac 4200
   ctaccaaggc aacgctatgt tctcttgctt ttgtcagcaa gatagccaga tcaatgtcga 4260
   tcgtggctgg ctcgaagata cctgcaagaa tgtcattgcg ctgccattct ccaaattgca 4320
   gttcgcgctt agctggataa cgccacggaa tgatgtcgtc gtgcacaaca atggtgactt 4380
   ctacagcgcg gagaatctcg ctctctccag gggaagccga agtttccaaa aggtcgttga 4440
   tcaaagctcg ccgcgttgtt tcatcaagcc ttacggtcac cgtaaccagc aaatcaatat 4500
   cactgtgtgg cttcaggccg ccatccactg cggagccgta caaatgtacg gccagcaacg 4560
   tcggttcgag atggcgctcg atgacgccaa ctacctctga tagttgagtc gatacttcgg 4620
   cgatcaccgc ttccctcatg atgtttaact ttgttttagg gcgactgccc tgctgcgtaa 4680
   catcgttgct gctccataac atcaaacatc gacccacggc gtaacgcgct tgctgcttgg 4740
   atgcccgagg catagactgt accccaaaaa aacagtcata acaagccatg aaaaccgcca 4800
   ctgcgccgtt accaccgctg cgttcggtca aggttctgga ccagttgcgt gagcgcatac 4860
   gctacttgca ttacagctta cgaaccgaac aggcttatgt ccactgggtt cgtgccttca 4920
   tccgtttcca cggtgtgcgt cacccggcaa ccttgggcag cagcgaagtc gaggcatttc 4980
   tgtcctggct ggcgaacgag cgcaaggttt cggtctccac gcatcgtcag gcattggcgg 5040
   ccttgctgtt cttctacggc aaggtgctgt gcacggatct gccctggctt caggagatcg 5100
   gaagacctcg gccgtcgcgg cgcttgccgg tggtgctgac cccggatgaa gtggttcgca 5160
   tcctcggttt tctggaaggc gagcatcgtt tgttcgccca gcttctgtat ggaacgggca 5220
   tgcggatcag tgagggtttg caactgcggg tcaaggatct ggatttcgat cacggcacga 5280
   tcatcgtgcg ggagggcaag ggctccaagg atcgggcctt gatgttaccc gagagcttgg 5340
   cacccagcct gcgcgagcag gggaattgat ccggtggatg accttttgaa tgacctttaa 5400
   tagattatat tactaattaa ttggggaccc tagaggtccc cttttttatt ttaaaaattt 5460
   tttcacaaaa cggtttacaa gcataacggg ttttgctgcc cgcaaacggg ctgttctggt 5520
   gttgctagtt tgttatcaga atcgcagatc cggcttcagg tttgccggct gaaagcgcta 5580
   tttcttccag aattgccatg attttttccc cacgggaggc gtcactggct cccgtgttgt 5640
   cggcagcttt gattcgataa gcagcatcgc ctgtttcagg ctgtctatgt gtgactgttg 5700
   agctgtaaca agttgtctca ggtgttcaat ttcatgttct agttgctttg ttttactggt 5760
   ttcacctgtt ctattaggtg ttacatgctg ttcatctgtt acattgtcga tctgttcatg 5820
   gtgaacagct ttaaatgcac caaaaactcg taaaagctct gatgtatcta tcttttttac 5880
   accgttttca tctgtgcata tggacagttt tccctttgat atctaacggt gaacagttgt 5940
   tctacttttg tttgttagtc ttgatgcttc actgatagat acaagagcca taagaacctc 6000
   agatccttcc gtatttagcc agtatgttct ctagtgtggt tcgttgtttt tgcgtgagcc 6060
   atgagaacga accattgaga tcatgcttac tttgcatgtc actcaaaaat tttgcctcaa 6120
   aactggtgag ctgaattttt gcagttaaag catcgtgtag tgtttttctt agtccgttac 6180
   gtaggtagga atctgatgta atggttgttg gtattttgtc accattcatt tttatctggt 6240
   tgttctcaag ttcggttacg agatccattt gtctatctag ttcaacttgg aaaatcaacg 6300
   tatcagtcgg gcggcctcgc ttatcaacca ccaatttcat attgctgtaa gtgtttaaat 6360
   ctttacttat tggtttcaaa acccattggt taagcctttt aaactcatgg tagttatttt 6420
   caagcattaa catgaactta aattcatcaa ggctaatctc tatatttgcc ttgtgagttt 6480
   tcttttgtgt tagttctttt aataaccact cataaatcct catagagtat ttgttttcaa 6540
   aagacttaac atgttccaga ttatatttta tgaatttttt taactggaaa agataaggca 6600
   atatctcttc actaaaaact aattctaatt tttcgcttga gaacttggca tagtttgtcc 6660
   actggaaaat ctcaaagcct ttaaccaaag gattcctgat ttccacagtt ctcgtcatca 6720
   gctctctggt tgctttagct aatacaccat aagcattttc cctactgatg ttcatcatct 6780
   gagcgtattg gttataagtg aacgataccg tccgttcttt ccttgtaggg ttttcaatcg 6840
   tggggttgag tagtgccaca cagcataaaa ttagcttggt ttcatgctcc gttaagtcat 6900
   agcgactaat cgctagttca tttgctttga aaacaactaa ttcagacata catctcaatt 6960
   ggtctaggtg attttaatca ctataccaat tgagatgggc tagtcaatga taattactag 7020
   tccttttcct ttgagttgtg ggtatctgta aattctgcta gacctttgct ggaaaacttg 7080
   taaattctgc tagaccctct gtaaattccg ctagaccttt gtgtgttttt tttgtttata 7140
   ttcaagtggt tataatttat agaataaaga aagaataaaa aaagataaaa agaatagatc 7200
   ccagccctgt gtataactca ctactttagt cagttccgca gtattacaaa aggatgtcgc 7260
   aaacgctgtt tgctcctcta caaaacagac cttaaaaccc taaaggctta agtagcaccc 7320
   tcgcaagctc gggcaaatcg ctgaatattc cttttgtctc cgaccatcag gcacctgagt 7380
   cgctgtcttt ttcgtgacat tcagttcgct gcgctcacgg ctctggcagt gaatgggggt 7440
   aaatggcact acaggcgcct tttatggatt catgcaagga aactacccat aatacaagaa 7500
   aagcccgtca cgggcttctc agggcgtttt atggcgggtc tgctatgtgg tgctatctga 7560
   ctttttgctg ttcagcagtt cctgccctct gattttccag tctgaccact tcggattatc 7620
   ccgtgacagg tcattcagac tggctaatgc acccagtaag gcagcggtat catcaacagg 7680
   cttacccgtc ttactgtcaa c                                          7701
<210> 26
   <211> 3888
   <212> DNA
   <213> Artificial Sequence
<220>
   <223> plasmid
<400> 26
   tgcgccgcta cagggcgcgt ccattcgcca ttcaggctgc gcaactgttg ggaagggcga 60
   tcggtgcggg cctcttcgct attacgccag tttgggggtg agttcatgaa gtttcgtcgc 120
   agcggcagat tggtggactt aacaaattat ttgttaaccc atccgcacga gttaataccg 180
   ctaacctttt tctctgagcg gtatgaatct gcaaaatcat cgatcagtga agatttaaca 240
   attattaaac aaacctttga acagcagggg attggtactt tgcttactgt tcccggagct 300
   gccggaggcg ttaaatatat tccgaaaatg aagcaggctg aagctgaaga gtttgtgcag 360
   acacttggac agtcgctggc aaatcctgag cgtatccttc cgggcggtta tgtatattta 420
   acggatatct taggaaagcc atctgtactc tccaaggtag ggaagctgtt tgcttccgtg 480
   tttgcagagc gcgaaattga tgttgtcatg accgttgcca cgaaaggcat ccctcttgcg 540
   tacgcagctg caagctattt gaatgtgcct gttgtgatcg ttcgtaaaga caataaggta 600
   acagagggct ccacagtcag cattaattac gtttcaggct cctcaaaccg cattcaaaca 660
   atgtcacttg cgaaaagaag catgaaaacg ggttcaaacg tactcattat tgatgacttt 720
   atgaaagcag gcggcaccat taatggtatg attaacctgt tggatgagtt taacgcaaat 780
   gtggcgggaa tcggcgtctt agttgaagcc gaaggagtag atgaacgtct tgttgacgaa 840
   tatatgtcac ttcttactct ttcaaccatc aacatgaaag agaagtccat tgaaattcag 900
   aatggcaatt ttctgcgttt ttttaaagac aatcttttaa agaatggaga gacagaatca 960
   tgacaaaagc agtccacaca aaacatgccc cagcggcaat cgggccttat tcacaaggga 1020
   ttatcgtcaa caatatgttt tacagctcag gccaaatccc tttgactcct tcaggcgaaa 1080
   tggtgaatgg cgatattaag gagcagactc atcaagtatt cagcaattta aaggcggttc 1140
   tggaagaagc gggtgcttct tttgaaacag ttgtaaaagc aactgtattt atcgcggata 1200
   tggaacagtt tgcggaagta aacgaagtgt acggacaata ttttgacact cacaaaccgg 1260
   cgagatcttg tgttgaagtc gcgagactcc cgaaggatgc gttagtcgag atcgaagtta 1320
   ttgcactggt gaaataataa gaaaagtgat tctgggagag ccgggatcac ttttttattt 1380
   accttatgcc cgaaatgaaa gctttatgac cctgcattaa tgaatcggcc aacgcgcggg 1440
   gagaggcggt ttgcgtattg ggcgctcttc cgcttcctcg ctcactgact cgctgcgctc 1500
   ggtcgttcgg ctgcggcgag cggtatcagc tcactcaaag gcggtaatac ggttatccac 1560
   agaatcaggg gataacgcag gaaagaacat gtgagcaaaa ggccagcaaa aggccaggaa 1620
   ccgtaaaaag gccgcgttgc tggcgttttt cgataggctc cgcccccctg acgagcatca 1680
   caaaaatcga cgctcaagtc agaggtggcg aaacccgaca ggactataaa gataccaggc 1740
   gtttccccct ggaagctccc tcgtgcgctc tcctgttccg accctgccgc ttaccggata 1800
   cctgtccgcc tttctccctt cgggaagcgt ggcgctttct catagctcac gctgtaggta 1860
   tctcagttcg gtgtaggtcg ttcgctccaa gctgggctgt gtgcacgaac cccccgttca 1920
   gcccgaccgc tgcgccttat ccggtaacta tcgtcttgag tccaacccgg taagacacga 1980
   cttatcgcca ctggcagcag ccactggtaa caggattagc agagcgaggt atgtaggcgg 2040
   tgctacagag ttcttgaagt ggtggcctaa ctacggctac actagaagga cagtatttgg 2100
   tatctgcgct ctgctgaagc cagttacctt cggaaaaaga gttggtagct cttgatccgg 2160
   caaacaaacc accgctggta gcggtggttt ttttgtttgc aagcagcaga ttacgcgcag 2220
   aaaaaaagga tctcaagaag atcctttgat cttttctacg gggtctgacg ctcagtggaa 2280
   cgaaaactca cgttaaggga ttttggtcat gagattatca aaaaggatct tcacctagat 2340
   ccttttaaat taaaaatgaa gttttaaatc aatctaaagt atatatgagt aaacttggtc 2400
   tgacagttac caatgcttaa tcagtgaggc acctatctca gcgatctgtc tatttcgttc 2460
   atccatagtt gcctgactcc ccgtcgtgta gataactacg atacgggagg gcttaccatc 2520
   tggccccagt gctgcaatga taccgcgaga cccacgctca ccggctccag atttatcagc 2580
   aataaaccag ccagccggaa gggccgagcg cagaagtggt cctgcaactt tatccgcctc 2640
   catccagtct attaattgtt gccgggaagc tagagtaagt agttcgccag ttaatagttt 2700
   gcgcaacgtt gttggcattg ctacaggcat cgtggtgtca cgctcgtcgt ttggtatggc 2760
   ttcattcagc tccggttccc aacgatcaag gcgagttaca tgatccccca tgttgtgcaa 2820
   aaaagcggtt agctccttcg gtcctccgat cgttgtcaga agtaagttgg ccgcagtgtt 2880
   atcactcatg gttatggcag cactgcataa ttctcttact gtcatgccat ccgtaagatg 2940
   cttttctgtg actggtgagt actcaaccaa gtcattctga gaataccgcg cccggcgacc 3000
   gagttgctct tgcccggcgt caatacggga taatagtgta tgacatagca gaactttaaa 3060
   agtgctcatc attggaaaac gttcttcggg gcgaaaactc tcaaggatct taccgctgtt 3120
   gagatccagt tcgatgtaac ccactcgtgc acccaactga tcttcagcat cttttacttt 3180
   caccagcgtt tctgggtgag caaaaacagg aaggcaaaat gccgcaaaaa agggaataag 3240
   ggcgacacgg aaatgttgaa tactcatact cttccttttt caatattatt gaagcattta 3300
   tcagggttat tgtctcatga gcggatacat atttgaatgt atttagaaaa ataaacaaat 3360
   aggggttccg cgcacatttc cccgaaaagt gccacctgta tgcggtgtga aataccgcac 3420
   agatgcgtaa ggagaaaata ccgcatcagg cgaaattgta aacgttaata ttttgttaaa 3480
   attcgcgtta aatatttgtt aaatcagctc attttttaac caataggccg aaatcggcaa 3540
   aatcccttat aaatcaaaag aatagaccga gatagggttg agtgttgttc cagtttggaa 3600
   caagagtcca ctattaaaga acgtggactc caacgtcaaa gggcgaaaaa ccgtctatca 3660
   gggcgatggc ccactacgtg aaccatcacc caaatcaagt tttttgcggt cgaggtgccg 3720
   taaagctcta aatcggaacc ctaaagggag cccccgattt agagcttgac ggggaaagcc 3780
   ggcgaacgtg gcgagaaagg aagggaagaa agcgaaagga gcgggcgcta gggcgctggc 3840
   aagtgtagcg gtcacgctgc gcgtaaccac cacacccgcc gcgcttaa 3888
<210> 27
   <211> 4606
   <212> DNA
   <213> Artificial Sequence
<220>
   <223> Description of Artificial Sequence:plasmid
<400> 27
   tgcgccgcta cagggcgcgt ccattcgcca ttcaggctgc gcaactgttg ggaagggcga 60
   tcggtgcggg cctcttcgct attacgccag ctggcgaaag ggggatgtgc tgcaaggcga 120
   ttaagttggg taacgccagg gttttcccag tcacgacgtt gtaaaacgac ggccagtgaa 180
   ttgtaatacg actcactata gggcgaattg ggcccgacgt cgcatgctcc cggccgccat 240
   ggccgcggga tgcggccgcg tcgacgtgaa ataccgcaca gatgcgtaag gagaaaatac 300
   cgcatcaggc gataaaccca gcgaaccatt tgaggtgata ggtaagatta taccgaggta 360
   tgaaaacgag aattggacct ttacagaatt actctatgaa gcgccatatt taaaaagcta 420
   ccaagacgaa gaggatgaag aggatgagga ggcagattgc cttgaatata ttgacaatac 480
   tgataagata atatatcttt tatatagaag atatcgccgt atgtaaggat ttcagggggc 540
   aaggcatagg cagcgcgctt atcaatatat ctatagaatg ggcaaagcat aaaaacttgc 600
   atggactaat gcttgaaacc caggacaata accttatagc ttgtaaattc tatcataatt 660
   gtggtttcaa aatcggctcc gtcgatacta tgttatacgc caactttcaa aacaactttg 720
   aaaaagctgt tttctggtat ttaaggtttt agaatgcaag gaacagtgaa ttggagttcg 780
   tcttgttata attagcttct tggggtatct ttaaatactg tagaaaagag gaaggaaata 840
   ataaatggct aaaatgagaa tatcaccgga attgaaaaaa ctgatcgaaa aataccgctg 900
   cgtaaaagat acggaaggaa tgtctcctgc taaggtatat aagctggtgg gagaaaatga 960
   aaacctatat ttaaaaatga cggacagccg gtataaaggg accacctatg atgtggaacg 1020
   ggaaaaggac atgatgctat ggctggaagg aaagctgcct gttccaaagg tcctgcactt 1080
   tgaacggcat gatggctgga gcaatctgct catgagtgag gccgatggcg tcctttgctc 1140
   ggaagagtat gaagatgaac aaagccctga aaagattatc gagctgtatg cggagtgcat 1200
   caggctcttt cactccatcg acatatcgga ttgtccctat acgaatagct tagacagccg 1260
   cttagccgaa ttggattact tactgaataa cgatctggcc gatgtggatt gcgaaaactg 1320
   ggaagaagac actccattta aagatccgcg cgagctgtat gattttttaa agacggaaaa 1380
   gcccgaagag gaacttgtct tttcccacgg cgacctggga gacagcaaca tctttgtgaa 1440
   agatggcaaa gtaagtggct ttattgatct tgggagaagc ggcagggcgg acaagtggta 1500
   tgacattgcc ttctgcgtcc ggtcgatcag ggaggatatc ggggaagaac agtatgtcga 1560
   gctatttttt gacttactgg ggatcaagcc tgattgggag aaaataaaat attatatttt 1620
   actggatgaa ttgttttagt acctagattt agatgtctaa aaagctttaa ctacaagctt 1680
   tttagacatc taatcttttc tgaagtacat ccgcaactgt ccatactctg atgttttata 1740
   tcttttctaa aagttcgcta gataggggtc ccgagcgcct acgaggaatt tgtatcgcca 1800
   ttcgccattc aggctgcgca actgttggga agggcgatcg gtgcgggtac cgggatcact 1860
   agtgcggccg cctgcaggtc gaccatatgg gagagctccc aacgcgttgg atgcatagct 1920
   tgagtattct atagtgtcac ctaaatagct tggcgtaatc atggtcatag ctgtttcctg 1980
   tgtgaaattg ttatccgctc acaattccac acaacatacg agccggaagc ataaagtgta 2040
   aagcctgggg tgcctaatga gtgagctaac tcacattaat tgcgttgcgc tcactgcccg 2100
   ctttccagtc gggaaacctg tcgtgccagc tgcattaatg aatcggccaa cgcgcgggga 2160
   gaggcggttt gcgtattggg cgctcttccg cttcctcgct cactgactcg ctgcgctcgg 2220
   tcgttcggct gcggcgagcg gtatcagctc actcaaaggc ggtaatacgg ttatccacag 2280
   aatcagggga taacgcagga aagaacatgt gagcaaaagg ccagcaaaag gccaggaacc 2340
   gtaaaaaggc cgcgttgctg gcgtttttcg ataggctccg cccccctgac gagcatcaca 2400
   aaaatcgacg ctcaagtcag aggtggcgaa acccgacagg actataaaga taccaggcgt 2460
   ttccccctgg aagctccctc gtgcgctctc ctgttccgac cctgccgctt accggatacc 2520
   tgtccgcctt tctcccttcg ggaagcgtgg cgctttctca tagctcacgc tgtaggtatc 2580
   tcagttcggt gtaggtcgtt cgctccaagc tgggctgtgt gcacgaaccc cccgttcagc 2640
   ccgaccgctg cgccttatcc ggtaactatc gtcttgagtc caacccggta agacacgact 2700
   tatcgccact ggcagcagcc actggtaaca ggattagcag agcgaggtat gtaggcggtg 2760
   ctacagagtt cttgaagtgg tggcctaact acggctacac tagaaggaca gtatttggta 2820
   tctgcgctct gctgaagcca gttaccttcg gaaaaagagt tggtagctct tgatccggca 2880
   aacaaaccac cgctggtagc ggtggttttt ttgtttgcaa gcagcagatt acgcgcagaa 2940
   aaaaaggatc tcaagaagat cctttgatct tttctacggg gtctgacgct cagtggaacg 3000
   aaaactcacg ttaagggatt ttggtcatga gattatcaaa aaggatcttc acctagatcc 3060
   ttttaaatta aaaatgaagt tttaaatcaa tctaaagtat atatgagtaa acttggtctg 3120
   acagttacca atgcttaatc agtgaggcac ctatctcagc gatctgtcta tttcgttcat 3180
   ccatagttgc ctgactcccc gtcgtgtaga taactacgat acgggagggc ttaccatctg 3240
   gccccagtgc tgcaatgata ccgcgagacc cacgctcacc ggctccagat ttatcagcaa 3300
   taaaccagcc agccggaagg gccgagcgca gaagtggtcc tgcaacttta tccgcctcca 3360
   tccagtctat taattgttgc cgggaagcta gagtaagtag ttcgccagtt aatagtttgc 3420
   gcaacgttgt tggcattgct acaggcatcg tggtgtcacg ctcgtcgttt ggtatggctt 3480
   cattcagctc cggttcccaa cgatcaaggc gagttacatg atcccccatg ttgtgcaaaa 3540
   aagcggttag ctccttcggt cctccgatcg ttgtcagaag taagttggcc gcagtgttat 3600
   cactcatggt tatggcagca ctgcataatt ctcttactgt catgccatcc gtaagatgct 3660
   tttctgtgac tggtgagtac tcaaccaagt cattctgaga ataccgcgcc cggcgaccga 3720
   gttgctcttg cccggcgtca atacgggata atagtgtatg acatagcaga actttaaaag 3780
   tgctcatcat tggaaaacgt tcttcggggc gaaaactctc aaggatctta ccgctgttga 3840
   gatccagttc gatgtaaccc actcgtgcac ccaactgatc ttcagcatct tttactttca 3900
   ccagcgtttc tgggtgagca aaaacaggaa ggcaaaatgc cgcaaaaaag ggaataaggg 3960
   cgacacggaa atgttgaata ctcatactct tcctttttca atattattga agcatttatc 4020
   agggttattg tctcatgagc ggatacatat ttgaatgtat ttagaaaaat aaacaaatag 4080
   gggttccgcg cacatttccc cgaaaagtgc cacctgtatg cggtgtgaaa taccgcacag 4140
   atgcgtaagg agaaaatacc gcatcaggcg aaattgtaaa cgttaatatt ttgttaaaat 4200
   tcgcgttaaa tatttgttaa atcagctcat tttttaacca ataggccgaa atcggcaaaa 4260
   tcccttataa atcaaaagaa tagaccgaga tagggttgag tgttgttcca gtttggaaca 4320
   agagtccact attaaagaac gtggactcca acgtcaaagg gcgaaaaacc gtctatcagg 4380
   gcgatggccc actacgtgaa ccatcaccca aatcaagttt tttgcggtcg aggtgccgta 4440
   aagctctaaa tcggaaccct aaagggagcc cccgatttag agcttgacgg ggaaagccgg 4500
   cgaacgtggc gagaaaggaa gggaagaaag cgaaaggagc gggcgctagg gcgctggcaa 4560
   gtgtagcggt cacgctgcgc gtaaccacca cacccgccgc gcttaa                4606
<210> 28
   <211> 5399
   <212> DNA
   <213> Artificial Sequence
<220>
   <223> Description of Artificial Sequence:plasmid
<400> 28
   tgcgccgcta cagggcgcgt ccattcgcca ttcaggctgc gcaactgttg ggaagggcga 60
   tcggtgcggg cctcttcgct attacgccag tttgggggtg agttcatgaa gtttcgtcgc 120
   agcggcagat tggtggactt aacaaattat ttgttaaccc atccgcacga gttaataccg 180
   ctaacctttt tctctgagcg gtatgaatct gcaaaatcat cgatcagtga agatttaaca 240
   attattaaac aaacctttga acagcagggg attggtactt tgcttactgt tcccggagct 300
   gccggaggcg ttaaatatat tccgaaaatg aagcaggctg aagctgaaga gtttgtgcag 360
   acacttggac agtcgctggc aaatcctgag cgtatccttc cgggcggtta tgtatattta 420
   acggatatct taggaaagcc atctgtactc tccaaggtag ggaagctgtt tgcttccgtg 480
   tttgcagagc gcgaaattga tgttgtcatg accgttgcca cgaaaggcat ccctcttgcg 540
   tacgcagctg cggccgcgtc gacaaaccca gtgaaccatt tgaggtgata ggtaagatta 600
   taccgaggta tgaaaacgag aattggacct ttacagaatt actctatgaa gcgccatatt 660
   taaaaagcta ccaagacgaa gaggatgaag aggatgagga ggcagattgc cttgaatata 720
   ttgacaatac tgataagata atatatcttt tatatagaag atatcgccgt atgtaaggat 780
   ttcagggggc aaggcatagg cagcgcgctt atcaatatat ctatagaatg ggcaaagcat 840
   aaaaacttgc atggactaat gcttgaaacc caggacaata accttatagc ttgtaaattc 900
   tatcataatt gtggtttcaa aatcggctcc gtcgatacta tgttatacgc caactttcaa 960
   aacaactttg aaaaagctgt tttctggtat ttaaggtttt agaatgcaag gaacagtgaa 1020
   ttggagttcg tcttgttata attagcttct tggggtatct ttaaatactg tagaaaagag 1080
   gaaggaaata ataaatggct aaaatgagaa tatcaccgga attgaaaaaa ctgatcgaaa 1140
   aataccgctg cgtaaaagat acggaaggaa tgtctcctgc taaggtatat aagctggtgg 1200
   gagaaaatga aaacctatat ttaaaaatga cggacagccg gtataaaggg accacctatg 1260
   atgtggaacg ggaaaaggac atgatgctat ggctggaagg aaagctgcct gttccaaagg 1320
   tcctgcactt tgaacggcat gatggctgga gcaatctgct catgagtgag gccgatggcg 1380
   tcctttgctc ggaagagtat gaagatgaac aaagccctga aaagattatc gagctgtatg 1440
   cggagtgcat caggctcttt cactccatcg acatatcgga ttgtccctat acgaatagct 1500
   tagacagccg cttagccgaa ttggattact tactgaataa cgatctggcc gatgtggatt 1560
   gcgaaaactg ggaagaagac actccattta aagatccgcg cgagctgtat gattttttaa 1620
   agacggaaaa gcccgaagag gaacttgtct tttcccacgg cgacctggga gacagcaaca 1680
   tctttgtgaa agatggcaaa gtaagtggct ttattgatct tgggagaagc ggcagggcgg 1740
   acaagtggta tgacattgcc ttctgcgtcc ggtcgatcag ggaggatatc ggggaagaac 1800
   agtatgtcga gctatttttt gacttactgg ggatcaagcc tgattgggag aaaataaaat 1860
   attatatttt actggatgaa ttgttttagt acctagattt agatgtctaa aaagctttaa 1920
   ctacaagctt tttagacatc taatcttttc tgaagtacat ccgcaactgt ccatactctg 1980
   atgttttata tcttttctaa aagttcgcta gataggggtc ccgagcgcct acgaggaatt 2040
   tgtatcacca ggtaccagct gcaagctatt tgaatgtgcc tgttgtgatc gttcgtaaag 2100
   acaataaggt aacagagggc tccacagtca gcattaatta cgtttcaggc tcctcaaacc 2160
   gcattcaaac aatgtcactt gcgaaaagaa gcatgaaaac gggttcaaac gtactcatta 2220
   ttgatgactt tatgaaagca ggcggcacca ttaatggtat gattaacctg ttggatgagt 2280
   ttaacgcaaa tgtggcggga atcggcgtct tagttgaagc cgaaggagta gatgaacgtc 2340
   ttgttgacga atatatgtca cttcttactc tttcaaccat caacatgaaa gagaagtcca 2400
   ttgaaattca gaatggcaat tttctgcgtt tttttaaaga caatctttta aagaatggag 2460
   agacagaatc atgacaaaag cagtccacac aaaacatgcc ccagcggcaa tcgggcctta 2520
   ttcacaaggg attatcgtca acaatatgtt ttacagctca ggccaaatcc ctttgactcc 2580
   ttcaggcgaa atggtgaatg gcgatattaa ggagcagact catcaagtat tcagcaattt 2640
   aaaggcggtt ctggaagaag cgggtgcttc ttttgaaaca gttgtaaaag caactgtatt 2700
   tatcgcggat atggaacagt ttgcggaagt aaacgaagtg tacggacaat attttgacac 2760
   tcacaaaccg gcgagatctt gtgttgaagt cgcgagactc ccgaaggatg cgttagtcga 2820
   gatcgaagtt attgcactgg tgaaataata agaaaagtga ttctgggaga gccgggatca 2880
   cttttttatt taccttatgc ccgaaatgaa agctttatga ccctgcatta atgaatcggc 2940
   caacgcgcgg ggagaggcgg tttgcgtatt gggcgctctt ccgcttcctc gctcactgac 3000
   tcgctgcgct cggtcgttcg gctgcggcga gcggtatcag ctcactcaaa ggcggtaata 3060
   cggttatcca cagaatcagg ggataacgca ggaaagaaca tgtgagcaaa aggccagcaa 3120
   aaggccagga accgtaaaaa ggccgcgttg ctggcgtttt tcgataggct ccgcccccct 3180
   gacgagcatc acaaaaatcg acgctcaagt cagaggtggc gaaacccgac aggactataa 3240
   agataccagg cgtttccccc tggaagctcc ctcgtgcgct ctcctgttcc gaccctgccg 3300
   cttaccggat acctgtccgc ctttctccct tcgggaagcg tggcgctttc tcatagctca 3360
   cgctgtaggt atctcagttc ggtgtaggtc gttcgctcca agctgggctg tgtgcacgaa 3420
   ccccccgttc agcccgaccg ctgcgcctta tccggtaact atcgtcttga gtccaacccg 3480
   gtaagacacg acttatcgcc actggcagca gccactggta acaggattag cagagcgagg 3540
   tatgtaggcg gtgctacaga gttcttgaag tggtggccta actacggcta cactagaagg 3600
   acagtatttg gtatctgcgc tctgctgaag ccagttacct tcggaaaaag agttggtagc 3660
   tcttgatccg gcaaacaaac caccgctggt agcggtggtt tttttgtttg caagcagcag 3720
   attacgcgca gaaaaaaagg atctcaagaa gatcctttga tcttttctac ggggtctgac 3780
   gctcagtgga acgaaaactc acgttaaggg attttggtca tgagattatc aaaaaggatc 3840
   ttcacctaga tccttttaaa ttaaaaatga agttttaaat caatctaaag tatatatgag 3900
   taaacttggt ctgacagtta ccaatgctta atcagtgagg cacctatctc agcgatctgt 3960
   ctatttcgtt catccatagt tgcctgactc cccgtcgtgt agataactac gatacgggag 4020
   ggcttaccat ctggccccag tgctgcaatg ataccgcgag acccacgctc accggctcca 4080
   gatttatcag caataaacca gccagccgga agggccgagc gcagaagtgg tcctgcaact 4140
   ttatccgcct ccatccagtc tattaattgt tgccgggaag ctagagtaag tagttcgcca 4200
   gttaatagtt tgcgcaacgt tgttggcatt gctacaggca tcgtggtgtc acgctcgtcg 4260
   tttggtatgg cttcattcag ctccggttcc caacgatcaa ggcgagttac atgatccccc 4320
   atgttgtgca aaaaagcggt tagctccttc ggtcctccga tcgttgtcag aagtaagttg 4380
   gccgcagtgt tatcactcat ggttatggca gcactgcata attctcttac tgtcatgcca 4440
   tccgtaagat gcttttctgt gactggtgag tactcaacca agtcattctg agaataccgc 4500
   gcccggcgac cgagttgctc ttgcccggcg tcaatacggg ataatagtgt atgacatagc 4560
   agaactttaa aagtgctcat cattggaaaa cgttcttcgg ggcgaaaact ctcaaggatc 4620
   ttaccgctgt tgagatccag ttcgatgtaa cccactcgtg cacccaactg atcttcagca 4680
   tcttttactt tcaccagcgt ttctgggtga gcaaaaacag gaaggcaaaa tgccgcaaaa 4740
   aagggaataa gggcgacacg gaaatgttga atactcatac tcttcctttt tcaatattat 4800
   tgaagcattt atcagggtta ttgtctcatg agcggataca tatttgaatg tatttagaaa 4860
   aataaacaaa taggggttcc gcgcacattt ccccgaaaag tgccacctgt atgcggtgtg 4920
   aaataccgca cagatgcgta aggagaaaat accgcatcag gcgaaattgt aaacgttaat 4980
   attttgttaa aattcgcgtt aaatatttgt taaatcagct cattttttaa ccaataggcc 5040
   gaaatcggca aaatccctta taaatcaaaa gaatagaccg agatagggtt gagtgttgtt 5100
   ccagtttgga acaagagtcc actattaaag aacgtggact ccaacgtcaa agggcgaaaa 5160
   accgtctatc agggcgatgg cccactacgt gaaccatcac ccaaatcaag ttttttgcgg 5220
   tcgaggtgcc gtaaagctct aaatcggaac cctaaaggga gcccccgatt tagagcttga 5280
   cggggaaagc cggcgaacgt ggcgagaaag gaagggaaga aagcgaaagg agcgggcgct 5340
   agggcgctgg caagtgtagc ggtcacgctg cgcgtaacca ccacacccgc cgcgcttaa  5399
<210> 29
   <211> 6805
   <212> DNA
   <213> Artificial Sequence
<220>
   <223> Description of Artificial Sequence:plasmid
<400> 29
   ttgcggccgc ttcgaaagct gtaatataaa aaccttcttc aactaacggg gcaggttagt 60
   gacattagaa aaccgactgt aaaaagtaca gtcggcatta tctcatatta taaaagccag 120
   tcattaggcc tatctgacaa ttcctgaata gagttcataa acaatcctgc atgataacca 180
   tcacaaacag aatgatgtac ctgtaaagat agcggtaaat atattgaatt acctttatta 240
   atgaattttc ctgctgtaat aatgggtaga aggtaattac tattattatt gatatttaag 300
   ttaaacccag taaatgaagt ccatggaata atagaaagag aaaaagcatt ttcaggtata 360
   ggtgttttgg gaaacaattt ccccgaacca ttatatttct ctacatcaga aaggtataaa 420
   tcataaaact ctttgaagtc attctttaca ggagtccaaa taccagagaa tgttttagat 480
   acaccatcaa aaattgtata aagtggctct aacttatccc aataacctaa ctctccgtcg 540
   ctattgtaac cagttctaaa agctgtattt gagtttatca cccttgtcac taagaaaata 600
   aatgcagggt aaaatttata tccttcttgt tttatgtttc ggtataaaac actaatatca 660
   atttctgtgg ttatactaaa agtcgtttgt tggttcaaat aatgattaaa tatctctttt 720
   ctcttccaat tgtctaaatc aattttatta aagttcattt gatatgcctc ctaaattttt 780
   atctaaagtg aatttaggag gcttacttgt ctgctttctt cattagaatc aatccttttt 840
   taaaagtcaa tattactgta acataaatat atattttaaa aatatcccac tttatccaat 900
   tttcgtttgt tgaactaatg ggtgctttag ttgaagaata aagaccacat taaaaaatgt 960
   ggtcttttgt gtttttttaa aggatttgag cgtagcgaaa aatccttttc tttcttatct 1020
   tgataataag ggtaactatt gaattcggta ccaagagttt gtagaaacgc aaaaaggcca 1080
   tccgtcagga tggccttctg cttaatttga tgcctggcag tttatggcgg gcgtcctgcc 1140
   cgccaccctc cgggccgttg cttcgcaacg ttcaaatccg ctcccggcgg atttgtccta 1200
   ctcaggagag cgttcaccga caaacaacag ataaaacgaa aggcccagtc tttcgactga 1260
   gcctttcgtt ttatttgatg cctggcagtt ccctactctc gcatggggag accccacact 1320
   accatcggcg ctacggcgtt tcacttctga gttcggcatg gggtcaggtg ggaccaccgc 1380
   gctactgccg ccaggcaaat tctgttttat cagaccgctt ctgcgttctg atttaatctg 1440
   tatcaggctg aaaatcttct ctcatccgcc aaaacaggat ccaattatgg cagatcaatg 1500
   agcttcacag acacaatatc agggacattt gttagttctt tcacaatttt atcttccaga 1560
   tgtctgtcaa aggaaagcat catgatggct tctccgcctt tttccttacg gccaacctgc 1620
   atagttgcaa tgttaatatc attatctccg agaatacgtc ctactcggcc gatgacacct 1680
   gttgtatctt gatgctggat atacaccaag tgaccagtcg gataaaaatc aatattaaat 1740
   ccattgatct cgacaattcg ttctccgaaa tgaggaatat acgtagccgt tacagtaaag 1800
   gtgctgcggt ctcctgtcac ttttacgctg atgcagttat cgtatccaga ttcagaagag 1860
   gaaatttttt cactgaagct aatgccgcgt tcttttgcga cacccccggc attgacctca 1920
   ttaacagtag agtctacgcg cggttttaaa aagcctgaca gaagggcttt tgtaatgaac 1980
   gatgtttcaa gtttagcaat tgtgccttca tattgaatgg caacatcctg tactggttct 2040
   ttcatgcact gtgatacaag gctgccaatt tttcctgcaa tttgatggta aggcttaatt 2100
   ttagcaaatt catcttttgt catggcaggc aggttgatag ctgacatgac aggcaggcct 2160
   tttgcgaact gcagaacttc ttctgacact tgggcggcga cattgagctg tgcttctttc 2220
   gttgatgctc ccaagtgagg agtggcaatg actaatggat gatcaacaag tttgttgtca 2280
   actggcggtt cgacttcgaa aacgtcaagc gctgctcccg caacatgccc gttttccaaa 2340
   gcttcgagaa gtgctgcttc atcgataatt ccgcctcgcg cacagttaat taagcgaacg 2400
   ccttttttcg tttttgcaat cgtttcttta ttcaataagc cttttgtttc ttttgttaaa 2460
   ggcgtgtgaa cggtaatgat atccgcactt tcaagcactt cttcaaatgt acggctgttt 2520
   acgccgattt ttttcgctct ttcttccgtt aagaaaggat caaaaacgtg cacagtcata 2580
   ccgaacgctc ctcgacgctg tgcaatttca cttccgattc ggcctaatcc tacaatacca 2640
   agcgtttttc cataaagctc tgaaccgaca taagctgtgc ggttccactc tctggatttc 2700
   actgagatat tagcctgcgg aatgtgtctc attaaagaag agatcattgc aaatgtatgc 2760
   tcagctgtcg aaatggtgtt gccgttcgga gcattgatca cgattacccc gtgtttcgta 2820
   gcctcatcaa tatcgatatt atcgacaccg acaccggctc ttccgacaat ttttaaagaa 2880
   gtcattttgt tgaaaaggtc ttctgttact tttgtcgcgc ttcgcaccaa aagagcatca 2940
   aaagtatgta attcatcttc tgcatctgct acgttttttt gaacgatttc aataaagtct 3000
   gattcaataa gtggctgtaa accgtcgttg ctcattttgt ctgagaccaa tactcgaaac 3060
   atgttttctc ctcctctaga gcgtcctgct gttgttaaga ttattatacc acaccttgta 3120
   gataaagtca acaacttttt gcaaaatttt tcaggaattt tagcagaggt tgttctggat 3180
   gtagaacaaa acatctttcc gctcttgtgc tgttaggata tctttcttgg aagctaggta 3240
   ggcctcgagt tatggcagtt ggttaaaagg aaacaaaaag accgttttca cacaaaacgg 3300
   tctttttcga tttcttttta cagtcacagc cacttttgca aaaaccggac agcttcatgc 3360
   cttataactg ctgtttcggt cgacctgcag gcatgcaagc ttcgcgaagc ggccgccgac 3420
   gcgaggctgg atggccttcc ccattatgat tcttctcgct tccggcggca tcgggatgcc 3480
   cgcgttgcag gccatgctgt ccaggcaggt agatgacgac catcagggac agcttcaagg 3540
   atcgctcgcg gctcttacca gcctaacttc gatcactgga ccgctgatcg tcacggcgat 3600
   ttatgccgcc tcggcgagca catggaacgg gttggcatgg attgtaggcg ccgccctata 3660
   ccttgtctgc ctccccgcgt tgcgtcgcgg tgcatggagc cgggccacct cgacctgaat 3720
   ggaagccggc ggcacctcgc taacggattc accactccaa gaattggagc caatcaattc 3780
   ttgcggagaa ctgtgaatgc gcaaaccaac ccttggcaga acatatccat cgcgtccgcc 3840
   atctccagca gccgcacgcg gcgcatctcg ggcagcgttg ggtcctggcc acgggtgcgc 3900
   atgatcgtgc tcctgtcgtt gaggacccgg ctaggctggc ggggttgcct tactggttag 3960
   cagaatgaat caccgatacg cgagcgaacg tgaagcgact gctgctgcaa aacgtctgcg 4020
   acctgagcaa caacatgaat ggtcttcggt ttccgtgttt cgtaaagtct ggaaacgcgg 4080
   aagtcagcgc cctgcaccat tatgttccgg atctgcatcg caggatgctg ctggctaccc 4140
   tgtggaacac ctacatctgt attaacgaag cgctggcatt gaccctgagt gatttttctc 4200
   tggtcccgcc gcatccatac cgccagttgt ttaccctcac aacgttccag taaccgggca 4260
   tgttcatcat cagtaacccg tatcgtgagc atcctctctc gtttcatcgg tatcattacc 4320
   cccatgaaca gaaattcccc cttacacgga ggcatcaagt gaccaaacag gaaaaaaccg 4380
   cccttaacat ggcccgcttt atcagaagcc agacattaac gcttctggag aaactcaacg 4440
   agctggacgc ggatgaacag gcagacatct gtgaatcgct tcacgaccac gctgatgagc 4500
   tttaccgcag ctgcctcgcg cgtttcggtg atgacggtga aaacctctga cacatgcagc 4560
   tcccggagac ggtcacagct tgtctgtaag cggatgccgg gagcagacaa gcccgtcagg 4620
   gcgcgtcagc gggtgttggc gggtgtcggg gcgcagccat gacccagtca cgtagcgata 4680
   gcggagtgta tactggctta actatgcggc atcagagcag attgtactga gagtgcacca 4740
   tatgcggtgt gaaataccgc acagatgcgt aaggagaaaa taccgcatca ggcgctcttc 4800
   cgcttcctcg ctcactgact cgctgcgctc ggtcgttcgg ctgcggcgag cggtatcagc 4860
   tcactcaaag gcggtaatac ggttatccac agaatcaggg gataacgcag gaaagaacat 4920
   gtgagcaaaa ggccagcaaa aggccaggaa ccgtaaaaag gccgcgttgc tggcgttttt 4980
   ccataggctc cgcccccctg acgagcatca caaaaatcga cgctcaagtc agaggtggcg 5040
   aaacccgaca ggactataaa gataccaggc gtttccccct ggaagctccc tcgtgcgctc 5100
   tcctgttccg accctgccgc ttaccggata cctgtccgcc tttctccctt cgggaagcgt 5160
   ggcgctttct catagctcac gctgtaggta tctcagttcg gtgtaggtcg ttcgctccaa 5220
   gctgggctgt gtgcacgaac cccccgttca gcccgaccgc tgcgccttat ccggtaacta 5280
   tcgtcttgag tccaacccgg taagacacga cttatcgcca ctggcagcag ccactggtaa 5340
   caggattagc agagcgaggt atgtaggcgg tgctacagag ttcttgaagt ggtggcctaa 5400
   ctacggctac actagaagga cagtatttgg tatctgcgct ctgctgaagc cagttacctt 5460
   cggaaaaaga gttggtagct cttgatccgg caaacaaacc accgctggta gcggtggttt 5520
   ttttgtttgc aagcagcaga ttacgcgcag aaaaaaagga tctcaagaag atcctttgat 5580
   cttttctacg gggtctgacg ctcagtggaa cgaaaactca cgttaaggga ttttggtcat 5640
   gagattatca aaaaggatct tcacctagat ccttttaaat taaaaatgaa gttttaaatc 5700
   aatctaaagt atatatgagt aaacttggtc tgacagttac caatgcttaa tcagtgaggc 5760
   acctatctca gcgatctgtc tatttcgttc atccatagtt gcctgactcc ccgtcgtgta 5820
   gataactacg atacgggagg gcttaccatc tggccccagt gctgcaatga taccgcgaga 5880
   cccacgctca ccggctccag atttatcagc aataaaccag ccagccggaa gggccgagcg 5940
   cagaagtggt cctgcaactt tatccgcctc catccagtct attaattgtt gccgggaagc 6000
   tagagtaagt agttcgccag ttaatagttt gcgcaacgtt gttgccattg ctgcaggcat 6060
   cgtggtgtca cgctcgtcgt ttggtatggc ttcattcagc tccggttccc aacgatcaag 6120
   gcgagttaca tgatccccca tgttgtgcaa aaaagcggtt agctccttcg gtcctccgat 6180
   cgttgtcaga agtaagttgg ccgcagtgtt atcactcatg gttatggcag cactgcataa 6240
   ttctcttact gtcatgccat ccgtaagatg cttttctgtg actggtgagt actcaaccaa 6300
   gtcattctga gaatagtgta tgcggcgacc gagttgctct tgcccggcgt caatacggga 6360
   taataccgcg ccacatagca gaactttaaa agtgctcatc attggaaaac gttcttcggg 6420
   gcgaaaactc tcaaggatct taccgctgtt gagatccagt tcgatgtaac ccactcgtgc 6480
   acccaactga tcttcagcat cttttacttt caccagcgtt tctgggtgag caaaaacagg 6540
   aaggcaaaat gccgcaaaaa agggaataag ggcgacacgg aaatgttgaa tactcatact 6600
   cttccttttt caatattatt gaagcattta tcagggttat tgtctcatga gcggatacat 6660
   atttgaatgt atttagaaaa ataaacaaat aggggttccg cgcacatttc cccgaaaagt 6720
   gccacctgac gtctaagaaa ccattattat catgacatta acctataaaa ataggcgtat 6780
   cacgaggccc tttcgtcttc aagaa                                      6805
<210> 30
   <211> 5983
   <212> DNA
   <213> Artificial Sequence
<220>
   <223> Description of Artificial Sequence:plasmid
<400> 30
   tgcgccgcta cagggcgcgt ccattcgcca ttcaggctgc gcaactgttg ggaagggcga 60
   tcggtgcggg cctcttcgct attacgccag ctggcgaaag ggggatgtgc tgcaaggcga 120
   ttaagttggg taacgccagg gttttcccag tcacgacgtt gtaaaacgac ggccagtgaa 180
   ttgtaatacg actcactata gggcgaattg ggcccgacgt cgcatgctcc cggccgccat 240
   ggccgcggga tatcactagt gcggccgcct gcaggtcgac catatgggag agcccggatc 300
   caattatggc agatcaatga gcttcacaga cacaatatca gggacatttg ttagttcttt 360
   cacaatttta tcttccagat gtctgtcaaa ggaaagcatc atgatggctt ctccgccttt 420
   ttccttacgg ccaacctgca tagttgcaat gttaatatca ttatctccga gaatacgtcc 480
   tactcggccg atgacacctg ttgtatcttg atgctggata tacaccaagt gaccagtcgg 540
   ataaaaatca atattaaatc cattgatctc gacaattcgt tctccgaaat gaggaatata 600
   cgtagccgtt acagtaaagg tgctgcggtc tcctgtcact tttacgctga tgcagttatc 660
   gtatccagat tcagaagagg aaattttttc actgaagcta atgccgcgtt cttttgcgac 720
   acccccggca ttgacctcat taacagtaga gtctacgcgc ggttttaaaa agcctgacag 780
   aagggctttt gtaatgaacg atgtttcaag tttagcaatt gtgccttcat attgaatggc 840
   aacatcctgt actggttctt tcatgcactg tgatacaagg ctgccaattt ttcctgcaat 900
   ttgatggtaa ggcttaattt tagcaaattc atcttttgtc atggcaggca ggttgatagc 960
   tgacatgaca ggcaggcctt ttgcgaactg cagaacttct tctgacactt gggcggcgac 1020
   attgagctgt gcttctttcg ttgatgctcc caagtgagga gtggcaatga ctaatggatg 1080
   atcaacaagt ttgttgtcaa ctggcggttc gacttcgaaa acgtcaagcg ctgctcccgc 1140
   aacatgcccg ttttccaaag ctttttagac atctaaatct aggtactaaa acaattcatc 1200
   cagtaaaata taatatttta ttttctccca atcaggcttg atccccagta agtcaaaaaa 1260
   tagctcgaca tactgttctt ccccgatatc ctccctgatc gaccggacgc agaaggcaat 1320
   gtcataccac ttgtccgccc tgccgcttct cccaagatca ataaagccac ttactttgcc 1380
   atctttcaca aagatgttgc tgtctcccag gtcgccgtgg gaaaagacaa gttcctcttc 1440
   gggcttttcc gtctttaaaa aatcatacag ctcgcgcgga tctttaaatg gagtgtcttc 1500
   ttcccagttt tcgcaatcca catcggccag atcgttattc agtaagtaat ccaattcggc 1560
   taagcggctg tctaagctat tcgtataggg acaatccgat atgtcgatgg agtgaaagag 1620
   cctgatgcac tccgcataca gctcgataat cttttcaggg ctttgttcat cttcatactc 1680
   ttccgagcaa aggacgccat cggcctcact catgagcaga ttgctccagc catcatgccg 1740
   ttcaaagtgc aggacctttg gaacaggcag ctttccttcc agccatagca tcatgtcctt 1800
   ttcccgttcc acatcatagg tggtcccttt ataccggctg tccgtcattt ttaaatatag 1860
   gttttcattt tctcccacca gcttatatac cttagcagga gacattcctt ccgtatcttt 1920
   tacgcagcgg tatttttcga tcagtttttt caattccggt gatattctca ttttagccat 1980
   ttattatttc cttcctcttt tctacagtat ttaaagatac cccaagaagc taattataac 2040
   aagacgaact ccaattcact gttccttgca ttctaaaacc ttaaatacca gaaaacagct 2100
   ttttcaaagt tgttttgaaa gttggcgtat aacatagtat cgacggagcc gattttgaaa 2160
   ccacaattat gatagaattt acaagctata aggttattgt cctgggtttc aagcattagt 2220
   ccatgcaagt ttttatgctt tgcccattct atagatatat tgataagcgc gctgcctatg 2280
   ccttgccccc tgaaatcctt acatacggcg atatcttcta tataaaagat atattatctt 2340
   atcagtattg tcaatatatt caaggcaatc tgcctcctca tcctcttcat cctcttcgtc 2400
   ttggtagctt tttaaatatg gcgcttcata gagtaattct gtaaaggtcc aattctcgtt 2460
   ttcatacctc ggtataatct tacctatcac ctcaaatggt tcgctgggtt tatcgcctga 2520
   tgcggtattt tctccttacg catctgtgcg gtatttcacg tcgacgcggc cgccatggcc 2580
   gcgggatccc ggtaccgaaa catcgttaga tttcctccta aattgacaaa ctaaatatct 2640
   gataatttaa catattctca aaagagtgtc aacgtgtatt gacgcagtaa aggataaaag 2700
   taaagcctaa taaatcaatg atctgacagc ttgcaggtaa tatatttaat ttgaagcaat 2760
   tctctataca gccaaccagt tatcgtttat aatgtaatta aatttcatat gatcaatctt 2820
   cggggcaggg tgaaattccc taccggcggt gatgagccaa tggctctaag cccgcgagct 2880
   gtctttacag caggattcgg tgagattccg gagccgacag tacagtctgg atgggagaag 2940
   atggaggttc ataagcgttt tgaaattgaa tttttcaaac gtttctttgc ctagcctaat 3000
   tttcgaaacc ccgcttttat atatgaagcg gtttttttat tggctggaaa agaacctttc 3060
   cgttttcgag taagatgtga tcgaaaagga gagaatgaag tgaaagtaaa aaaattagtt 3120
   gtggtcagca tgctgagcag cattgcattt gttttgatgc tgttaaattt cccgtttccg 3180
   ggtcttccgg attatttaaa aatcgatttt agcgacgttc ccgcaattat tgccattctg 3240
   atttacggac ctttggcggg atcactagag ggctcccaac gcgttggatg catagcttga 3300
   gtattctata gtgtcaccta aatagcttgg cgtaatcatg gtcatagctg tttcctgtgt 3360
   gaaattgtta tccgctcaca attccacaca acatacgagc cggaagcata aagtgtaaag 3420
   cctggggtgc ctaatgagtg agctaactca cattaattgc gttgcgctca ctgcccgctt 3480
   tccagtcggg aaacctgtcg tgccagctgc attaatgaat cggccaacgc gcggggagag 3540
   gcggtttgcg tattgggcgc tcttccgctt cctcgctcac tgactcgctg cgctcggtcg 3600
   ttcggctgcg gcgagcggta tcagctcact caaaggcggt aatacggtta tccacagaat 3660
   caggggataa cgcaggaaag aacatgtgag caaaaggcca gcaaaaggcc aggaaccgta 3720
   aaaaggccgc gttgctggcg tttttcgata ggctccgccc ccctgacgag catcacaaaa 3780
   atcgacgctc aagtcagagg tggcgaaacc cgacaggact ataaagatac caggcgtttc 3840
   cccctggaag ctccctcgtg cgctctcctg ttccgaccct gccgcttacc ggatacctgt 3900
   ccgcctttct cccttcggga agcgtggcgc tttctcatag ctcacgctgt aggtatctca 3960
   gttcggtgta ggtcgttcgc tccaagctgg gctgtgtgca cgaacccccc gttcagcccg 4020
   accgctgcgc cttatccggt aactatcgtc ttgagtccaa cccggtaaga cacgacttat 4080
   cgccactggc agcagccact ggtaacagga ttagcagagc gaggtatgta ggcggtgcta 4140
   cagagttctt gaagtggtgg cctaactacg gctacactag aaggacagta tttggtatct 4200
   gcgctctgct gaagccagtt accttcggaa aaagagttgg tagctcttga tccggcaaac 4260
   aaaccaccgc tggtagcggt ggtttttttg tttgcaagca gcagattacg cgcagaaaaa 4320
   aaggatctca agaagatcct ttgatctttt ctacggggtc tgacgctcag tggaacgaaa 4380
   actcacgtta agggattttg gtcatgagat tatcaaaaag gatcttcacc tagatccttt 4440
   taaattaaaa atgaagtttt aaatcaatct aaagtatata tgagtaaact tggtctgaca 4500
   gttaccaatg cttaatcagt gaggcaccta tctcagcgat ctgtctattt cgttcatcca 4560
   tagttgcctg actccccgtc gtgtagataa ctacgatacg ggagggctta ccatctggcc 4620
   ccagtgctgc aatgataccg cgagacccac gctcaccggc tccagattta tcagcaataa 4680
   accagccagc cggaagggcc gagcgcagaa gtggtcctgc aactttatcc gcctccatcc 4740
   agtctattaa ttgttgccgg gaagctagag taagtagttc gccagttaat agtttgcgca 4800
   acgttgttgg cattgctaca ggcatcgtgg tgtcacgctc gtcgtttggt atggcttcat 4860
   tcagctccgg ttcccaacga tcaaggcgag ttacatgatc ccccatgttg tgcaaaaaag 4920
   cggttagctc cttcggtcct ccgatcgttg tcagaagtaa gttggccgca gtgttatcac 4980
   tcatggttat ggcagcactg cataattctc ttactgtcat gccatccgta agatgctttt 5040
   ctgtgactgg tgagtactca accaagtcat tctgagaata ccgcgcccgg cgaccgagtt 5100
   gctcttgccc ggcgtcaata cgggataata gtgtatgaca tagcagaact ttaaaagtgc 5160
   tcatcattgg aaaacgttct tcggggcgaa aactctcaag gatcttaccg ctgttgagat 5220
   ccagttcgat gtaacccact cgtgcaccca actgatcttc agcatctttt actttcacca 5280
   gcgtttctgg gtgagcaaaa acaggaaggc aaaatgccgc aaaaaaggga ataagggcga 5340
   cacggaaatg ttgaatactc atactcttcc tttttcaata ttattgaagc atttatcagg 5400
   gttattgtct catgagcgga tacatatttg aatgtattta gaaaaataaa caaatagggg 5460
   ttccgcgcac atttccccga aaagtgccac ctgtatgcgg tgtgaaatac cgcacagatg 5520
   cgtaaggaga aaataccgca tcaggcgaaa ttgtaaacgt taatattttg ttaaaattcg 5580
   cgttaaatat ttgttaaatc agctcatttt ttaaccaata ggccgaaatc ggcaaaatcc 5640
   cttataaatc aaaagaatag accgagatag ggttgagtgt tgttccagtt tggaacaaga 5700
   gtccactatt aaagaacgtg gactccaacg tcaaagggcg aaaaaccgtc tatcagggcg 5760
   atggcccact acgtgaacca tcacccaaat caagtttttt gcggtcgagg tgccgtaaag 5820
   ctctaaatcg gaaccctaaa gggagccccc gatttagagc ttgacgggga aagccggcga 5880
   acgtggcgag aaaggaaggg aagaaagcga aaggagcggg cgctagggcg ctggcaagtg 5940
   tagcggtcac gctgcgcgta accaccacac ccgccgcgct taa                   5983
<210> 31
   <211> 7330
   <212> DNA
   <213> Artificial Sequence
<220>
   <223> Description of Artificial Sequence:plasmid
<400> 31
   ttgcggccgc ttcgaaagct gtaatataaa aaccttcttc aactaacggg gcaggttagt 60
   gacattagaa aaccgactgt aaaaagtaca gtcggcatta tctcatatta taaaagccag 120
   tcattaggcc tatctgacaa ttcctgaata gagttcataa acaatcctgc atgataacca 180
   tcacaaacag aatgatgtac ctgtaaagat agcggtaaat atattgaatt acctttatta 240
   atgaattttc ctgctgtaat aatgggtaga aggtaattac tattattatt gatatttaag 300
   ttaaacccag taaatgaagt ccatggaata atagaaagag aaaaagcatt ttcaggtata 360
   ggtgttttgg gaaacaattt ccccgaacca ttatatttct ctacatcaga aaggtataaa 420
   tcataaaact ctttgaagtc attctttaca ggagtccaaa taccagagaa tgttttagat 480
   acaccatcaa aaattgtata aagtggctct aacttatccc aataacctaa ctctccgtcg 540
   ctattgtaac cagttctaaa agctgtattt gagtttatca cccttgtcac taagaaaata 600
   aatgcagggt aaaatttata tccttcttgt tttatgtttc ggtataaaac actaatatca 660
   atttctgtgg ttatactaaa agtcgtttgt tggttcaaat aatgattaaa tatctctttt 720
   ctcttccaat tgtctaaatc aattttatta aagttcattt gatatgcctc ctaaattttt 780
   atctaaagtg aatttaggag gcttacttgt ctgctttctt cattagaatc aatccttttt 840
   taaaagtcaa tattactgta acataaatat atattttaaa aatatcccac tttatccaat 900
   tttcgtttgt tgaactaatg ggtgctttag ttgaagaata aagaccacat taaaaaatgt 960
   ggtcttttgt gtttttttaa aggatttgag cgtagcgaaa aatccttttc tttcttatct 1020
   tgataataag ggtaactatt gaattcggta ccaagagttt gtagaaacgc aaaaaggcca 1080
   tccgtcagga tggccttctg cttaatttga tgcctggcag tttatggcgg gcgtcctgcc 1140
   cgccaccctc cgggccgttg cttcgcaacg ttcaaatccg ctcccggcgg atttgtccta 1200
   ctcaggagag cgttcaccga caaacaacag ataaaacgaa aggcccagtc tttcgactga 1260
   gcctttcgtt ttatttgatg cctggcagtt ccctactctc gcatggggag accccacact 1320
   accatcggcg ctacggcgtt tcacttctga gttcggcatg gggtcaggtg ggaccaccgc 1380
   gctactgccg ccaggcaaat tctgttttat cagaccgctt ctgcgttctg atttaatctg 1440
   tatcaggctg aaaatcttct ctcatccgcc aaaacaggat ccaattatgg cagatcaatg 1500
   agcttcacag acacaatatc agggacattt gttagttctt tcacaatttt atcttccaga 1560
   tgtctgtcaa aggaaagcat catgatggct tctccgcctt tttccttacg gccaacctgc 1620
   atagttgcaa tgttaatatc attatctccg agaatacgtc ctactcggcc gatgacacct 1680
   gttgtatctt gatgctggat atacaccaag tgaccagtcg gataaaaatc aatattaaat 1740
   ccattgatct cgacaattcg ttctccgaaa tgaggaatat acgtagccgt tacagtaaag 1800
   gtgctgcggt ctcctgtcac ttttacgctg atgcagttat cgtatccaga ttcagaagag 1860
   gaaatttttt cactgaagct aatgccgcgt tcttttgcga cacccccggc attgacctca 1920
   ttaacagtag agtctacgcg cggttttaaa aagcctgaca gaagggcttt tgtaatgaac 1980
   gatgtttcaa gtttagcaat tgtgccttca tattgaatgg caacatcctg tactggttct 2040
   ttcatgcact gtgatacaag gctgccaatt tttcctgcaa tttgatggta aggcttaatt 2100
   ttagcaaatt catcttttgt catggcaggc aggttgatag ctgacatgac aggcaggcct 2160
   tttgcgaact gcagaacttc ttctgacact tgggcggcga cattgagctg tgcttctttc 2220
   gttgatgctc ccaagtgagg agtggcaatg actaatggat gatcaacaag tttgttgtca 2280
   actggcggtt cgacttcgaa aacgtcaagc gctgctcccg caacatgccc gttttccaaa 2340
   gcttcgagaa gtgctgcttc atcgataatt ccgcctcgcg cacagttaat taagcgaacg 2400
   ccttttttcg tttttgcaat cgtttcttta ttcaataagc cttttgtttc ttttgttaaa 2460
   ggcgtgtgaa cggtaatgat atccgcactt tcaagcactt cttcaaatgt acggctgttt 2520
   acgccgattt ttttcgctct ttcttccgtt aagaaaggat caaaaacgtg cacagtcata 2580
   ccgaacgctc ctcgacgctg tgcaatttca cttccgattc ggcctaatcc tacaatacca 2640
   agcgtttttc cataaagctc tgaaccgaca taagctgtgc ggttccactc tctggatttc 2700
   actgagatat tagcctgcgg aatgtgtctc attaaagaag agatcattgc aaatgtatgc 2760
   tcagctgtcg aaatggtgtt gccgttcgga gcattgatca cgattacccc gtgtttcgta 2820
   gcctcatcaa tatcgatatt atcgacaccg acaccggctc ttccgacaat ttttaaagaa 2880
   gtcattttgt tgaaaaggtc ttctgttact tttgtcgcgc ttcgcaccaa aagagcatca 2940
   aaagtatgta attcatcttc tgcatctgct acgttttttt gaacgatttc aataaagtct 3000
   gattcaataa gtggctgtaa accgtcgttg ctcattttgt ctgagaccaa tactcgaaac 3060
   atgttttctc ctcctctaga gcgtcctgct gttgttaaga ttattatacc acaccttgta 3120
   gataaagtca acaacttttt gcaaaatttt tcaggaattt tagcagaggt tgttctggat 3180
   gtagaacaaa acatctttcc gctcttgtgc tgttaggata tctttcttgg aagctaggta 3240
   ggcctcgagt tatggcagtt ggttaaaagg aaacaaaaag accgttttca cacaaaacgg 3300
   tctttttcga tttcttttta cagtcacagc cacttttgca aaaaccggac agcttcatgc 3360
   cttataactg ctgtttcggt cgacgaaaca tcgttagatt tcctcctaaa ttgacaaact 3420
   aaatatctga taatttaaca tattctcaaa agagtgtcaa cgtgtattga cgcagtaaag 3480
   gataaaagta aagcctaata aatcaatgat ctgacagctt gcaggtaata tatttaattt 3540
   gaagcaattc tctatacagc caaccagtta tcgtttataa tgtaattaaa tttcatatga 3600
   tcaatcttcg gggcagggtg aaattcccta ccggcggtga tgagccaatg gctctaagcc 3660
   cgcgagctgt ctttacagca ggattcggtg agattccgga gccgacagta cagtctggat 3720
   gggagaagat ggaggttcat aagcgttttg aaattgaatt tttcaaacgt ttctttgcct 3780
   agcctaattt tcgaaacccc gcttttatat atgaagcggt ttttttattg gctggaaaag 3840
   aacctttccg ttttcgagta agatgtgatc gaaaaggaga gaatgaagtg aaagtaaaaa 3900
   aattagttgt ggtcagcatg caagcttcgc gaagcggccg ccgacgcgag gctggatggc 3960
   cttccccatt atgattcttc tcgcttccgg cggcatcggg atgcccgcgt tgcaggccat 4020
   gctgtccagg caggtagatg acgaccatca gggacagctt caaggatcgc tcgcggctct 4080
   taccagccta acttcgatca ctggaccgct gatcgtcacg gcgatttatg ccgcctcggc 4140
   gagcacatgg aacgggttgg catggattgt aggcgccgcc ctataccttg tctgcctccc 4200
   cgcgttgcgt cgcggtgcat ggagccgggc cacctcgacc tgaatggaag ccggcggcac 4260
   ctcgctaacg gattcaccac tccaagaatt ggagccaatc aattcttgcg gagaactgtg 4320
   aatgcgcaaa ccaacccttg gcagaacata tccatcgcgt ccgccatctc cagcagccgc 4380
   acgcggcgca tctcgggcag cgttgggtcc tggccacggg tgcgcatgat cgtgctcctg 4440
   tcgttgagga cccggctagg ctggcggggt tgccttactg gttagcagaa tgaatcaccg 4500
   atacgcgagc gaacgtgaag cgactgctgc tgcaaaacgt ctgcgacctg agcaacaaca 4560
   tgaatggtct tcggtttccg tgtttcgtaa agtctggaaa cgcggaagtc agcgccctgc 4620
   accattatgt tccggatctg catcgcagga tgctgctggc taccctgtgg aacacctaca 4680
   tctgtattaa cgaagcgctg gcattgaccc tgagtgattt ttctctggtc ccgccgcatc 4740
   cataccgcca gttgtttacc ctcacaacgt tccagtaacc gggcatgttc atcatcagta 4800
   acccgtatcg tgagcatcct ctctcgtttc atcggtatca ttacccccat gaacagaaat 4860
   tcccccttac acggaggcat caagtgacca aacaggaaaa aaccgccctt aacatggccc 4920
   gctttatcag aagccagaca ttaacgcttc tggagaaact caacgagctg gacgcggatg 4980
   aacaggcaga catctgtgaa tcgcttcacg accacgctga tgagctttac cgcagctgcc 5040
   tcgcgcgttt cggtgatgac ggtgaaaacc tctgacacat gcagctcccg gagacggtca 5100
   cagcttgtct gtaagcggat gccgggagca gacaagcccg tcagggcgcg tcagcgggtg 5160
   ttggcgggtg tcggggcgca gccatgaccc agtcacgtag cgatagcgga gtgtatactg 5220
   gcttaactat gcggcatcag agcagattgt actgagagtg caccatatgc ggtgtgaaat 5280
   accgcacaga tgcgtaagga gaaaataccg catcaggcgc tcttccgctt cctcgctcac 5340
   tgactcgctg cgctcggtcg ttcggctgcg gcgagcggta tcagctcact caaaggcggt 5400
   aatacggtta tccacagaat caggggataa cgcaggaaag aacatgtgag caaaaggcca 5460
   gcaaaaggcc aggaaccgta aaaaggccgc gttgctggcg tttttccata ggctccgccc 5520
   ccctgacgag catcacaaaa atcgacgctc aagtcagagg tggcgaaacc cgacaggact 5580
   ataaagatac caggcgtttc cccctggaag ctccctcgtg cgctctcctg ttccgaccct 5640
   gccgcttacc ggatacctgt ccgcctttct cccttcggga agcgtggcgc tttctcatag 5700
   ctcacgctgt aggtatctca gttcggtgta ggtcgttcgc tccaagctgg gctgtgtgca 5760
   cgaacccccc gttcagcccg accgctgcgc cttatccggt aactatcgtc ttgagtccaa 5820
   cccggtaaga cacgacttat cgccactggc agcagccact ggtaacagga ttagcagagc 5880
   gaggtatgta ggcggtgcta cagagttctt gaagtggtgg cctaactacg gctacactag 5940
   aaggacagta tttggtatct gcgctctgct gaagccagtt accttcggaa aaagagttgg 6000
   tagctcttga tccggcaaac aaaccaccgc tggtagcggt ggtttttttg tttgcaagca 6060
   gcagattacg cgcagaaaaa aaggatctca agaagatcct ttgatctttt ctacggggtc 6120
   tgacgctcag tggaacgaaa actcacgtta agggattttg gtcatgagat tatcaaaaag 6180
   gatcttcacc tagatccttt taaattaaaa atgaagtttt aaatcaatct aaagtatata 6240
   tgagtaaact tggtctgaca gttaccaatg cttaatcagt gaggcaccta tctcagcgat 6300
   ctgtctattt cgttcatcca tagttgcctg actccccgtc gtgtagataa ctacgatacg 6360
   ggagggctta ccatctggcc ccagtgctgc aatgataccg cgagacccac gctcaccggc 6420
   tccagattta tcagcaataa accagccagc cggaagggcc gagcgcagaa gtggtcctgc 6480
   aactttatcc gcctccatcc agtctattaa ttgttgccgg gaagctagag taagtagttc 6540
   gccagttaat agtttgcgca acgttgttgc cattgctgca ggcatcgtgg tgtcacgctc 6600
   gtcgtttggt atggcttcat tcagctccgg ttcccaacga tcaaggcgag ttacatgatc 6660
   ccccatgttg tgcaaaaaag cggttagctc cttcggtcct ccgatcgttg tcagaagtaa 6720
   gttggccgca gtgttatcac tcatggttat ggcagcactg cataattctc ttactgtcat 6780
   gccatccgta agatgctttt ctgtgactgg tgagtactca accaagtcat tctgagaata 6840
   gtgtatgcgg cgaccgagtt gctcttgccc ggcgtcaata cgggataata ccgcgccaca 6900
   tagcagaact ttaaaagtgc tcatcattgg aaaacgttct tcggggcgaa aactctcaag 6960
   gatcttaccg ctgttgagat ccagttcgat gtaacccact cgtgcaccca actgatcttc 7020
   agcatctttt actttcacca gcgtttctgg gtgagcaaaa acaggaaggc aaaatgccgc 7080
   aaaaaaggga ataagggcga cacggaaatg ttgaatactc atactcttcc tttttcaata 7140
   ttattgaagc atttatcagg gttattgtct catgagcgga tacatatttg aatgtattta 7200
   gaaaaataaa caaatagggg ttccgcgcac atttccccga aaagtgccac ctgacgtcta 7260
   agaaaccatt attatcatga cattaaccta taaaaatagg cgtatcacga ggccctttcg 7320
   tcttcaagaa                                                        7330

## Description

### Related Applications

This application is related to International Patent Application No. PCT/US02/00925, entitled "Microorganisms and Processes for Enhanced Production of Pantothenate", filed January 18, 2002 (pending), and to International Patent Application No. PCT/US00/25993, entitled "Methods and Microorganisms for Production of Panto-Compounds", filed September 21, 2000 (expired).

### Background of the Invention

Pantothenate, also known as pantothenic acid or vitamin B5, is a member of the B complex of vitamins and is a nutritional requirement for mammals, including livestock and humans (*e.g*., from food sources, as a water soluble vitamin supplement or as a feed additive). In cells, pantothenate is used primarily for the biosynthesis of coenzyme A (CoA) and acyl carrier protein (ACP). These coenzymes function in the metabolism of acyl moieties which form thioesters with the sulfhydryl group of the 4'-phosphopantetheine portion of these molecules. These coenzymes are essential in all cells, participating in over 100 different intermediary reactions in cellular metabolism.

The conventional means of synthesizing pantothenate (in particular, the bioactive D isomer) is *via* chemical synthesis from bulk chemicals, a process which is hampered by excessive substrate cost as well as the requirement for optical resolution of racemic intermediates. Accordingly, researchers have recently looked to bacterial or microbial systems that produce enzymes useful in pantothenate biosynthesis processes (as bacteria are themselves capable of synthesizing pantothenate). In particular, bioconversion processes have been evaluated as a means of favoring production of the preferred isomer of pantothenic acid. Moreover, methods of direct microbial synthesis have recently been examined as a means of facilitating D-pantothenate production.

There is still, however, significant need for improved pantothenate production processes, in particular, for microbial processes optimized to produce higher yields of desired product.

### Summary of the Invention

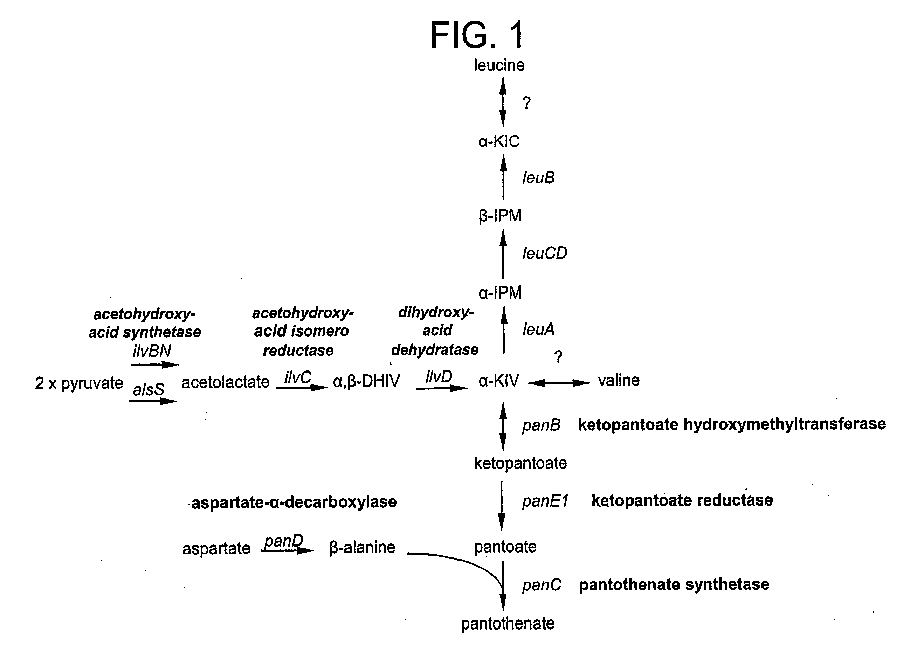

The present invention relates to improved processes (*e.g*., microbial syntheses) for the production of pantothenate. Pantothenate production processes have been described in related applications which feature, for example, microbes engineered to overexpress key enzymes of the pantothenate biosynthetic pathway and the isoleucine-valine biosynthetic pathway (see *e.g*., Figure 1). Strains have been engineered that are capable of producing > 50 g/l of pantothenate in standard fermentation processes (see *e.g*., International Public. No. WO 01/21772 and U.S. Patent Application No. 60/262,995). In particular, increasing the expression of the *panB, panC, panD* and *panE1* genes and increasing the expression of the *ilvBNC* and *ilvD* genes results in strains that convert glucose (pyruvate) to commercially attractive quantities of pantothenate.

In order to enhance production levels of for example, pantothenate, various improvements on the above-described methods have now been developed. For example, U.S. Patent Application Serial No. 09/667,569 describes production strains having modified (*e.g*., deleted or decreased-activity) pantothenate kinase enzymes. In such strains, the pantothenate levels are effectively increased by decreasing utilization of pantothenate for coenzyme A ("CoA") synthesis. U.S. Patent Application Serial No. 60/262,995 further describes improved pantothenate-production strains that have been engineered to minimize utilization of various pantothenate biosynthetic enzymes and/or isoleucine-valine biosynthetic enzymes and/or their respective substrates from being used to produce an alternative product identified as [R]-3-(2-hydroxy-3-methyl-butyrylamino)-propionic acid ("HMBPA").

The present invention features methods to further enhance pantothenate production by modulating a biosynthetic pathway that supplies a substrate for the pantothenate biosynthetic pathway, namely the methylenetetrahydrofolate ("MTF") biosynthetic pathway. In particular, it has been discovered that increasing levels of MTF by modification of the MTF biosynthetic pathway results in enhanced levels of the key pantothenate biosynthetic pathway intermediate, ketopantoate. Enhanced ketopantoate. levels, in turn, result in significantly enhanced pantothenate production levels in appropriately engineered strains. In essence, the present inventors have identified a limiting step in the production of panto-compounds (*e.g*., pantothenate) by strains engineered to overexpress, for example, the *panB, panC, panD, panE1*, *ilvBNC* and *ilvD* genes, and describe herein a means for overcoming this limitation by modification of the MTF biosynthetic pathway.

At least three effective means of modifying the MTF biosynthetic pathway are described herein. In one aspect, it has been demonstrated that increasing serine levels in the culture medium of pantothenate-producing microorganisms results in enhanced panto-compound production. It has also been demonstrated that increasing the synthesis or activity of 3-phosphoglycerate dehydrogenase (the *serA* gene product), or the synthesis or activity of serine hydroxymethyl transferase (the *glyA* gene product), thereby enhancing serine and methylenetetrahydrofolate biosynthesis in appropriately engineered microorganisms, increases panto-compound production. Increased synthesis of 3-phosphoglycerate dehydrogenase (the *serA* gene product) is achieved, for example, by overexpressing *serA* from an appropriately-engineered expression cassette. Increased synthesis of serine hydroxymethyl transferase (the *glyA* gene product) is achieved, for example, by overexpressing *glyA* from an appropriately-engineered expression cassette. Alternatively, levels of serine hydroxymethyl transferase (the *glyA* gene product) are increased by altering the regulation of the *glyA* gene. For example, mutation or deletion of the gene encoding a negative regulator (*i.e*., repressor) *of glyA* expression, the *purR* gene, effectively increases *glyA* expression. Additional methods suitable for increasing MTF levels in panto-compound producing microoganisms involve deregulating enzymes responsible for converting glycine to MTF (*e.g.*, glycine cleavage enzymes).

Accordingly, in one aspect the invention features processes for the enhanced production of pantoate and pantothenate that involve culturing microorganisms having modified pantothenate biosynthetic enzyme activities and having modified methylenetetrahydrofolate (MTF) biosynthetic enzyme activities under conditions such that pantothenate production is enhanced. In another aspect the invention features processes for the enhanced production of pantoate and pantothenate that involve culturing microorganisms having modified pantothenate biosynthetic enzyme activities, having modified isoleucine-valine (*ilv*) biosynthetic enzymes, and having modified methylenetetrahydrofolate (MTF) biosynthetic enzyme activities under conditions such that pantothenate production is enhanced. In particular, the invention features methods for enhancing production of desired products (*e.g*., pantoate and/or pantothenate) by increasing the levels of a key intermediate, ketopantoate, by enzymes that contribute to its synthesis. Preferred methods result in production of pantothenate at levels greater than 50, 60, 70 or more g/L after 36 hours of culturing the microorganisms, or such that at least 60, 70, 80, 90, 100, 110, 120 or more g/L pantothenate is produced after 36 hours of culturing the microorganisms. Recombinant microorganism and conditions for culturing same are also are featured.

Other features and advantages of the invention will be apparent from the following detailed description and claims.

### Brief Description of the Drawings

*Figure 1* is a schematic representation of the pantothenate and isoleucine-valine (*ilv*) biosynthetic pathways. Pantothenate biosynthetic enzymes are depicted in bold and their corresponding genes indicated in italics. Isoleucine-valine (*ilv*) biosynthetic enzymes are depicted in bold italics and their corresponding genes indicated in italics.
*Figure 2* is a schematic representation of the methylenetetrahydrofolate ("MTF") biosynthetic pathway in *E*. *coli* (and presumably in *B. subtilis*).
*Figure 3* is a schematic representation of the construction of the plasmid pAN665.
*Figure 4* is a schematic representation of the construction of the plasmid pAN670.
*Figure 5* is a schematic representation of the plasmid pAN004.
*Figure 6* is a schematic representation of the plasmid pAN396.
*Figure* 7 is a schematic representation of the plasmid pAN393.
*Figure 8* is a schematic representation of the structure of pAN835F, a clone of the *B. subtilis purR* gene.
*Figure 9* is a schematic representation of the structure of pAN838F, a plasmid designed to install a disruption of the *B. subtilis purR* gene.
*Figure 10* is a schematic representation of the structure of pAN821, a plasmid designed to delete a portion of the serA gene, selecting for kanamycin resistance.
*Figure 11* is a schematic representation of the structure of pAN824, a plasmid designed to integrate a non-amplifiable *P₂₆ serA* cassette at the *serA* locus, selecting for *Ser*⁺.
*Figure 12* is a schematic representation of the structure of pAN395, a medium copy plasmid designed to integrate and amplify a *P26 serA* expression cassette at the *serA* locus.

### Detailed Description of the Invention

The present invention is directed to improved methods for producing panto-compounds (*e.g*., ketopantoate, pantoate and/or pantothenate) and strains engineered for use in said improved methods. Strains capable of producing > 50 g/l of pantothenate can be constructed as taught in International Patent Application Serial No. WO 01/21772 and in U.S. Patent Application Serial No. 60/262,995. By increasing the expression of the *panB, panC, panD* and *panE1* genes and by increasing the expression of the *ilvBNC* and *ilvD* genes, one can design strains (*e.g., Bacillus* strains) that convert glucose (pyruvate) to commercially attractive quantities of pantothenate. Processes for enhanced production of pantothenate are shown for example in WO02/057474, WO03/004672 and EP1390519.

However, it has now been discovered that in strains engineered to express high levels of the *panB* gene product, ketopantoate hydroxymethyltransferase *(e.g.,* PA824, described in U.S. Patent Application Serial No. 09/667,569 and PA668-24, described in U.S. Patent Application Serial No. 60/262,995), a limiting step for further increases in the production of pantothenate is still the conversion of α-ketoisovalerate (α-KIV) to ketopantoate. Methods to increase the synthesis of α-KIV were described previously in International Patent Application Serial No. WO 01/21772 and U.S. Patent Application Serial No. 60/262,995. Here we disclose that even further increases in pantothenate production can be achieved by engineering panto-compound producing microorganisms such that the level of MTF, or the rate of MTF synthesis is enhanced or increased.

Accordingly, the present invention features methods for improving panto-compound production that involve modulating the methylenetetrahydrofolate ("MTF") biosynthetic pathway. In particular, increasing MTF levels in panto-compound producing microbes is an effective means of enhancing ketopantoate production, and in turn results in enhanced pantoate and/or pantothenate production in appropriately-engineered recombinant microorganisms.

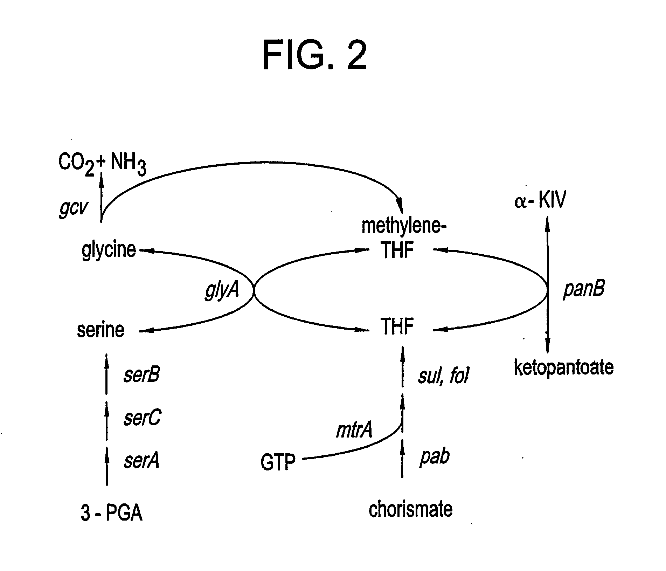

Ketopantoate hydroxymethylenetransferase catalyzes the production of ketopantoate from α-ketoisovalerate ("α-KIV") and MTF (see *e.g*., Figure 1). In particular, the enzyme catalyzes the transfer of a hydroxymethyl group from MTF to α-KIV to yield ketopantoate. Both α-KIV and MTF are substrates for this reaction, and their syntheses can be increased in order to improve production of ketopantoate. The pathway for MTF biosynthesis in *E*. *coli* (and also in *Bacillus subtilis*) is outlined in Figure 2. MTF is synthesized from tetrahydrofolate and serine in a reaction catalyzed by the *glyA* gene that encodes serine hydroxymethyl transferase. For improved MTF synthesis the cells need increased quantities of both substrates and the product of the *glyA* gene. In one embodiment, the invention features a process for the enhanced production of pantothenate as compared to pantothenate production in said microorganism prior to deregulation, comprising culturing a microorganism having a deregulated methylenetetrahydrofolate (MTF) biosynthetic pathway, under conditions such that pantothenate production is enhanced, wherein the microorganism has a deregulated serine hydroxymethyltransferase gene and a mutated, deleted or disrupted purine repressor gene modulating repression of the serine hydroxymethyltransferase gene.

In one embodiment, the invention features processes for the enhanced production of pantothenate that involve culturing a microorganism having (i) a deregulated pantothenate biosynthetic pathway (*e.g*., having one, two, three or four pantothenate biosynthetic enzymes deregulated) and (ii) a deregulated methylenetethrhydrofolate (MTF) biosynthetic pathway (*e.g*., having at least one or two MTF biosynthetic enzymes deregulated), under conditions such that pantothenate production is enhanced. Exemplary pantothenate biosynthetic enzymes include ketopantoate hydroxymethyltransferase, ketopantoate reductase, pantothenate synthetase and aspartate-α-decarboxylase. Exemplary "MTF biosynthetic enzymes include the *serA* gene product and the *glyA* gene product

In another embodiment, the invention features processes for the enhanced production of pantothenate that involve culturing a microorganism having (i) a deregulated pantothenate biosynthetic pathway (*e.g*., having one, two, three or four pantothenate biosynthetic enzymes deregulated), (ii) a deregulated isoleucine-valine (*ilv*) biosynthetic pathway (*e.g*., having one, two or three *ilv* biosynthetic enzymes deregulated), and (iii) a deregulated MTF biosynthetic pathway (*e.g*., having at least one or two MTF biosynthetic enzymes deregulated), under conditions such that pantothenate production is enhanced. Exemplary *ilv* biosynthetic enzymes include acetohydroxyacid acid synthetase, acetohydroxyacid isomeroreductase, and dihydroxyacid dehydratase.

In another embodiment, the invention features processes for the production of pantothenate that involve culturing a microorganism having a deregulated pantothenate biosynthetic pathway, a deregulated *ilv* biosynthetic pathway, and a deregulated MTF biosynthetic pathway, such that at least 50 g/L pantothenate is produced after 36 hours of culturing the microorganism, preferably such that at least 60 g/L pantothenate is produced after 36 hours of culturing the microorganism, more preferably such that at least 70 g/L pantothenate is produced after 36 hours of culturing the microorganism, and most preferably such that at least 80 g/L pantothenate, at least 90 g/L pantothenate, at least 100 g/L pantothenate, at least 110 g/L pantothenate, or at least 120 g/L pantothenate (or more) is produced after 3 6 hours of culturing the microorganism.

In another embodiment, the invention features processes for the production of pantothenate that involve culturing a microorganism having a deregulated pantothenate biosynthetic pathway, a deregulated *ilv* biosynthetic pathway, and a deregulated MTF biosynthetic pathway, deregulated such that at least 70 g/L pantothenate is produced after 48 hours of culturing the microorganism, preferably such that at least 80 g/L pantothenate is produced after 48 hours of culturing the microorganism, and more preferably such that at least 90 g/L pantothenate is produced after 48 hours of culturing the microorganism.

In one exemplary embodiment, deregulation of the MTF biosynthetic pathway is achieved by deregulating the *serA* gene product in a panto-compound producing strain, for example, by expressing the *serA* gene constitutively or by introducing a feedback resistant allele of *serA.* In another exemplary embodiment, deregulation of the MTF biosynthetic pathway is achieved by deregulating the *glyA* gene product in a panto-compound producing strain, for example, by overexpressing the *glyA* gene or modulating repression of the *glyA* gene by mutating or disrupting the *purR* gene product. In other exemplary embodiments, MTF biosynthesis is modulated by increasing serine in the culture medium or deregualting glycine cleavage enzymes.

The invention further features methods as described above, wherein pantothenate production is further enhanced by regulating pantothenate kinase activity (*e.g*., wherein pantothenate kinase activity is decreased). In one embodiment, CoaA is deleted and CoaX is downregulated. In another embodiment, CoaX is deleted and CoaA is downregulated. In yet another embodiment, CoaX and CoaA are downregulated. The invention further features methods as described above, wherein the microorganisms are cultured under conditions of excess serine. The invention further features methods as described above, wherein the microorganisms have the pantothenate biosynthetic pathway deregulated such that pantothenate production is independent of β-alanine feed.

Recombinant microorganisms for use in the processes of the invention are also featured. In one embodiment, the invention features a recombinant microorganism for the enhanced production of pantothenate having a deregulated pantothenate biosynthetic pathway and a deregulated MTF biosynthetic pathway. In another embodiment, the invention features a recombinant microorganism for the enhanced production of pantothenate having a deregulated pantothenate biosynthetic pathway, a deregulated MTF biosynthetic pathway and a deregulated *ilv* pathway. Microorganisms can further have reduced pantothenate kinase activity. Preferred microorganisms belong to the genus *Bacillus,* for example *Bacillus subtilis.*

As described above, certain aspects of the invention feature processes for the enhanced production of panto-compounds (*e.g.,* pantoate and/or pantothenate) that involve culturing microorganisms having at least a deregulated pantothenate biosynthetic pathway. The term "pantothenate biosynthetic pathway" includes the biosynthetic pathway involving pantothenate biosynthetic enzymes (*e.g.*, polypeptides encoded by biosynthetic enzyme-encoding genes), compounds (*e.g.*, substrates, intermediates or products), cofactors and the like utilized in the formation or synthesis of pantothenate. The term "pantothenate biosynthetic pathway" includes the biosynthetic pathway leading to the synthesis of pantothenate in microorganism (*e.g*., *in vivo*) as well as the biosynthetic pathway leading to the synthesis of pantothenate *in vitro.*

As used herein, a microorganism "having a deregulated pantothenate biosynthetic pathway" includes a microorganism having at least one pantothenate biosynthetic enzyme deregulated (*e.g.*, overexpressed) (both terms as defined herein) such that pantothenate production is enhanced (*e.g*., as compared to pantothenate production in said microorganism prior to deregulation of said biosynthetic enzyme or as compared to a wild-type microorganism). The term "pantothenate" includes the free acid form of pantothenate, also referred to as "pantothenic acid" as well as any salt thereof (*e.g*., derived by replacing the acidic hydrogen of pantothenate or pantothenic acid with a cation, for example, calcium, sodium, potassium, ammonium, magnesium), also referred to as a "pantothenate salt". The term "pantothenate" also includes alcohol derivatives of pantothenate. Preferred pantothenate salts are calcium pantothenate or sodium pantothenate. A preferred alcohol derivative is pantothenol. Pantothenate salts and/or alcohols of the present invention include salts and/or alcohols prepared *via* conventional methods from the free acids described herein. In another embodiment, a pantothenate salt is synthesized directly by a microorganism of the present invention. A pantothenate salt of the present invention can likewise be converted to a free acid form of pantothenate or pantothenic acid by conventional methodology. The term "pantothenate" is also abbreviated as "pan" herein.

Preferably, a microorganism "having a deregulated pantothenate biosynthetic pathway" includes a microorganism having at least one pantothenate biosynthetic enzyme deregulated (*e.g.*, overexpressed) such that pantothenate production is I g/L or greater. More preferably, a microorganism "having a deregulated pantothenate biosynthetic pathway" includes a microorganism having at least one pantothenate biosynthetic enzyme deregulated (*e.g.*, overexpressed) such that pantothenate production is 2 g/L or greater. Even more preferably, a microorganism "having a deregulated pantothenate biosynthetic pathway" includes a microorganism having at least one pantothenate biosynthetic enzyme deregulated (*e.g.*, overexpressed) such that pantothenate production is 10 g/L, 20 g/L,30 g/L,40 g/L, 50 g/L, 60 g/L, 70 g/L, 80 g/L, 90 g/L, or greater.

The term "pantothenate biosynthetic enzyme" includes any enzyme utilized in the formation of a compound (*e.g*., intermediate or product) of the pantothenate biosynthetic pathway. For example, synthesis of pantoate from α-ketoisovalerate (α-KIV) proceeds *via* the intermediate, ketopantoate. Formation of ketopantoate is catalyzed by the pantothenate biosynthetic enzyme PanB or ketopantoate hydroxymethyltransferase (the *panB* gene product). Formation ofpantoate is catalyzed by the pantothenate biosynthetic enzyme PanE 1 or ketopantoate reductase (the *panE1* gene product). Synthesis of β-alanine from aspartate is catalyzed by the pantothenate biosynthetic enzyme PanD or aspartate-α-decarboxylase (the *panD* gene product). Formation of pantothenate from pantoate and β-alanine (*e.g.*, condensation) is catalyzed by the pantothenate biosynthetic enzyme PanC or pantothenate synthetase (the *pan*C gene product). Pantothenate biosynthetic enzymes may also perform an alternative function as enzymes in the HMBPA biosynthetic pathway described herein.

Accordingly, in one embodient, the invention features a process for the enhanced production of pantothenate that includes culturing a microorganism having at least one pantothenate biosynthetic enzyme deregulated (*e.g*., deregulated such that pantothenate production is enhanced), said enzyme being selected, for example, from the group consisting of PanB (or ketopantoate hydroxymethyltransferase), PanC (or pantothenate synthetase), PanD (or aspartate-α-decarboxylase), PanE1 (or ketopantoate reductase). In another embodiment, the invention features a process for the enhanced production of pantothenate that includes culturing a microorganism having at least two pantothenate biosynthetic enzymes deregulated, said enzymes being selected, for example, from the group consisting of PanB (or ketopantoate hydroxymethyltransferase), PanC (or pantothenate synthetase), PanD (or aspartate-α-decarboxylase), and PanE1 (or ketopantoate reductase). In another embodiment, the invention features a process for the enhacned production of pantothenate that includes culturing a microorganism having at least three pantothenate biosynthetic enzymes deregulated, said enzymes being selected, for example, from the group consisting of PanB (or ketopantoate hydroxymethyltransferase), PanC (or pantothenate synthetase), PanD (or aspartate-α-decarboxylase), and PanE1 (or ketopantoate reductase). In another embodiment, the invention features a process for the enhanced production of pantothenate that includes culturing a microorganism having at least four pantothenate biosynthetic enzymes deregulated, for example, a microorganism having PanB (or ketopantoate hydroxymethyltransferase), PanC (or pantothenate synthetase), PanD (or aspartate-α-decarboxylase), and PanE1 (or ketopantoate reductase) deregulated.

In another aspect, the invention features processes for the enhanced production of pantothenate that involve culturing microorganisms having a deregulated isoleucine-valine biosynthetic pathway. The term "isoleucine-valine biosynthetic pathway" includes the biosynthetic pathway involving isoleucine-valine biosynthetic enzymes (*e.g*., polypeptides encoded by biosynthetic enzyme-encoding genes), compounds (*e.g.,* substrates, intermediates or products), cofactors and the like utilized in the formation or synthesis of conversion of pyruvate to valine or isoleucine. The term "isoleucine-valine biosynthetic pathway" includes the biosynthetic pathway leading to the synthesis of valine or isoleucine in microorganisms (*e.g, in vivo*) as well as the biosynthetic pathway leading to the synthesis of valine or isoleucine *in vitro*.

As used herein, a microorganism "having a deregulated isoleucine-valine (*ilv*) pathway" includes a microorganism having at least one isoleucine-valine (*ilv*) biosynthetic enzyme deregulated (*e.g*., overexpressed) (both terms as defined herein) such that isoleucine and/or valine and/or the valine precursor, α-ketoisovaerate (α-KIV) production is enhanced (*e.g.*, as compared to isoleucine and/or valine and/or α-KIV production in said microorganism prior to deregulation of said biosynthetic enzyme or as compared to a wild-type microorganism). Figure 1 includes a schematic representation of the isoleucine-valine biosynthetic pathway. Isoleucine-valine biosynthetic enzymes are depicted in bold italics and their corresponding genes indicated in italics. The term "isoleucine-valine biosynthetic enzyme" includes any enzyme utilized in the formation of a compound (*e.g*., intermediate or product) of the isoleucine-valine biosynthetic pathway. According to Figure 1, synthesis of valine from pyruvate proceeds *via* the intermediates, acetolactate, α,β-dihydroxyisovalerate (α,β-DHIV) and α-ketoisovalerate (α-KIV). Formation of acetolactate from pyruvate is catalyzed by the isoleucine-valine biosynthetic enzyme acetohydroxyacid synthetase (the *ilvBN* gene products, or alternatively, the *alsS* gene product). Formation of α,β-DHIV from acetolactate is catalyzed by the isoleucine-valine biosynthetic enzyme acetohydroxyacid isomeroreductase (the *ilvC* gene product). Synthesis of α-KIV from α,β-DHIV is catalyzed by the isoleucine-valine biosynthetic enzyme dihydroxyacid dehydratase (the *ilvD* gene product). Moreover, valine and isoleucine can be interconverted with their respective α-keto compounds by branched chain amino acid transaminases. Isoleucine-valine biosynthetic enzymes may also perform an alternative function as enzymes in the HMBPA biosynthetic pathway described herein.

Accordingly, in one embodient, the invention features a process for the enhanced production of pantothenate that includes culturing a microorganism having at least one isoleucine-valine (*ilv*) biosynthetic enzyme deregulated (*e.g.*, deregulated such that valine and/or isoleucine and/or α-KIV production is enhanced), said enzyme being selected, for example, from the group consisting of IlvBN, AlsS (or acetohydroxyacid synthetase), IlvC (or acetohydroxyacid isomeroreductase) and IlvD (or dihydroxyacid dehydratase). In another embodiment, the invention features a process for the enhanced production of pantothenate that includes culturing a microorganism having at least two isoleucine-valine (*ilv*) biosynthetic enzymes deregulated, said enzyme being selected, for example, from the group consisting of IlvBN, AlsS (or acetohydroxyacid synthetase), IlvC (or acetohydroxyacid isomeroreductase) and IlvD (or dihydroxyacid dehydratase). In another embodiment, the invention features a process for the enhanced production of pantothenate that includes culturing a microorganism having at least three isoleucine-valine (*ilv*) biosynthetic enzymes deregulated, for example, said microorganism having IlvBN or AlsS (or acetohydroxyacid synthetase), IlvC (or acetohydroxyacid isomeroreductase) and IlvD (or dihydroxyacid dehydratase) deregulated.

As mentioned herein, enzymes of the pantothenate biosynthetic pathway and/or the isoleucine-valine (*ilv*) pathway have been discovered to have an alternative activity in the synthesis of [R]-3-(2-hydroxy-3-methyl-butyrylamino)-propionic acid ("HMBPA") or the [R]-3-(2-hydroxy-3-methyl-butyrylamino)-propionic acid ("HMBPA") biosynthetic pathway. The term "[R]-3-(2-hydroxy-3-methyl-butyrylamino)-propionic acid ("HMBPA") biosynthetic pathway" includes the alternative biosynthetic pathway involving biosynthetic enzymes and compounds (*e.g*., substrates and the like) traditionally associated with the pantothenate biosynthetic pathway and/or isoleucine-valine *(ilv)* biosynthetic pathway utilized in the formation or synthesis of HMBPA. The term "HMBPA biosynthetic pathway" includes the biosynthetic pathway leading to the synthesis of HMBPA in microorganisms (*e.g., in vivo*) as well as the biosynthetic pathway leading to the synthesis of HMBPA *in vitro.*

The term "HMBPA biosyntheric enzyme" includes any enzyme utilized in the formation of a compound (*e.g.*, intermediate or product) of the HMBPA biosynthetic pathway. For example, synthesis of 2-hydroxyisovaleric acid (α-HIV) from α-ketoisovalerate (α-KIV) is catalyzed by the *panE1* or *panE2* gene product (PanE1 is alternatively referred to herein as ketopantoate reductase) and/or is catalyzed by the *ilvC* gene product (alternatively referred to herein as acetohydroxyacid isomeroreductase). Formation of HMBPA from β-alanine and α-HIV is catalyzed by the *panC* gene product (alternatively referred to herein as pantothenate synthetase).

The term "[R]-3-(2-hydroxy-3-methyl-butyrylamino)-propionic acid ("HMBPA")" includes the free acid form of HMBPA, also referred to as "[R]-3-(2-hydroxy-3-methyl-butyrylamino)-propionate" as well as any salt thereof (*e.g*., derived by replacing the acidic hydrogen of 3-(2-hydroxy-3-methyl-butyrylamino)-propionic acid or 3-(2-hydroxy-3-methyl-butyrylamino)-propionate with a cation, for example, calcium, sodium, potassium, ammonium, magnesium), also referred to as a "3-(2-hydroxy-3-methyl-butyrylamino)-propionic acid salt" or "HMBPA salt". Preferred HMBPA salts are calcium HMBPA or sodium HMBPA. HMBPA salts of the present invention include salts prepared *via* conventional methods from the free acids described herein. An HMBPA salt of the present invention can likewise be converted to a free acid form of 3-(2-hydroxy-3-methyl-butyrylamino)-propionic acid or 3-(2-hydrohy-3-methyl-butyrylamino)-propionate by conventional methodology.

In preferred embodiments, the invention features processes for the enhanced production of panto-compounds (*e.g*., pantoate and/or pantothenate) that involve culturing a microorganism having a deregulated methylenetetrahydrofolate (MTF) biosynthetic pathway. The term "methylenetetrahydrofolate (MTF) biosynthetic pathway" refers to the biosynthetic pathway involving MTF biosynthetic enzymes (*e.g*., polypeptides encoded by biosynthetic enzyme-encoding genes), compounds (*e.g.*, substrates, intermediates or products), cofactors and the like utilized in the formation or synthesis of the PanB substrate, MTF. The term "methylenetetrahydrofolate (MTF) biosynthetic pathway" refers to the biosynthetic pathway leading to the synthesis of MTF *in vivo* (*e.g*., the pathway in *E. coli,* as depicted in Figure 2) as well as the biosynthetic pathway leading to the synthesis of MTF *in vitro.* The term "methylenetetrahydrofolate (MTF) biosynthetic enzyme" includes any enzyme utilized in the formation of a compound (*e.g.*, intermediate or product) of the methylenetetrahydrofolate (MTF) biosynthetic pathway.

The present invention is based, at least in part, on the discovery that deregulation of certain MTF biosynthetic enzymes results in enhanced production of MTF. A MTF biosynthetic enzyme, the deregulation of which results in enhanced MTF production, is termed a "MTF biosynthesis-enhancing enzyme". Exemplary "MTF biosynthesis-enhancing enzymes" are the *serA* gene product (3-phosphoglycerate dehydrogenase) and the *glyA* gene product (serine hydroxymethyl transferase). A microorganism "having a deregulated methylenetetrahydrofolate (MTF) biosynthetic pathway", is a microorganism having at least one MTF biosynthesis-enhancing enzyme deregulated (*e.g.*, overexpressed) such that MTF production or biosynthesis is enhanced (*e.g*., as compared to MTF production in said microorganism prior to deregulation of said biosynthetic enzyme or as compared to a wild-type microorganism).

In one embodiment, the invention features a process for the enhanced production of panto-compounds (*e.g.*, pantoate and/or pantothenate) that includes culturing a microorganism having a deregulated "methylenetetrahydrofolate (MTF) biosynthetic pathway", as defined herein. In another embodiment, the invention features a process for the enhanced production of panto-compounds (*e.g*., pantoate and/or pantothenate) that includes culturing a microorganism having a deregulated MTF biosynthesis-enhancing enzyme. In preferred embodiments, the invention features processes for the enhanced production of panto-compounds (*e.g.*, pantoate and/or pantothenate) that includes culturing a microorganism having a deregulated *glyA* gene product (serine hydroxymethyl transferase) and/or a deregulated *serA* gene product (3-phosphoglycerate dehydrogenase).

Yet another aspect of the present invention features processes for the enhanced production of pantothenate that include culturing microorganisms under culture conditions selected to favor pantothenate production, for example, by culturing microorganisms with excess serine (a *glyA* substrate) in the medium. The term "excess serine" includes serine levels increased or higher that those routinely utilized for culturing the microorganism in question. For example, culturing the *Bacillus* microorganisms described in the instant Examples is routinely done in the presence of about 0-2.5 g/L serine. Accordingly, excess serine levels can include levels of greater than 2.5 g/L serine, for example, between about 2.5 and 10 g/L serine. Excess serine levels can include levels of greater than 5 g/L serine, for example, between about 5 and 10 g/L serine.

Yet another aspect of the present invention features culturing the microorganisms described herein under conditions such that pantothenate production is further increased, for example, by increasing pantothenate and/or isoleucine-valine *(ilv)* biosynthetic pathway precursors and/or intermediates as defined herein (*e.g.,* culturing microorganisms in the presence of excess β-alanine, valine and/or α-KIV) or, alternatively, further modifying said microorganisms such that they are capable of producing significant levels of β-alanine in the absence of a β-alanine feed (*i.e*., β-alanine independent microorganisms, as described in U.S. Patent Application Serial No. 09/09/667,569).

Yet another aspect of the invention features further regulating pantothenate kinase activity in pantothenate-producing strains such that pantothenate production is enhanced. Pantothenate kinase is a key enzyme catalyzing the formation of Coenzyme A (CoA) from pantothenate (see *e.g*., U.S. Patent Application Serial No. 09/09/667,569). Regulation of pantothenate kinase (*e.g*., decreasing the activity or level of pantothenate kinase) reduces the production of CoA, favoring pantothenate accumulation. In one embodiment, pantotheante kinase activity is decreased by deleting CoaA and downregulating CoaX activity (CoaA and CoaX are both capable of catalyzing the first step in CoA biosynthesis in certain microorganisms). In another embodiment, pantothenate kinase activity is decreased by deleting CoaX and downregulating CoaA. In yet another embodiment, pantotheante kinase activity is decreased by downregulating CoaA and CoaX activities.

Various aspects of the invention are described in further detail in the following subsections.

### I. Targeting Genes Encoding Various Pantothenate and/or Isoleucine-Valine(ilv) and/or Methylnetetrahydrofolate (MTF) Biosynthetic Enzyme

In one embodiment, the present invention features modifying or increasing the level of various biosynthetic enzymes of the pantothenate and/or isoleucule-valine(*ilv*) and/or methylenetetrahydrofolate (MTF) biosynthetic pathways. In particular, the invention features modifying various enzymatic activities associated with said pathways by modifying or altering the genes encoding said biosynthetic enzymes.

The term "gene", as used herein, includes a nucleic acid molecule (*e.g.*, a DNA molecule or segment thereof) that, in an organism, can be separated from another gene or other genes, by intergenic DNA (*i.e*., intervening or spacer DNA which naturally flanks the gene and/or separates genes in the chromosomal DNA of the organism). Alternatively, a gene may slightly overlap another gene (*e.g.*, the 3' end of a first gene overlapping the 5' end of a second gene), the overlapping genes separated from other genes by intergenic DNA. A gene may direct synthesis of an enzyme or other protein molecule (*e.g*., may comprise coding seqeunces, for example, a contiguous open reading frame (ORF) which encodes a protein) or may itself be functional in the organism. A gene in an organism, may be clustered in an operon, as defined herein, said operon being separated from other genes and/or operons by the intergenic DNA. An "isolated gene", as used herein, includes a gene which is essentially free of sequences which naturally flank the gene in the chromosomal DNA of the organism from which the gene is derived (*i.e.,* is free of adjacent coding sequences that encode a second or distinct protein, adjacent structural sequences or the like) and optionally includes 5' and 3' regulatory sequences, for example promoter sequences and/or terminator sequences. In one embodiment, an isolated gene includes predominantly coding sequences for a protein (*e.g.*, sequences which encode *Bacillus* proteins). In another embodiment, an isolated gene includes coding sequences for a protein (*e.g.*, for a *Bacillus* protein) and adjacent 5' and/or 3' regulatory sequences from the chromosomal DNA of the organism from which the gene is derived (*e.g.*, adjacent 5' and/or 3' *Bacillus* regulatory sequences). Preferably, an isolated gene contains less than about 10 kb, 5 kb, 2 kb, 1 kb, 0.5 kb, 0.2 kb, 0.1 kb, 50 bp, 25 bp or 10 bp of nucleotide sequences which naturally flank the gene in the chromosomal DNA of the organism from which the gene is derived.

The term "operon" includes at least two adjacent genes or ORFs, optionally overlapping in sequence at either the 5' or 3' end of at least one gene or ORF. The term "operon" includes a coordinated unit of gene expression that contains a promoter and possibly a regulatory element associated with one or more adjacent genes or ORFs (*e.g.*, structural genes encoding enzymes, for example, biosynthetic enzymes). Expression of the genes (*e.g*., structural genes) can be coordinately regulated, for example, by regulatory proteins binding to the regulatory element or by anti-termination of transcription. The genes of an operon (*e.g.*, structural genes) can be transcribed to give a single mRNA that encodes all of the proteins.

A "gene having a mutation" or "mutant gene" as used herein, includes a gene having a nucleotide sequence which includes at least one alteration (*e.g.*, substitution, insertion, deletion) such that the polypeptide or protein encoded by said mutant exhibits an activity that differs from the polypeptide or protein encoded by the wild-type nucleic acid molecule or gene. In one embodiment, a gene having a mutation or mutant gene encodes a polypeptide or protein having an increased activity as compared to the polypeptide or protein encoded by the wild-type gene, for example, when assayed under similar conditions (*e.g.*, assayed in microorganisms cultured at the same temperature). As used herein, an "increased activity" or "increased enzymatic activity" is one that is at least 5% greater than that of the polypeptide or protein encoded by the wild-type nucleic acid molecule or gene, preferably at least 5-10% greater, more preferably at least 10-25% greater and even more preferably at least 25-50%, 50-75% or 75-100% greater than that of the polypeptide or protein encoded by the wild-type nucleic acid molecule or gene. Ranges intermediate to the above-recited values, *e.g.*, 75-85%, 85-90%, 90-95%, are also intended to be encompassed by the present invention. As used herein, an "increased activity" or "increased enzymatic activity" can also include an activity that is at least 1.25-fold greater than the activity of the polypeptide or protein encoded by the wild-type gene, preferably at least 1.5-fold greater, more preferably at least 2-fold greater and even more preferably at least 3-fold, 4-fold, 5-fold, 10-fold, 20-fold, 50-fold, 100-fold greater than the activity of the polypeptide or protein encoded by the wild-type gene.

In another embodiment, a gene having a mutation or mutant gene encodes a polypeptide or protein having a reduced activity as compared to the polypeptide or protein encoded by the wild-type gene, for example, when assayed under similar conditions (*e.g.*, assayed in microorganisms cultured at the same temperature). A mutant gene also can encode no polypeptide or have a reduced level of production of the wild-type polypeptide. As used herein, a "reduced activity" or "reduced enzymatic activity" is one that is at least 5% less than that of the polypeptide or protein encoded by the wild-type nucleic acid molecule or gene, preferably at least 5-10% less, more preferably at least 10-25% less and even more preferably at least 25-50%, 50-75% or 75-100% less than that of the polypeptide or protein encoded by the wild-type nucleic acid molecule or gene. Ranges intermediate to the above-recited values, *e.g,* 75-85%, 85-90%, 90-95%, are also intended to be encompassed by the present invention. As used herein, a "reduced activity" or "reduced enzymatic activity" can also include an activity that has been deleted or "knocked out" (*e.g.*, approximately 100% less activity than that of the polypeptide or protein encoded by the wild-type nucleic acid molecule or gene).

Activity can be determined according to any well accepted assay for measuring activity of a particular protein of interest. Activity can be measured or assayed directly, for example, measuring an activity of a protein in a crude cell extract or isolated or purified from a cell or microorganism. Alternatively, an activity can be measured or assayed within a cell or microorganism or in an extracellular medium. For example, assaying for a mutant gene (*i.e.*, said mutant encoding a reduced enzymatic activity) can be accomplished by expressing the mutated gene in a microorganism, for example, a mutant microorganism in which the enzyme is a temperature-sensitive, and assaying the mutant gene for the ability to complement a temperature sensitive (Ts) mutant for enzymatic activity. A mutant gene that encodes an "increased enzymatic activity" can be one that complements the Ts mutant more effectively than, for example, a corresponding wild-type gene. A mutant gene that encodes a "reduced enzymatic activity" is one that complements the Ts mutant less effectively than, for example, a corresponding wild-type gene.

It will be appreciated by the skilled artisan that even a single substitution in a nucleic acid or gene sequence (*e.g.*, a base substitution that encodes an amino acid change in the corresponding amino acid sequence) can dramatically affect the activity of an encoded polypeptide or protein as compared to the corresponding wild-type polypeptide or protein. A mutant gene (*e.g.*, encoding a mutant polypeptide or protein), as defined herein, is readily distinguishable from a nucleic acid or gene encoding a protein homologue in that a mutant gene encodes a protein or polypeptide having an altered activity, optionally observable as a different or distinct phenotype in a microorganism expressing said mutant gene or producing said mutant protein or polypeptide (*i.e.*, a mutant microorganism) as compared to a corresponding microorganism expressing the wild-type gene. By contrast, a protein homologue can have an identical or substantially similar activity, optionally phenotypically indiscernable when produced in a microorganism, as compared to a corresponding microorganism expressing the wild-type gene. Accordingly it is not, for example, the degree of sequence identity between nucleic acid molecules, genes, protein or polypeptides that serves to distinguish between homologues and mutants, rather it is the activity of the encoded protein or polypeptide that distinguishes between homologues and mutants: homologues having, for example, low (*e.g.*, 30-50% sequence identity) sequence identity yet having substantially equivalent functional activities, and mutants, for example sharing 99% sequence identity yet having dramatically different or altered functional activities.

It will also be appreciated by the skilled artisan that nucleic acid molecules, genes, protein or polypeptides for use in the instant invention can be derived from any microorganisms having a MTF biosynthetic pathway, an *ilv* biosynthetic pathway or a pantothenate biosynthetic pathway. Such nucleic acid molecules, genes, protein or polypeptides can be identified by the skilled artisan using known techniques such as homology screening, sequence comparison and the like, and can be modified by the skilled artisan in such a way that expression or production of these nucleic acid molecules, genes, protein or polypeptides occurs in a recombinant microorganism (*e.g.*, by using appropriate promotors, ribosomal binding sites, expression or integration vectors, modifying the sequence of the genes such that the transcription is increased (taking into account the preferable codon usage), etc., according to techniques described herein and those known in the art).

In one embodiment, the genes of the present invention are derived from a Gram positive microorganism organism (*e.g.*, a microorganism which retains basic dye, for example, crystal violet, due to the presence of a Gram-positive wall surrounding the microorganism). The term "derived from" (*e.g.*, "derived from" a Gram positive microorganism) refers to a gene which is naturally found in the microorganism (*e.g.*, is naturally found in a Gram positive microorganism). In a preferred embodiment, the genes of the present invention are derived from a microorganism belonging to a genus selected from the group consisting of *Bacillus, Cornyebacterium* (*e.g., Cornyebacterium glutamicum*), *Lactobacillus*, *Lactococci* and *Streptomyces*. In a more preferred embodiment, the genes of the present invention are derived from a microorganism is of the genus *Bacillus.* In another preferred embodiment, the genes of the present invention are derived from a microorganism selected from the group consisting *of Bacillus subtilis, Bacillus lentimorbus, Bacillus lentus, Bacillus firmus, Bacillus pantothenticus, Bacillus amyloliquefaciens, Bacillus cereus, Bacillus circulans, Bacillus coagulans, Bacillus licheniformis, Bacillus megaterium, Bacillus pumilus, Bacillus thuringiensis, Bacillus halodurans,* and other Group I *Bacillus* species, for example, as characterized by 16S rRNA type. In another preferred embodiment, the gene is derived from *Bacillus brevis* or *Bacillus stearothermophilus.* In another preferred embodiment, the genes of the present invention are derived from a microorganism selected from the group consisting of *Bacillus licheniformis, Bacillus amyloliquefaciens, Bacillus subtilis,* and *Bacillus pumilus.* In a particularly preferred embodiment, the gene is derived from *Bacillus subtilis* (*e.g.,* is *Bacillus subtilis-derived*). The term "derived from *Bacillus subtilis"* or *"Bacillus subtilis-derived"* includes a gene which is naturally found in the microorganism *Bacillus subtilis.* Included within the scope of the present invention are *Bacillus*-derived genes (*e.g., B. subtilis*-derived genes), for example, *Bacillus* or *B*. *subtilis purR* genes, *serA* genes, *glyA genes, coaX* genes, *coaA* genes, *pan* genes and/or *ilv* genes.

In another embodiment, the genes of the present invention are derived from a Gram negative (excludes basic dye) microorganism. In a preferred embodiment, the genes of the present invention are derived from a microorganism belonging to a genus selected from the group consisting of *Salmonella* (*e.g., Salmonella typhimurium*), *Escherichia, Klebsiella, Serratia,* and *Proteus.* In a more preferred embodiment, the genes of the present invention are derived from a microorganism of the genus *Escherichia.* In an even more preferred embodiment, the genes of the present invention are derived from *Escherichia coli.* In another embodiment, the genes of the present invention are derived from *Saccharomyces* (*e.g., Saccharomyces cerevisiae*).

### II. Recombinant Nucleic Acid Molecules and Vectors

The present invention further features recombinant nucleic acid molecules (*e.g.*, recombinant DNA molecules) that include genes described herein (*e.g.,* isolated genes), preferably *Bacillus* genes, more preferably *Bacillus subtilis genes,* even more preferably *Bacillus subtilis* pantothenate biosynthetic genes and/or isoleucine-valine *(ilv)* biosynthetic genes and/or methylenetetrahydrofolate (MTF) biosynthetic genes. The term "recombinant nucleic acid molecule" includes a nucleic acid molecule (*e.g.,* a DNA molecule) that has been altered, modified or engineered such that it differs in nucleotide sequence from the native or natural nucleic acid molecule from which the recombinant nucleic acid molecule was derived (*e.g.*, by addition, deletion or substitution of one or more nucleotides). Preferably, a recombinant nucleic acid molecule (*e.g.*, a recombinant DNA molecule) includes an isolated gene of the present invention operably linked to regulatory sequences. The phrase "operably linked to regulatory sequence(s)" means that the nucleotide sequence of the gene of interest is linked to the regulatory sequence(s) in a manner which allows for expression (*e.g.*, enhanced, increased, constitutive, basal, attenuated, decreased or repressed expression) of the gene, preferably expression of a gene product encoded by the gene (*e.g.*, when the recombinant nucleic acid molecule is included in a recombinant vector, as defined herein, and is introduced into a microorganism).

The term "regulatory sequence" includes nucleic acid sequences which affect (*e.g.*, modulate or regulate) expression of other nucleic acid sequences (*i.e.*, genes). In one embodiment, a regulatory sequence is included in a recombinant nucleic acid molecule in a similar or identical position and/or orientation relative to a particular gene of interest as is observed for the regulatory sequence and gene of interest as it appears in nature, *e.g.*, in a native position and/or orientation. For example, a gene of interest can be included in a recombinant nucleic acid molecule operably linked to a regulatory sequence which accompanies or is adjacent to the gene of interest in the natural organism (*e.g.*, operably linked to "native" regulatory sequences (*e.g.*, to the "native" promoter). Alternatively, a gene of interest can be included in a recombinant nucleic acid molecule operably linked to a regulatory sequence which accompanies or is adjacent to another (*e.g.*, a different) gene in the natural organism. Alternatively, a gene of interest can be included in a recombinant nucleic acid molecule operably linked to a regulatory sequence from another organism. For example, regulatory sequences from other microbes (*e.g.*, other bacterial regulatory sequences, bacteriophage regulatory sequences and the like) can be operably linked to a particular gene of interest.

In one embodiment, a regulatory sequence is a non-native or non-naturally-occurring sequence (*e.g.*, a sequence which has been modified, mutated, substituted, derivatized, deleted including sequences which are chemically synthesized). Preferred regulatory sequences include promoters, enhancers, termination signals, anti-termination signals and other expression control elements (*e.g.*, sequences to which repressors or inducers bind and/or binding sites for transcriptional and/or translational regulatory proteins, for example, in the transcribed mRNA). Such regulatory sequences are described, for example, in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989. Regulatory sequences include those which direct constitutive expression of a nucleotide sequence in a microorganism (*e.g.*, constitutive promoters and strong constitutive promoters), those which direct inducible expression of a nucleotide sequence in a microorganism (*e.g.*, inducible promoters, for example, xylose inducible promoters) and those which attenuate or repress expression of a nucleotide sequence in a microorganism (*e.g.*, attenuation signals or repressor binding sequences, for example, a PurR binding site). It is also within the scope of the present invention to regulate expression of a gene of interest by removing or deleting regulatory sequences. For example, sequences involved in the negative regulation of transcription can be removed such that expression of a gene of interest is enhanced.

In one embodiment, a recombinant nucleic acid molecule of the present invention includes a nucleic acid sequence or gene that encodes at least one bacterial gene product (*e.g.*, a pantothenate biosynthetic enzyme, an isoleucine-valine biosynthetic enzyme and/or a methylenetetrahydrofolate (MTF) biosynthetic enzyme) operably linked to a promoter or promoter sequence. Preferred promoters of the present invention include *Bacillus* promoters and/or bacteriophage promoters (*e.g.*, bacteriophage which infect *Bacillus*). In one embodiment, a promoter is a *Bacillus* promoter, preferably a strong *Bacillus* promoter (*e.g.*, a promoter associated with a biochemical housekeeping gene in *Bacillus* or a promoter associated with a glycolytic pathway gene in *Bacillus*). In another embodiment, a promoter is a bacteriophage promoter. In a preferred embodiment, the promoter is from the bacteriophage SP01. In a particularly preferred embodiment, a promoter is selected from the group consisting of *P₁₅*, *P₂₆* or *P_{veg},* having for example, the following respective seqeunces:
GCTATTGACGACAGCTATGGTTCACTGTCGACCAACCAAAAGTGTGGTCAGT ACCGCCAATATTTCTCCCTTGAGGGGTACAAAGAGGTGTCCCTAGAAGAGAT CCACGCTGTGTAAAAATTTTACAAAAAGGTATTGACTTTCCCTACAGGGTGT GTAATAATTTAATTACAGGCGGGGGCAACCCCGCCTGT(SEQ ID NO:1), GCCTACCTAGCTTCCAAGAAAGATATCCTAACAGCACAAGAGCGGAAAGAT GTTTTGTTCTACATCCAGAACAACCTCTGCTAAAATTCCTGAAAAATTTTGCA AAAAGTTGTTGACTTTATCTACAAGGTGTGGTATAATAATCTTAACAACAGC AGGACGC (SEQ ID NO:2), and
GAGGAATCATAGAATTTTGTCAAAATAATTTTATTGACAACGTCTTATTAAC GTTGATATAATTTAAATTTTATTTGACAAAAATGGGCTCGTGTTGTACAATA AATGTAGTGAGGTGGATGCAATG (SEQ ID NO:3). Additional preferred promoters include *tef* (the translational elongation factor (TEF) promoter) and *pyc* (the pyruvate carboxylase (PYC) promoter), which promote high level expression in *Bacillus* (*e.g.*, *Bacillus subtilis*). Additional preferred promoters, for example, for use in Gram positive microorganisms include, but are not limited to, *amy* and SPO2 promoters. Additional preferred promoters, for example, for use in Gram negative microorganisms include, but are not limited to, *cos, tac, trp, tet, trp-tet, lpp, lac, lpp-lac, lacIQ,* T7, T5, T3, *gal, trc, ara,* SP6, λ-PR or λ-PL.

In another embodiment, a recombinant nucleic acid molecule of the present invention includes a terminator sequence or terminator sequences (*e.g.*, transcription terminator sequences). The term "terminator sequences" includes regulatory sequences that serve to terminate transcription of mRNA. Terminator sequences (or tandem transcription terminators) can further serve to stabilize mRNA (*e.g.*, by adding structure to mRNA), for example, against nucleases.

In yet another embodiment, a recombinant nucleic acid molecule of the present invention includes sequences that allow for detection of the vector containing said sequences (*i.e.*, detectable and/or selectable markers), for example, genes that encode antibiotic resistance sequences or that overcome auxotrophic mutations, for example, *trpC,* drug markers, fluorescent markers, and/or colorimetric markers (*e.g.*, *lacZ*/β-galactosidase). In yet another embodiment, a recombinant nucleic acid molecule of the present invention includes an artificial ribosome binding site (RBS) or a sequence that gets transcribed into an artificial RBS. The term "artificial ribosome binding site (RBS)" includes a site within an mRNA molecule (*e.g.*, coded within DNA) to which a ribosome binds (*e.g.*, to initiate translation) which differs from a native RBS (*e.g.*, a RBS found in a naturally-occurring gene) by at least one nucleotide. Preferred artificial RBSs include about 5-6, 7-8, 9-10, 11-12, 13-14, 15-16, 17-18, 19-20, 21-22, 23-24, 25-26, 27-28, 29-30 or more nucleotides of which about 1-2, 3-4, 5-6, 7-8, 9-10, 11-12, 13-15 or more differ from the native RBS (*e.g.*, the native RBS of a gene of interest, for example, the native *panB* RBS TAAACATGAGGAGGAGAAAACATG (SEQ-ID NO:4) or the native *panD* RBS ATTCGAGAAATGGAGAGAATATAATATG (SEQ ID NO:5)). Preferably, nucleotides that differ are substituted such that they are identical to one or more nucleotides of an ideal RBS when optimally aligned for comparisons. Ideal RBSs include, but are not limited to, AGAAAGGAGGTGA (SEQ ID NO:6),
TTAAGAAAGGAGGTGANNNNATG (SEQ ID NO:7),
TTAGAAAGGAGGTGANNNNNATG (SEQ ID NO:8),
AGAAAGGAGGTGANNNNNNNATG (SEQ ID NO:9), and
AGAAAGGAGGTGANNNNNNATG (SEQ ID NO:10). Artificial RBSs can be used to replace the naturally-occurring or native RBSs associated with a particular gene. Artificial RBSs preferably increase translation of a particular gene. Preferred artificial RBSs (*e.g.,* RBSs for increasing the translation of *panB*, for example, of *B. subtilis panB*) include CCCTCTAGAAGGAGGAGAAAACATG (SEQ ID NO:11) and CCCTCTAGAGGAGGAGAAAACATG (SEQ ID NO:12). Preferred artificial RBSs (e.g., RBSs for increasing the translation of *panD,* for example, of *B. subtilis panD*) include TTAGAAAGGAGGATTTAAATATG (SEQ ID NO:13),
TTAGAAAGGAGGTTTAATTAATG (SEQ ID NO:14),
TTAGAAAGGAGGTGATTTAAATG (SEQ ID NO:15),
TTAGAAAGGAGGTGTTTAAAATG (SEQ ID NO:16), ATTCGAGAAAGGAGG TGAATATAATATG (SEQ ID NO:17), ATTCGAGAAAGGAGGTGAATAATAATG (SEQ ID NO:18), and ATTCGTAGAAAGGAGGTGAATTAATATG (SEQ ID NO:19).

The present invention further features vectors (*e.g.*, recombinant vectors) that include nucleic acid molecules (*e.g.*, genes or recombinant nucleic acid molecules comprising said genes) as described herein. The term "recombinant vector" includes a vector (*e.g.*, plasmid, phage, phasmid, virus, cosmid or other purified nucleic acid vector) that has been altered, modified or engineered such that it contains greater, fewer or different nucleic acid sequences than those included in the native or natural nucleic acid molecule from which the recombinant vector was derived. Preferably, the recombinant vector includes a biosynthetic enzyme-encoding gene or recombinant nucleic acid molecule including said gene, operably linked to regulatory sequences, for example, promoter sequences, terminator sequences and/or artificial ribosome binding sites (RBSs), as defined herein. In another embodiment, a recombinant vector of the present invention includes sequences that enhance replication in bacteria (*e.g.*, replication-enhancing sequences). In one embodiment, replication-enhancing sequences function in *E*. *coli.* In another embodiment, replication-enhancing sequences are derived from pBR322.

In yet another embodiment, a recombinant vector of the present invention includes antibiotic resistance sequences. The term "antibiotic resistance sequences" includes sequences which promote or confer resistance to antibiotics on the host organism *(e.g., Bacillus).* In one embodiment, the antibiotic resistance sequences are selected from the group consisting of *cat* (chloramphenicol resistance) sequences, *tet* (tetracycline resistance) sequences, *erm* (erythromycin resistance) sequences, *neo* (neomycin resistance) sequences, *kan* (kanamycin resistence) sequences and *spec* (spectinomycin resistance) sequences. Recombinant vectors of the present invention can further include homologous recombination sequences (*e.g.*, sequences designed to allow recombination of the gene of interest into the chromosome of the host organism). For example, *bpr, vpr*, or *amyE* sequences can be used as homology targets for recombination into the host chromosome. It will further be appreciated by one of skill in the art that the design of a vector can be tailored depending on such factors as the choice of microorganism to be genetically engineered, the level of expression of gene product desired and the like.

### III. Recombinant Microorganisms

The present invention further features microorganisms, *i.e.*, recombinant microorganisms, that include vectors or genes (*e.g.*, wild-type and/or mutated genes) as described herein. As used herein, the term "recombinant microorganism" includes a microorganism (*e.g.*, bacteria, yeast cell, fungal cell, etc.) that has been genetically altered, modified or engineered (*e.g.*, genetically engineered) such that it exhibits an altered, modified or different genotype and/or phenotype (*e.g.*, when the genetic modification affects coding nucleic acid sequences of the microorganism) as compared to the naturally-occurring microorganism from which it was derived.

In one embodiment, a recombinant microorganism of the present invention is a Gram positive organism (*e.g.*, a microorganism which retains basic dye, for example, crystal violet, due to the presence of a Gram-positive wall surrounding the microorganism). In a preferred embodiment, the recombinant microorganism is a microorganism belonging to a genus selected from the group consisting of *Bacillus, Cornyebacterium* (*e.g., Cornyebacterium glutamicum*), *Lactobacillus, Lactococci* and *Streptomyces.* In a more preferred embodiment, the recombinant microorganism is of the genus *Bacillus.* In another preferred embodiment, the recombinant microorganism is selected from the group consisting of *Bacillus subtilis, Bacillus lentimorbus, Bacillus lentus, Bacillus firmus, Bacillus pantothenticus, Bacillus amyloliquefaciens, Bacillus cereus, Bacillus circulans, Bacillus coagulans, Bacillus licheniformis, Bacillus megaterium, Bacillus pumilus, Bacillus thuringiensis, Bacillus halodurans,* and other Group 1 *Bacillus* species, for example, as characterized by 16S rRNA type. In another preferred embodiment, the recombinant microorganism is *Bacillus brevis* or *Bacillus stearothermophilus.* In another preferred embodiment, the recombinant microorganism is selected from the group consisting of *Bacillus licheniformis, Bacillus amyloliquefaciens, Bacillus subtilis,* and *Bacillus pumilus.*

In another embodiment, the recombinant microorganism is a Gram negative (excludes basic dye) organism. In a preferred embodiment, the recombinant microorganism is a microorganism belonging to a genus selected from the group consisting of *Salmonella* (*e.g., Salmonella typhimurium*), *Escherichia, Klebsiella, Serratia,* and *Proteus.* In a more preferred embodiment, the recombinant microorganism is of the genus *Escherichia.* In an even more preferred embodiment, the recombinant microorganism is *Escherichia coli.* In another embodiment, the recombinant microorganism is *Saccharomyces* (*e.g., Saccharomyces cerevisiae*).

A preferred "recombinant" microorganism of the present invention is a microorganism having a deregulated pantothenate biosynthesis pathway or enzyme, a deregulated isoleucine-valine (*ilv*) biosynthetic pathway or enzyme and/or a modified or deregulated methylenetetrahydrofolate (MTF) biosynthetic pathway or enzyme. The term "deregulated" or "deregulation" includes the alteration or modification of at least one gene in a microorganism that encodes an enzyme in a biosynthetic pathway, such that the level or activity of the biosynthetic enzyme in the microorganism is altered or modified. Preferably, at least one gene that encodes an enzyme in a biosynthetic pathway is altered or modified such that the gene product is enhanced or increased. The phrase "deregulated pathway" includes a biosynthetic pathway in which more than one gene that encodes an enzyme in a biosynthetic pathway is altered or modified such that the level or activity of more than one biosynthetic enzyme is altered or modified. The ability to "deregulate" a pathway (*e.g.*, to simultaneously deregulate more than one gene in a given biosynthetic pathway) in a microorganism in some cases arises from the particular phenomenon of microorganisms in which more than one enzyme (*e.g.*, two or three biosynthetic enzymes) are encoded by genes occurring adjacent to one another on a contiguous piece of genetic material termed an "operon" (defined herein). Due to the coordinated regulation of genes included in an operon, alteration or modification of the single promoter and/or regulatory element can result in alteration or modification of the expression of each gene product encoded by the operon. Alteration or modification of a regulatory element can include, but is not limited to removing the endogenous promoter and/or regulatory element(s), adding strong promoters, inducible promoters or multiple promoters or removing regulatory sequences such that expression of the gene products is modified, modifying the chromosomal location of a gene or operon, altering nucleic acid sequences adjacent to a gene or operon (or within an operon) such as a ribosome binding site, increasing the copy number of a gene or operon, modifying proteins (*e.g.*, regulatory proteins, suppressors, enhancers, transcriptional activators and the like) involved in transcription of a gene or operon and/or translation of a gene product or gene products of a gene or operon, respectively, or any other conventional means of deregulating expression of genes routine in the art (including but not limited to use of antisense nucleic acid molecules, for example, to block expression of repressor proteins). Deregulation can also involve altering the coding region of one or more genes to yield, for example, an enzyme that is feedback resistant or has a higher or lower specific activity.

In another preferred embodiment, a recombinant microorganism is designed or engineered such that at least one pantothenate biosynthetic enzyme, at least one isoleucine-valine biosynthetic enzyme, and/or at least one MTF biosynthetic enzyme is overexpressed. The term "overexpressed" or "overexpression" includes expression of a gene product (*e.g.*, a biosynthetic enzyme) at a level greater than that expressed prior to manipulation of the microorganism or in a comparable microorganism which has not been manipulated. In one embodiment, the microorganism can be genetically designed or engineered to overexpress a level of gene product greater than that expressed in a comparable microorganism which has not been engineered.

Genetic engineering can include, but is not limited to, altering or modifying regulatory sequences or sites associated with expression of a particular gene (*e.g.*, by adding strong promoters, inducible promoters or multiple promoters or by removing regulatory sequences such that expression is constitutive), modifying the chromosomal location of a particular gene, altering nucleic acid sequences adjacent to a particular gene such as a ribosome binding site, increasing the copy number of a particular gene, modifying proteins (*e.g.*, regulatory proteins, suppressors, enhancers, transcriptional activators and the like) involved in transcription of a particular gene and/or translation of a particular gene product, or any other conventional means of deregulating expression of a particular gene routine in the art (including but not limited to use of antisense nucleic acid molecules, for example, to block expression of repressor proteins). Genetic engineering can also include deletion of a gene, for example, to block a pathway or to remove a repressor.

In another embodiment, the microorganism can be physically or environmentally manipulated to overexpress a level of gene product greater than that expressed prior to manipulation of the microorganism or in a comparable microorganism which has not been manipulated. For example, a microorganism can be treated with or cultured in the presence of an agent known or suspected to increase transcription of a particular gene and/or translation of a particular gene product such that transcription and/or translation are enhanced or increased. Alternatively, a microorganism can be cultured at a temperature selected to increase transcription of a particular gene and/or translation of a particular gene product such that transcription and/or translation are enhanced or increased.

### IV. Culturing and Fermenting Recombinant Microorganisms

The term "culturing" includes maintaining and/or growing a living microorganism of the present invention (*e.g.*, maintaining and/or growing a culture or strain). In one embodiment, a microorganism of the invention is cultured in liquid media. In another embodiment, a microorganism of the invention is cultured in solid media or semi-solid media. In a preferred embodiment, a microorganism of the invention is cultured in media (*e.g.*, a sterile, liquid medium) comprising nutrients essential or beneficial to the maintenance and/or growth of the microorganism (*e.g.*, carbon sources or carbon substrate, for example carbohydrate, hydrocarbons, oils, fats, fatty acids, organic acids, and alcohols; nitrogen sources, for example, peptone, yeast extracts, meat extracts, malt extracts, soy meal, soy flour, soy grits, urea, ammonium sulfate, ammonium chloride, ammonium nitrate and ammonium phosphate; phosphorus sources, for example, phosphoric acid, sodium and potassium salts thereof; trace elements, for example, magnesium, iron, manganese, calcium, copper, zinc, boron, molybdenum, and/or cobalt salts; as well as growth factors such as amino acids, vitamins, growth promoters and the like).

Preferably, microorganisms of the present invention are cultured under controlled pH. The term "controlled pH" includes any pH which results in production of the desired product (*e.g.*, pantoate and/or pantothenate). In one embodiment microorganisms are cultured at a pH of about 7. In another embodiment, microorganisms are cultured at a pH of between 6.0 and 8.5. The desired pH may be maintained by any number of methods known to those skilled in the art.

Also preferably, microorganisms of the present invention are cultured under controlled aeration. The term "controlled aeration" includes sufficient aeration (*e.g.*, oxygen) to result in production of the desired product (*e.g.*, pantoate and/or pantothenate). In one embodiment, aeration is controlled by regulating oxygen levels in the culture, for example, by regulating the amount of oxygen dissolved in culture media. Preferably, aeration of the culture is controlled by agitating the culture. Agitation may be provided by a propeller or similar mechanical agitation equipment, by revolving or shaking the cuture vessel (*e.g.*, tube or flask) or by various pumping equipment. Aeration may be further controlled by the passage of sterile air or oxygen through the medium (*e.g.*, through the fermentation mixture). Also preferably, microorganisms of the present invention are cultured without excess foaming (*e.g.*, *via* addition of antifoaming agents).

Moreover, microorganisms of the present invention can be cultured under controlled temperatures. The term "controlled temperature" includes any temperature which results in production of the desired product (*e.g.*, pantoate and/or pantothenate). In one embodiment, controlled temperatures include temperatures between 15°C and 95°C. In another embodiment, controlled temperatures include temperatures between 15°C and 70°C. Preferred temperatures are between 20°C and 55°C, more preferably between 30°C and 50°C.

Microorganisms can be cultured (*e.g.*, maintained and/or grown) in liquid media and preferably are cultured, either continuously or intermittently, by conventional culturing methods such as standing culture, test tube culture, shaking culture (*e.g.*, rotary shaking culture, shake flask culture, etc.), aeration spinner culture, or fermentation. In a preferred embodiment, the microorganisms are cultured in shake flasks. In a more preferred embodiment, the microorganisms are cultured in a fermentor (*e.g.*, a fermentation process). Fermentation processes of the present invention include, but are not limited to, batch, fed-batch and continuous processes or methods of fermentation. The phrase "batch process" or "batch fermentation" refers to a system in which the composition of media, nutrients, supplemental additives and the like is set at the beginning of the fermentation and not subject to alteration during the fermentation, however, attempts may be made to control such factors as pH and oxygen concentration to prevent excess media acidification and/or microorganism death. The phrase "fed-batch process" or "fed-batch" fermentation refers to a batch fermentation with the exception that one or more substrates or supplements are added (*e.g.*, added in increments or continuously) as the fermentation progresses. The phrase "continuous process" or "continuous fermentation" refers to a system in which a defined fermentation media is added continuously to a fermentor and an equal amount of used or "conditioned" media is simultaneously removed, preferably for recovery of the desired product (*e.g.*, pantoate and/or pantothenate). A variety of such processes have been developed and are well-known in the art.

The phrase "culturing under conditions such that a desired compound is produced" includes maintaining and/or growing microorganisms under conditions (*e.g.*, temperature, pressure, pH, duration, etc.) appropriate or sufficient to obtain production of the desired compound or to obtain desired yields of the particular compound being produced. For example, culturing is continued for a time sufficient to produce the desired amount of a compound (*e.g.*, pantoate and/or pantothenate). Preferably, culturing is continued for a time sufficient to substantially reach suitable production of the compound (*e.g.*, a time sufficient to reach a suitable concentration of pantoate and/or pantothenate or suitable ratio of pantoate and/or pantothenate:HMBPA). In one embodiment, culturing is continued for about 12 to 24 hours. In another embodiment, culturing is continued for about 24 to 36 hours, 36 to 48 hours, 48 to 72 hours, 72 to 96 hours, 96 to 120 hours, 120 to 144 hours, or greater than 144 hours. In yet another embodiment, microorganisms are cultured under conditions such that at least about 5 to 10 g/L of compound are produced in about 36 hours, at least about 10 to 20 g/L compound are produced in about 48 hours, or at least about 20 to 30 g/L compound in about 72 hours. In yet another embodiment, microorganisms are cultured under conditions such that at least about 5 to 20 g/L of compound are produced in about 36 hours, at least about 20 to 30 g/L compound are produced in about 48 hours, or at least about 30 to 50 or 60 g/L compound in about 72 hours. In yet another embodiment, microorganisms are cultured under conditions such that at least about 40 to 60 g/L of compound are produced in about 36 hours, or at least about 60 to 90 g/L compound are produced in about 48 hours. It will be appreciated by the skilled artisan that values above the upper limits of the ranges recited may be obtainable by the processes described herein, for example, in a particular fermentation run or with a particular engineered strain.

Preferably, a production method of the present invention results in production of a level of pantothenate that is "enhanced as compared to an appropriate control". The term "appropriate control", as defined herein, includes any control recognized by the skilled artisan as being approriate for determining enhanced, increased, or elevated levels of desired product. For example, where the process features culturing a microorganism having a deregulated pantothenate biosynthetic pathway and said microorganism further has a deregulated MTF biosynthetic pathway (*i.e.*, has been engineered such that at least one MTF biosynthetic enzyme is deregulated, for example, overexpressed) an appropriate control includes a culture of the microorganism before or absent manipulation of the MTF enzyme or pathway (*i.e.*, having only the pantothenate biosynthetic pathway deregulated). Likewise, where the process features culturing a microorganism having a deregulated pantothenate biosynthetic pathway and a deregulated *ilv* biosynthetic pathway and said microorganism further has a deregulated MTF biosynthetic pathway (*i.e.,* has been engineered such that at least one MTF biosynthetic enzyme is deregulated, for example, overexpressed) an appropriate control includes a culture of the microorganism before or absent manipulation of the MTF enzyme or pathway (*i.e.*, having only the pantothenate biosynthetic pathway and *ilv* biosynthetic pathway deregulated). Comparison need not be performed in each process practiced according to the present invention. For example, a skilled artisan can determine appropriate controls empirically from performing a series of reactions (*e.g.*, test tube cultures, shake flask cultures, fermentations), for example, under the same or similar conditions. Having appreciated a routine production level, for example, by a particular strain, the artisan is able to recognize levels that are enhanced, increased or elevated over such levels. In other words, comparison to an appropriate control includes comparison to a predetermined values (*e.g.*, a predetermined control).

Thus, in an embodiment wherein an appropriately engineered strain produces 40 g/L pantothenate in 36 hours (prior to manipulation such that pantothenate production is enhanced), production of 50, 60, 70 or more g/L pantothenate (after manipulation, for example, manipulation such that at least one MTF biosynthetic enzyme is overexpressed) exemplifies enhanced production. Likewise, in an embodiment wherein an appropriately engineered strain produces 50 g/L pantothenate in 48 hours (prior to manipulation such that pantothenate production is enhanced), production of 60, 70, 80, 90 or more g/L pantothenate (after manipulation, for example, manipulation such that at least one MTF biosynthetic enzyme is overexpressed) exemplifies enhanced production.

The methodology of the present invention can further include a step of recovering a desired compound (*e.g.*, pantoate and or pantothenate). The term "recovering" a desired compound includes extracting, harvesting, isolating or purifying the compound from culture media. Recovering the compound can be performed according to any conventional isolation or purification methodology known in the art including, but not limited to, treatment with a conventional resin (*e.g.*, anion or cation exchange resin, non-ionic adsorption resin, etc.), treatment with a conventional adsorbent (*e.g.*, activated charcoal, silicic acid, silica gel, cellulose, alumina, etc.), alteration of pH, solvent extraction (*e.g.*, with a conventional solvent such as an alcohol, ethyl acetate, hexane and the like), dialysis, filtration, concentration, crystallization, recrystallization, pH adjustment, lyophilization and the like. For example, a compound can be recovered from culture media by first removing the microorganisms from the culture. Media are then passed through or over a cation exchange resin to remove cations and then through or over an anion exchange resin to remove inorganic anions and organic acids having stronger acidities than the compound of interest. The resulting compound can subsequently be converted to a salt (*e.g.*, a calcium salt) as described herein.

Preferably, a desired compound of the present invention is "extracted", "isolated" or "purified" such that the resulting preparation is substantially free of other media components (*e.g.*, free of media components and/or fermentation byproducts). The language "substantially free of other media components" includes preparations of the desired compound in which the compound is separated from media components or fermentation byproducts of the culture from which it is produced. In one embodiment, the preparation has greater than about 80% (by dry weight) of the desired compound (*e.g.,* less than about 20% of other media components or fermentation byproducts), more preferably greater than about 90% of the desired compound (*e.g.*, less than about 10% of other media components or fermentation byproducts), still more preferably greater than about 95% of the desired compound (*e.g.*, less than about 5% of other media components or fermentation byproducts), and most preferably greater than about 98-99% desired compound (*e.g.*, less than about 1-2% other media components or fermentation byproducts). When the desired compound has been derivatized to a salt, the compound is preferably further free of chemical contaminants associated with the formation of the salt. When the desired compound has been derivatized to an alcohol, the compound is preferably further free of chemical contaminants associated with the formation of the alcohol.

In an alternative embodiment, the desired compound is not purified from the microorganism, for example, when the microorganism is biologically non-hazardous (*e.g.*, safe). For example, the entire culture (or culture supernatant) can be used as a source of product (*e.g.*, crude product). In one embodiment, the culture (or culture supernatant) is used without modification. In another embodiment, the culture (or culture supernatant) is concentrated. In yet another embodiment, the culture (or culture supernatant) is dried or lyophilized.

In yet another embodiment, the desired compound is partially purified. The term "partially purified" includes media preparations that have had at least some processing, for example, treatment (*e.g.*, batch treatment) with a commercial resin. In preferred embodiments, the "partially purified" preparation has greater than about 30% (by dry weight) of the desired compound, preferably greater than about 40% of the desired compound, more preferably greater than about 50% of the desired compound, still more preferably greater than about 60% of the desired compound, and most preferably greater than about 70% desired compound. "Partially purified" preparations also preferably have 80% or less (by dry weight) of the desired compound (*i.e.*, are less pure than "extracted", "isolated" or "purified" preparations, as defined herein).

Depending on the biosynthetic enzyme or combination of biosynthetic enzymes manipulated, it may be desirable or necessary to provide (*e.g.*, feed) microorganisms of the present invention at least one biosynthetic precursor such that the desired compound or compounds are produced. The term "biosynthetic precursor" or "precursor" includes an agent or compound which, when provided to, brought into contact with, or included in the culture medium of a microorganism, serves to enhance or increase biosynthesis of the desired product. In one embodiment, the biosynthetic precursor or precursor is aspartate. In another embodiment, the biosynthetic precursor or precursor is β-alanine. The amount of aspartate or β-alanine added is preferably an amount that results in a concentration in the culture medium sufficient to enhance productivity of the microorganism (*e.g.*, a concentration sufficient to enhance production of pantoate and/or pantothenate). Biosynthetic precursors of the present invention can be added in the form of a concentrated solution or suspension (*e.g.*, in a suitable solvent such as water or buffer) or in the form of a solid (*e.g.*, in the form of a powder). Moreover, biosynthetic precursors of the present invention can be added as a single aliquot, continuously or intermittently over a given period of time. The term "excess β-alanine" includes β-alanine levels increased or higher that those routinely utilized for culturing the microorganism in question. For example, culturing the *Bacillus* microorganisms described in the instant Examples is routinely done in the presence of about 0-0.01 g/L β-alanine. Accordingly, excess β-alanine levels can include levels of about 0.01-1, preferably about 1-20 g/L.

In yet another embodiment, the biosynthetic precursor is valine. In yet another embodiment, the biosynthetic precursor is α-ketoisovalerate. Preferably, valine or α-ketoisovalerate is added in an amount that results in a concentration in the medium sufficient for production of the desired product (*_{e.g.}*, pantoate and/or pantothenate) to occur. The term "excess α-KIV" includes α-KIV levels increased or higher that those routinely utilized for culturing the microorganism in question. For example, culturing the *Bacillus* microorganisms described in the instant Examples is routinely done in the presence of about 0-0.01 g/L α-KIV. Accordingly, excess α-KTV levels can include levels of about 0.01-1, preferably about 1-20 g/L α-KIV. The term "excess valine" includes valine levels increased or higher that those routinely utilized for culturing the microorganism in question. For example, culturing the *Bacillus* microorganisms described in the instant Examples is routinely done in the presence of about 0-0.5 g/L valine. Accordingly, excess valine levels can include levels of about 0.5-5 g/L, preferably about 5-20 g/L valine.

In yet another embodiment, the biosynthetic precursor is serine. Preferably, serine is added in an amount that results in a concentration in the medium sufficient for production of the desired product (*e.g.*, pantoate and/or pantothenate) to occur. Excess serine (as defined herein) can also be added according to the production processes described herein, for example, for the enhanced production of pantothenate. The skilled artisan will appreciate that extreme excesses of biosynthetic precursors can result in microorganism toxicity. Biosynthetic precursors are also referred to herein as "supplemental biosynthetic substrates".

Another aspect of the present invention includes biotransformation processes which feature the recombinant microorganisms described herein. The term "biotransformation process", also referred to herein as "bioconversion processes", includes biological processes which results in the production (*e.g*., transformation or conversion) of appropriate substrates and/or intermediate compounds into a desired product.

The microorganism(s) and/or enzymes used in the biotransformation reactions are in a form allowing them to perform their intended function (*e.g.*, producing a desired compound). The microorganisms can be whole cells, or can be only those portions of the cells necessary to obtain the desired end result. The microorganisms can be suspended (*e.g.*, in an appropriate solution such as buffered solutions or media), rinsed (*e.g.*, rinsed free of media from culturing the microorganism), acetone-dried, immobilized (*e.g.*, with polyacrylamide gel or k-carrageenan or on synthetic supports, for example, beads, matrices and the like), fixed, cross-linked or permeablized (*e.g.*, have permeablized membranes and/or walls such that compounds, for example, substrates, intermediates or products can more easily pass through said membrane or wall).

This invention is further illustrated by the following examples which should not be construed as limiting. The contents of all references, patents and published patent applications cited throughout this application are incorporated herein by reference.

### EXAMPLES

### Example I: Panto-Compound Production Strains

In developing *Bacillus* strains for the production of pantothenate, various genetic manipulations are made to genes and enzymes involved in the pantothenate biosynthetic pathway and the isoleucine-valine (*ilv*) pathway (Figure 1) as described in U.S. Patent Application Serial No. 09/400,494 and U.S. Patent Application Serial No. 09/667,569. For example, strains having a deregulated *panBCD* operon and/or having deregulated *panE1* exhibit enhanced pantothenate production (when cultured in the presence of β-alanine and α-ketoisovalerate (α-KIV)). Strains further deregulated for *ilvBNC* and *ilvD* exhibit enhanced pantothenate production in the presence of any β-alanine. Moreover, it is possible to achieve β-alanine independence by further deregulating *panD.*

An exemplary pantothenate production strain is PA824, a tryptophan prototroph, Spec and Tet resistant, deregulated for *panBCD* at the *panBCD* locus, deregulated for *panE1* at the *panE1* locus (two genes in the *B. subtilis* genome are homologous to *E. coli panE*, *panE1 and panE2*, the former encoding the major ketopantoate reductase involved in pantothenate production, while *panE2* does not contribute to pantothenate synthesis (U.S. Patent Application Serial No. 09/400,494), deregulated for *ilvD* at the *ilvD* locus, overexpressing an *ilvBNC* cassette at the *anryE* locus, and overexpressing *panD* at the *bpr* locus. PA824 routinely yields approximately 40-50 g/L pantothenate, when cultured for 48 hours in 14 L fermentor vessels according to standard fermentation procedures (see *e.g.*, provisional Patent Application Serial No. 60/263,053 or provisional Patent Application Serial No. 60/262,995, incorporated by reference herein). Briefly, batch media (4.5 L) containing trace elements is inoculated with shake flask cultures of PA824. The fermentations are controlled for temperature (*e.g.*, 43°C), dissolved O₂, and pH, and are run as a glucose limited fed batch process. After the initial batched glucose is consumed, glucose concentrations are maintained between about 0 and 1 g/L by continuous feeding of fresh FEED media. pH is set at 7.2, monitored, and maintained by feeding either a NH₃- or a H₃PO₄-solution. The dissolved oxygen concentration [pO₂] is maintained at about 10-30% by regulation of the agitation and aeration rate. Foaming is controlled by addition of an appropriate antifoam agent. The pantothenate titer in the fermentation broth is determined (by HPLC analysis) after removal of the cells by centrifugation.

A second exemplary strain is PA668. PA668 is a derivative of PA824 that contains extra copies of *P₂₆ panB* amplified at the *vpr* and/or *panB* locus. PA668 was constructed using a *panB* expression vector (pAN636) which allows for selection of multiple copies using chloramphenicol. Briefly, a pAN636 *NotI* restriction fragment (excluding vector sequences) was ligated and then used to transform PA824 with selection on plates containing 5 µg/ml chloramphenicol. Transformants resistant to 30 µg/ml chloramphenicol were isolated and screened for pantothenate production in 48 hour test tube cultures. The isolates produce about 10 percent more pantothenate than PA824. In 10-L fermentations, a first strain, PA668-2A, produces pantothenate in amounts comparable to PA824 cultured under similar comditions (*e.g.*, ∼45-50 g/L at 36 hours). After 36 hours, when pantothenate production routinely begins to slow with PA824, PA668-2A continues to produce significant levels of pantothenate (*e.g.*, ∼ 60-65 g/l pantothenate at 48 hours). A second strain, PA668-24, produces pantothenate at an even faster rate, reaching 60-70 g/L after 48 hours.

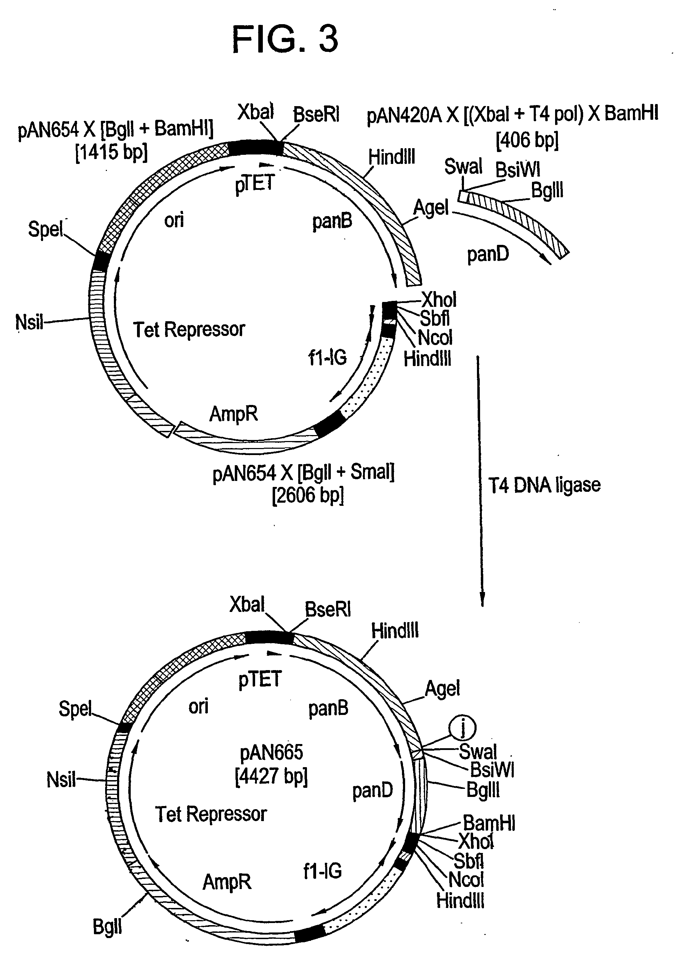

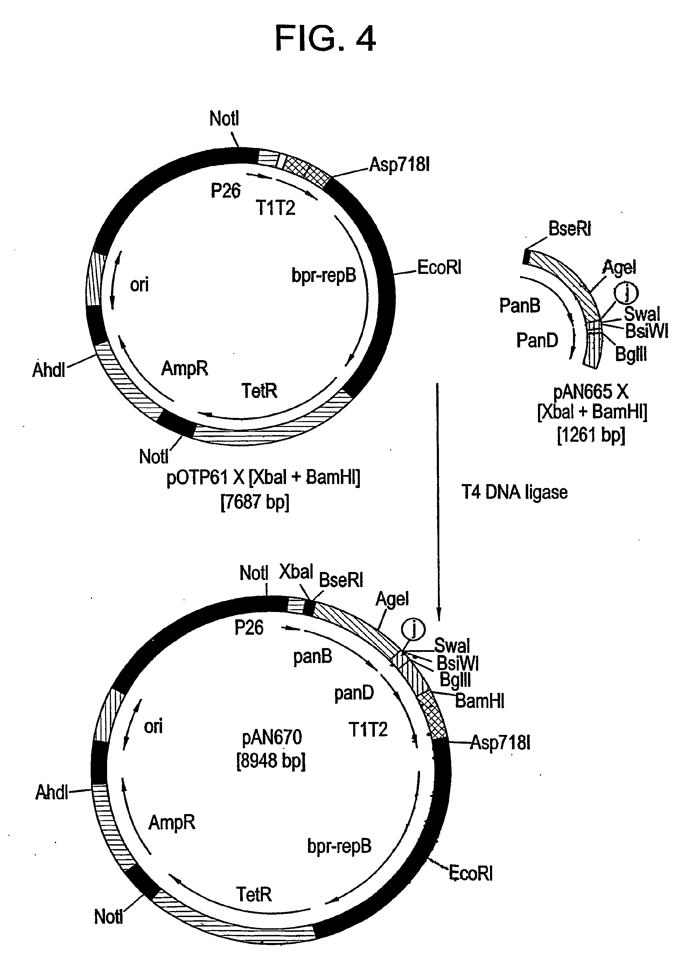

A third production strain, PA721B-39, was engineered to further include an amplifiable *P₂₆ panBpanD* cassette as follows. First, a single expression cassette was constructed that is capable of integrating both *panB* and *panD* at the *bpr* locus. Combining both genes into one expression cassette simplifies the resulting strain by eliminating an antibiotic resistance marker. The *P₂₆ panBpanD* expression cassette was constructed to include each of two different *panD* ribosome binding sites (the RBSs having previously been synthesized and tested in International Public. No. WO 01/21772 and U.S. Patent Application No. 60/262,995). The cassette further included the synthetic *panB* gene ribosome binding site (RBS 1), but the design permits future alteration of the panB RBS by simple oligonucleotide cassette substitution. In the first step of construction, the *panB* gene was joined to the two *panD* gene cassettes as illustrated in Figure 3 for the construction of pAN665. Next, the resulting *panBpanD* cassettes were transferred to *B. subtilis* expression vector pOTP61 as illustrated in Figure 4. A summary of the essential features of each plasmid (pAN670 and pAN674) constructed is presented in Table 1.

**Table 1. Plasmids containing various B. subtilis panBpanD gene expression cassettes.**

| Plasmid | *panD* RBS | Vector | Host strain |
|---|---|---|---|
| pAN665 | Standard | pASK-1BA3 | *E. coli* |
| pAN670 | " | pOTP61 | *B. subtilis* |
| pAN669 | ND-C2 | pASK-1BA3 | *E. coli* |
| pAN674 | " | pOTP61 | *B. subtilis* |

These new plasmids combine production of extra PanB and PanD from a single vector and were predicted to produce increased levels of PanB relative to the *panB* expression vector (pAN636) present in PA668. The strategy to install the *P26 panBpanD* vectors in pantothenate production strains took advantage of genetic linkage between *bpr* and *panE1.* A derivative of PA824 was first constructed that is cured of the resident *panD* expression cassette by transforming the strain with chromosomal DNA isolated from PA930 (*panE1::cat*) and selecting for resistance to chloramphenicol. The resulting Transformants were screened for sensitivity to tetracycline, and two Tetsensitive isolates named PA715 were saved. This strain is the host strain for testing the *P26 panBpanD* vectors (see below). In order to restore the *P26 panE1* cassette in PA715, each vector was first transformed into a strain (PA328) that contains *P26 panE1* but does not contain a cassette integrated at the *bpr* locus. PA328 does contain the *P26 panBCD* locus although it is not engineered for overproduction of α-KIV. Transformants of PA328 resistant to tetracycline were obtained using the appropriate *Notl* restriction fragments from the two vectors and the resulting strains were named PA710 and PA714.

The next step was to transfer the cassettes into PA715 so they could be evaluated in the PA824 strain background. This was accomplished by isolating chromosomal DNA from strains PA710 and PA714 and using each of the two DNAs separately to transform PA715, with selection for resistance to tetracycline. Tetracycline-resistant transformants were screened for sensitivity to chloramphenicol; this identifies the desired transformants that have also acquired the *P26 panE1* gene from the donor DNA by linkage with the *P26 panBpanD* cassettes at the *bpr* locus. Chloramphenicol-sensitive isolates derived from transformations in which PA710 or PA714 chromosomal DNA was used as the donor were obtained. The isolates that produced the highest pantothenate titers in test tube culture assays were saved. These strains were named PA717 and PA721, respectively. Duplicate test tube cultures of the new strains, as well as PA824 and PA715, were grown in SVY + 10 g/L aspartate at 43°C for 48 hours and then assayed for pantothenate, HMBPA, and β-alanine. In addition, extracts from each of the strains were run on a SDS-PAGE gel. The results of the test tube culture assays are presented in Table 2.

**Table 2. Production of pantothenate by strains PA717 and PA721 grown in SVY plus 10 g/l aspartate.**

| Strain | *panBD* cassette | [pan] (g/L) | [HMBPA] (g/L) | [β-ala] (g/L) |
|---|---|---|---|---|
| PA824 | - | 4.9 | 0.94 | 2.5 |
| " | | 4.6 | 0.79 | 2.3 |
| | | | | |
| PA715 | NONE | 1.7 | <0.1 | 0.5 |
| " | " | 1.7 | <0.1 | 0.4 |
| | | | | |
| PA717-24 | pAN670 | 4.8 | 0.34 | 1.3 |
| " | " | 4.9 | 0.40 | 1.3 |
| | | | | |
| PA721-35 | pAN674 | 5.7 | 0.50 | 1.4 |
| " | " | 5.3 | 0.40 | 1.3 |
| | | | | |
| PA721-39 | pAN674 | 4.1 | 0.38 | 2.0 |
| " | " | 4.6 | 0.40 | 2.2 |

As expected, each of the new strains produced more pantothenate and β-alanine than PA715. Two of the strains (PA717-24 and PA721-39) produced about as much pantothenate as PA824 while PA721-35 produced more pantothenate than PA824. All three of the new strains produced less HMBPA than PA824. The protein gel analysis showed that the three new strains produce more PanB than any of the control strains.

Strains PA717-24, PA721-35, and PA721-39 were also evaluated in shake flask cultures in a soy flour based medium. As shown in Table 3, these strains with the amplifiable *P₂₆ panBpanD* cassette produced pantothenate and HMBPA at levels similar to the levels seen with PA668-2 and PA668-24 which both contain separate amplifiable *P₂₆ panB* and *P₂₆ panD* cassettes.

**Table 3. Shake Flask Experiment 48 Hours**

| Medium | Strain | HMBPA (g/l) | PAN (g/l) |
|---|---|---|---|
| Soy flour + Glucose | PA668-2 | 1.2 | 6.8 |
| | PA668-24 | 1.6 | 5.2 |
| | PA717-24 | 2.0 | 5.9 |
| | PA721-35 | 2.6 | 7.0 |
| | PA721-39 | 2.5 | 8.6 |
| Soy flour + Maltose | PA668-2 | 0.0 | 9.0 |
| | PA668-24 | 0.4 | 10.4 |
| | PA717-24 | 0.7 | 8.6 |
| | PA721-35 | 1.0 | 9.2 |
| | PA721-39 | 0.4 | 9.1 |

| | | | |
|---|---|---|---|
| Conditions: 40ml medium / 200ml baffled shake flask, 4X Bioshield covers, 300 rpm, 2.5% inoculum (1.0 ml). Soy Medium: 20 g/l Cargill 200/20 soy Hour, 8 g/l (NH4)2SO4, 5g/l glutamate, 1x PSTE, 0.1 M phosphate pH 7.2 and 0.3M MOPS pH 7.2. 60 g/l glucose or maltose w/ 10 mM Mg and 1.4 mM Ca. Average of duplicate flasks. | | | |

In addition to producing pantothenate (as well as other panto-compounds depicted in Figure 1 and described herein), it has been demonstrated that certain strains engineered for producing commercial quantities of desired panto-compound also produce a by-product identified as 3-(2-hydroxy-3-methyl-butyrylamino)-propionic acid (HMBPA) (also referred to herein as "β-alanine 2-(*R*)-hydroxyisolvalerate", "β-alanine 2-hydroxyisolvalerate", "β-alanyl-α-hydroxyisovalarate" and/or "fantothenate"). (The term "fantothenate" is also abbreviated as "fan" herein.)

HMBPA is the condensation product of [R]-α-hydroxyisovaleric acid (a-HIV) and β-alanine, catalyzed by the PanC enzyme. α-HIV is generated by reduction of α-KIV, a reaction that is catalyzed by the α-keto reductases PanE (*e.g.*, PanE1 and/or PanE2) and/or IlvC. Thus it has been proposed that there exist at least two pathways in microorganisms that compete for α-KIV, the substrate for the biosynthetic enzyme PanB, namely the pantothenate biosynthetic pathway and the HMBPA biosynthetic pathway. (A third and fourth pathway competing for α-KIV are those resulting in the production of valine or leucine from α-KIV, see *e.g.*, Figure 1). At least the pantothenate biosynthetic pathway and the HMBPA biosynthetic pathway further produce competitive substrates for the enzyme PanC, namely α-HIV and pantoate. Production of HMBPA can have significant effects on pantothenate production. For example, the HMBPA pathway can compete with the pantothenate pathway for precursors (α-KIV and β-alanine) and for some of the enzymes (PanC, PanD, PanE1, and/or IlvC). In addition, because the structure of HMBPA is similar to that of pantothenate, it may have the undesirable property of negatively regulating one or more steps in the pantothenate pathway. Based on the identification of HMBPA, U.S. Provisional Patent Application Ser. No. 60/262,995 teaches that production of pantothenate can be improved or optimized by any means which favor use of substrates (α-KIV and β-alanine) and/or enzymes (PanC, PanD, PanE1, and/or IlvC) in pantothenate biosynthetic processes as compared to HMBPA biosynthetic processes.

### Example II: Increasing Pantothenate Production by Increasing Serine Availability

At least one method for optimizing pantothenate production involves regulating the availability of serine in the microorganism cultures. In particular, it can be demonstrated that increasing the availability of serine leads to increased pantothenate production (*e.g.*, relative to HMBPA production), whereas decreasing the availability of serine leads to decreased pantothenate production relative to HMBPA production. This method is based on the understanding that the compound, methylenetetrahydrofolate (MTF), which is derived from serine, donates a hydroxymethyl group to α-KIV during the pantothenate biosynthetic reaction to yield ketopantoate. (see *e.g.*, Figures 1 and 2). Thus, regulating serine levels is one means of effectively regulating ketopantoate levels and, in turn, regulating pantoate and/or pantothenate production in appropriately engineered microorganisms. To demonstrate this regulation, PA824 was grown in test tube cultures of SVY glucose plus 5 g/L β-alanine and ± 5 g/L serine for 48 hours and 43°C.

**Table 4: Production of pantothenate and HMBPA by PA824 with and without the addition of serine**

| serine added at 5 g/L | OD₆₀₀ | [pan] g/L | [HMBPA] g/L |
|---|---|---|---|
| - | 16.3 | 4.9 | 0.84 |
| - | 14.0 | 4.5 | 0.80 |
| + | 13.1 | 6.4 | 0.56 |
| + | 12.9 | 6.0 | 0.62 |

As demonstrated by the data presented in Table 4, addition of serine increases the level of production of pantothenate (while conversely decreasing HMBPA production).

### Example III. Engineering bacterial cells with increased amounts of serine hydroxylmethyl transferase, the glyA gene product.

As an alternative to feeding serine, another method of increasing serine levels and/or serine utilization levels (and accordingly, methylenetetrahydrofolate levels) in order to regulate pantothenate production levels is to increase synthesis or the activity of 3-phosphoglycerate dehydrogenase or of serine hydroxymethyl transferase (the *serA* and *glyA* gene products, respectively), thereby increasing serine and methylenetetrahydrofolate biosynthesis in appropriately engineered microorganisms.

Expression of the *glyA* gene was increased by transforming *B. subtilis* cells with an expression cassette containing the *B. subtilis g*/*yA* gene cloned downstream of a strong, constitutive promoter. To construct the expression cassette the primers RY417 and RY418 depicted in Table 5 were used to amplify the *glyA* gene by PCR from chromosomal DNA isolated from *B. subtilis* PY79.

**Table 5: Primers used in the amplification of B. subtilis glyA and serA**

| | | |
|---|---|---|
| RY405 | | SEQ ID NO:20 |
| RY406 | | SEQ ID NO:21 |
| RY417 | | SEQ ID NO:22 |
| RY418 | | SEQ ID NO:23 |

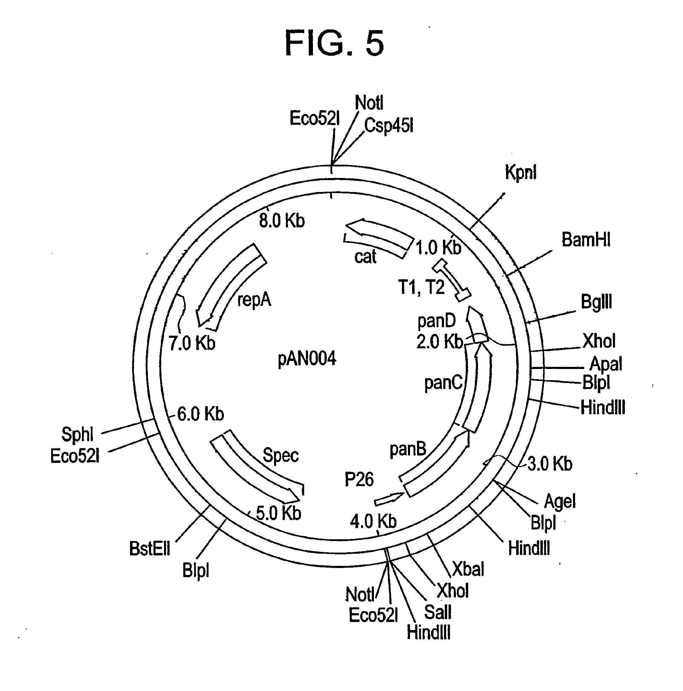

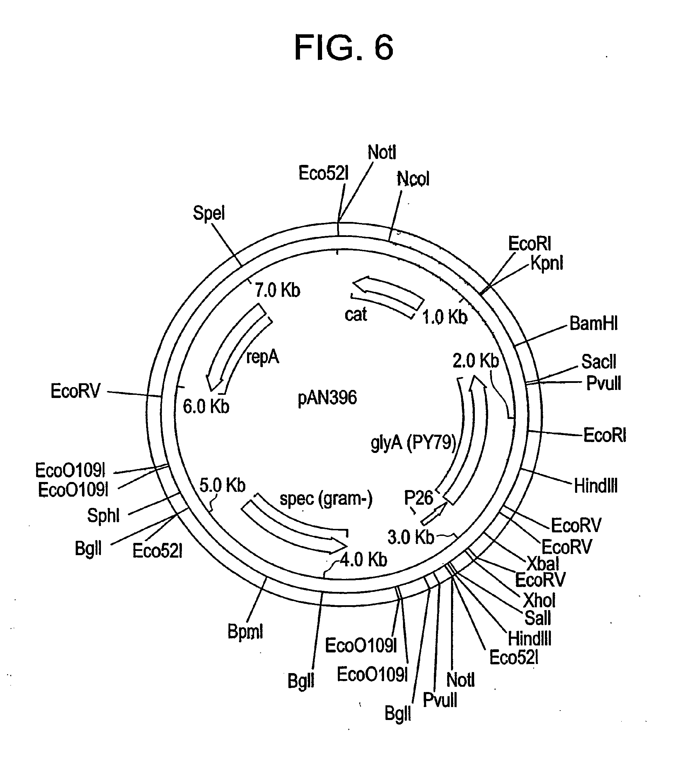

RY417 contains the RBS2 synthetic ribosome binding site just downstream from an *XbaI* site. The amplified DNA was then cut with *XbaI* and *BamHI* and cloned between the *XbaI* and *BamHI* sites in vector pAN004 (Figure 5) to yield plasmid pAN396 (Figure 6; SEQ ID NO:24). The pAN004 vector contains the phage SP01 P₂₆ promoter immediately upstream of the *XbaI* cloning site to drive expression of the cloned *glyA* gene. Just downstream of the expression cassette, pAN396 contains a *cat* gene that functions in *B. subtilis.* To transform *B. subtilis,* the *NotI* DNA fragment containing the *P₂₆ glyA* cassette and *cat* gene was isolated from pAN396, self ligated, and transformed into competent cells of *B. subtilis* PY79. Several chloramphenicol resistant transformants were selected and named PA1007 and PA1008. Chromosomal DNA was isolated from each of these strains and used to transform competent cells of PA721B-39 and PA824 to yield strains PA1011 and PA1014, respectively. SDS polyacrylamide gel electrophoresis of cell extracts of selected isolates of PA1011 and PA1014 confirmed that these strains contained increased amounts of the *glyA* gene product as compared to their parent strains PA721B-39 (described in Example I) and PA824 (described in International Public. No. WO 01/21772). To test the effect of increasing *glyA* expression on pantothenate production, PA101 and PA1014 were grown in test tube cultures of SVY glucose plus 5 g/L β-alanine at 43°C for 48 hours. As shown by the data presented in Table 6, PA 1014 produced more pantothenate (4.5 g/L) than its parent strain PA824 (3.2 g/L). Similarly, PA1011 produced on average more pantothenate (4.35 g/L) than its parent strain PA721B-39 (4.05 g/L).

**Table 6. Production of pantothenate and HMBPA by PA1011 and PA1014 compared to PA721B-39 and PA824.**

| Strain | OD₆₀₀ | Pantothenate g/L | HMBPA g/L |
|---|---|---|---|
| PA1014 #1 | 14 | 4.5 | 0.27 |
| PA1014 #2 | 15 | 4.5 | 0.31 |
| PA824 | 16 | 3.1 | 0.31 |
| PA824 | 15 | 3.3 | 0.28 |
| | | | |
| PA1011 #1 | 17 | 4.5 | 0.24 |
| PA1011 #2 | 12 | 4.2 | 0.27 |
| PA721B-39 | 18 | 4.0 | 0.22 |
| PA721B-39 | 16 | 4.1 | 0.25 |

### Example IV. Engineering bacterial cells with increased amounts of 3-phosphoglycerate dehydrogenase, the serA gene product.

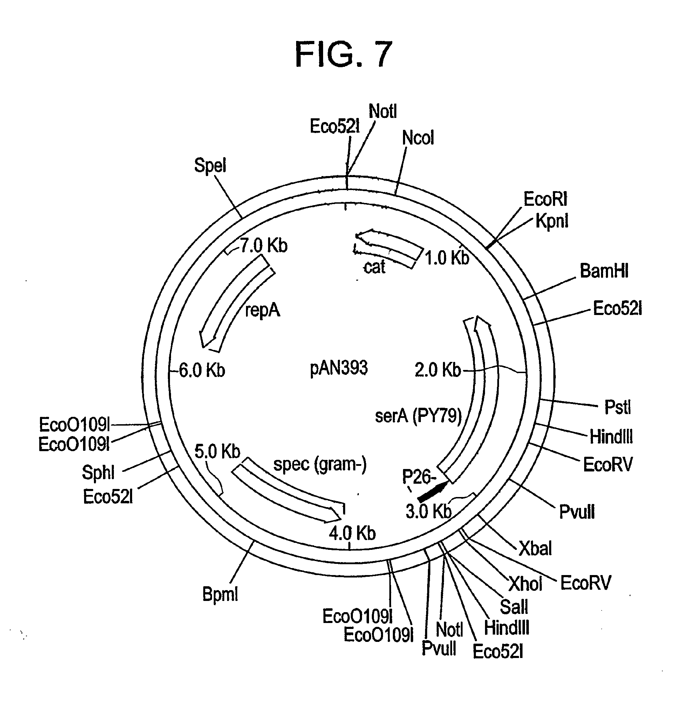

The product of the *serA* gene, 3-phosphoglycerate dehydrogenase, is the first committed enzyme in the pathway to serine biosynthesis (see Figure 2). Since serine is one of the substrates for the synthesis of MTF, we engineered the overexpression of the *serA* gene to increase serine levels in the cell. In a manner similar to that described above for the *glyA* gene in Example III, expression of the *serA* gene was increased by transforming *B. subtilis* cells with an expression cassette containing the *B. subtllis serA* gene cloned downstream of a strong, constitutive promoter. To construct the expression cassette the primers RY405 and RY406 depicted in Table 5 were used to amplify the *serA* gene by PCR from chromosomal DNA isolated from *B. subtilis* PY79. The amplified DNA was then cut with *XhaI* and *BamHI* and cloned between the *XbaI* and *BamHI* sites in vector pAN004 (Figure 5) to yield plasmid pAN393 (Figure 7; SEQ ID NO:25). To transform *B. subtilis*, the *NotI* DNA fragment containing the *P₂₆ serA* cassette and *cat* gene was isolated from pAN393, self-ligated, and transformed into competent cells *of B. subtilis* PY79. Several chloramphenicol resistant transformants were selected and named PA1004 and PA1005. Chromosomal DNA was isolated from each of these strains and used to transform competent cells of PA721B-39 and PA824 to yield strains PA1010 and PA1013, respectively. SDS polyacrylamide gel electrophoresis of cell extracts of selected isolates of PA1010 and PA1013 confirmed that these strains contained increased amounts of the *serA* gene product as compared to their parent strains PA721 B-39 and PA824.

To test the effect of increasing *serA* expression on pantothenate production, PA1010 and PA1013 were grown in test tube cultures of SVY glucose plus 5 g/L β-alanine at 43°C for 48 hours. As shown by the data presented in Table 7, PA1010 produced on average more pantothenate (4.7 g/L) than its parent strain PA721B-39 (4.1 g/L). Similarly, PA1013 produced on average more pantothenate (4.1 g/L) than its parent strain PA824 (3.1 g/L).

**Table 7. Production of pantothenate and HMBPA by PA1010 and PA1013 compared to PA721B-39 and PA824.**

| Strain | OD₆₀₀ | Pantothenate g/L | HMBPA g/L |
|---|---|---|---|
| PA1010 #3 | 16 | 4.8 | 0.23 |
| PA1010 #5 | 15 | 4.5 | 0.26 |
| PA1010 #6 | 22 | 4.7 | 0.24 |
| PA721B-39 | 18 | 4.0 | 0.22 |
| PA721B-39 | 16 | 4.1 | 0.25 |
| | | | |
| PA1013 #2 | 14 | 3.3 | 0.25 |
| PA1013 #4 | 14 | 4.2 | 0.28 |
| PA1013 #5 | 16 | 5.5 | 0.37 |
| PA1013 #8 | 13 | 3.6 | 0.24 |
| PA824 | 17 | 3.0 | 0.27 |
| PA824 | 16 | 3.1 | 0.29 |

### Example V. Shake flask and fermentor experiments with strains with increased expression of serA and glyA.

Based on performance in test tubes, two strains with an amplifiable *serA* cassette and two strains with an amplifiable *glyA* cassette were selected, one each from two parents, PA824 and PA721B-39. The four strains were grown beside the parents in shake flasks (Table 8). In Soy flour MOPS Glucose (SMG) medium, all of the 4 strains produced more pantothenate than their parent strains. In Soy flour MOPS Maltose (SMM) medium one out of the four strains appeared superior to the parent strain.

The *serA* overexpressing strain and the *glyA* overexpressing strain from each parent were run simultaneously in 10-liter Chemap bench fermentors. The *glyA* overexpressing strain derived from PA824, PA1014-3, that had given the highest pantothenate titer in SMM, also performed the best in fermentors (Table 9). Strain PA1014-3 produced 71 g/l pantothenate in 36 hours in the culture supernatant and 86 g/l pantothenate in 48 hours in the culture supernatant compared to the parent PA824 which produced 41 g/l and 46 g/l pantothenate, respectively. The *serA* strain, PA1012-4, also produced significantly more pantothenate than the PA824 control in the culture supernatant, 52 g/l and 60 g/l at 36 and 48 hours, respectively. These results clearly demonstrate the effectiveness of increasing both *glyA* and *serA.*

The *serA* overexpressing and *glyA* overexpressing derivatives of PA721B-39 were clearly improved over their parent strain as well. Both produced about 80 g/l pantothenate (82 g/l and 79 g/l, respectively) in the culture supernatants in 48 hours. The effect of the increased PanB levels in the PA721B-39 derivatives versus the PA824 derivatives manifests itself in the reduction of HMBPA. PA721B-39 and its derivatives produce less HMBPA after 48 hours than PA824 or even PA668-24. Increasing GlyA also appears to lower the flow of carbon to HMBPA.

**Table 8. Shake flask evaluation of pantothenate production strains overexpressing ser A or glyA.**

| **Carbon source** | **Strain** | **Added cassette** | **HMBPA (g/l)** | **Pantothenate (g/l)** |
|---|---|---|---|---|
| Glucose | PA824 | | 3.5 | 4.0 |
| | PA1012-4 | *serA* | 3.0 | 4.6 |
| | PA1014-3 | *gly A* | 2.5 | 4.7 |
| | PA721B-39 | | 0.9 | 5.0 |
| | PA1010-6 | *serA* | 1.9 | 9.6 |
| | PA1011-2 | *glyA* | 1.7 | 10.0 |
| Maltose | PA824 | | 1.2 | 10.4 |
| | PA1012-4 | *serA* | 0.8 | 9.8 |
| | PA1014-3 | *gly A* | 1.1 | 16.1 |
| | PA721B-39 | | 0.6 | 11.6 |
| | PA1014-6 | *serA* | 0.5 | 10.2 |
| | PA1011-2 | *gly A* | 0 | 10.3 |

| | | | | |
|---|---|---|---|---|
| All data are the average of duplicate shake flasks after 48 hours. Conditions: 40ml medium / 200ml baffled shake flask, 4X Bioshield covers, 300 rpm, 2.5% inoculum and 43°C. Medium: 20 g/l Cargill 200/20 soy flour, 1 x PSTE, 8 g/l (NH4)2SO4 and 5g/l glutamate. Buffer: 0.1 M phosphate pH 7.2 and 0.3M MOPS pH 7.2. Carbon Source (Sterilized separately as 20 x stock): 60 g/l glucose or maltose w/ 10 mM Mg and 1.4 mM Ca. | | | | |

**Table 9. 10 liter fermenter evaluations of pantothenate production strains overexpressing serA or glyA.**

| | | | | **HMBPA (g/l)** | | **Pantothenate (g/l)** | |
|---|---|---|---|---|---|---|---|
| **run** | **Strain** | **Parent** | **Added cassette** | **36 hrs** | **48 hrs** | **36 hrs** | **48 hrs** |
| P285 | PA824 | | | 18 | 25 | 41 | 46 |
| P284 | PA1012-4 | PA824 | *serA* | 20 | 21 | 52 | 60 |
| P286 | PA1014-3 | PA824 | *glyA* | 14 | 16 | 71 | 86 |
| P259 | PA721B-39 | | | 4 | 5 | 34 | 42 |
| P287 | PA1010-6 | PA721B-39 | *serA* | 4 | 5 | 65 | 82 |
| P289 | PA1011-2 | PA721B-39 | *glyA* | 2 | 3 | 56 | 79 |
| P275 | PA668-24 | PA824 | | 3 | 9 | 55 | 72 |

| | | | | | | | |
|---|---|---|---|---|---|---|---|
| The medium used is PFM-222. It is the same as medium PFM-155 described in U.S. Ser. No. 60/262,993 (filed January 19, 2001) except for the following changes: (I) In the Batch Material: There is no Amberex 1003. Cargill 200/20 (soy flour) 40 g/L has been changed to Cargill 20-80 (soy grits) 50 g/L, MgSO₄·7H₂O is replaced with MgCl₂·7H₂0, 1 g/L, and SM-1000X is replaced with PSTE-1000X (PSTE-1000X = MnCl₂·4H₂O, 2.0 g/L; ZnSO₄·7H₂O, 1.5 g/L; CoCl₂·6H₂O, 2.0 g/L; CuSO₄·5H₂O, 0.25 g/L; Na₂MoO₄·2H₂O, 0.75 g/L). In the Feed Maternal: SM-1000X is replaced with PSTE-1000X | | | | | | | |

Increasing pantothenate production can also be achieved by combining overexpression of *serA* and *glyA* in a single strain, and/or by introducing a mutation that leads to feedback resistant *serA* or *glyA,* or both.

### Example VI. Increasing the expression of the glyA gene by mutating the purR gene.

As described in Examples III and V, expression of the *glyA* gene can be increased by adding one or more copies of a cassette in which the *glyA* gene is driven by a strong, constitutive promoter. An alternative method to increase *glyA* expression is to alter its regulation. Literature describing a *glyA::lacZ* fusion suggests that the *glyA* promoter is of moderate strength under normal conditions (about 400 Miller Units), but that this promoter is capable of being induced to relatively high levels (1,800 Miller units) if its negative regulator, the *purR* gene, is deleted (Saxild et al. (2001) J. Bacteriol. 183:6175-6183). Therefore, experiments were preformed to determine if *glyA* expression, and consequently pantothenate production, could be increased by deleting *purR* from a pantothenate production strain.

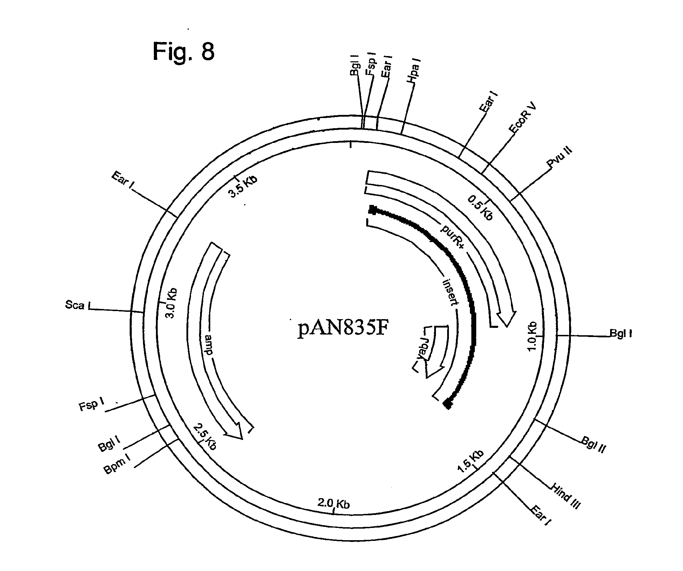

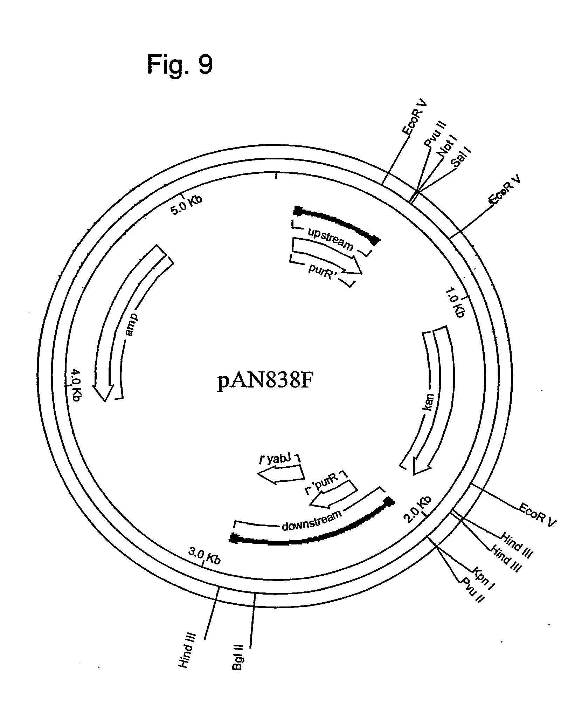

The *B. subtilis purR* gene was amplified from PY79 chromosomal DNA by PCR, and the resulting fragment was cloned into PvuII cleaved pGEM5-Zf(+) vector DNA to give plasmid pAN835F (SEQ ID NO:26, Figure 8). This step eliminated the PvuII sites at both ends of the insert, leaving a unique PvuII site in the middle of the *purR* open reading frame. Next, a blunt PCR DNA fragment containing the Gram positive kanamycin resistance gene from pAN363F (SEQ ID NO:27) was ligated into this unique PvuII site of pAN835F to give pAN838F (SEQ ID NO:28, Figure 9).

pAN838F was then transformed into PY79, PA668-24, and PA824, selecting for kanamycin resistance at 10 mg/l to give new sets of strains named PA1059, PA1060, and PA1061, respectively. It was shown by PCR that all new isolates contained the disrupted *purR::kan* allele that was expected from a double crossover event. Several isolates of PA1060 and PA1061 were tested for pantothenate production in test tube cultures grown in SVY glucose plus β-alanine (Table 9). The best isolates derived from PA668-24, PA1060-2 and PA1060-4, gave an improvement from 3.0 g/l pantothenate to 5.3 to 5.1 g/l, respectively, which is an increase of 75%. Likewise, the best isolates derived from PA824, PA1061-1 and PA1061-2 gave an increase from about 3.1 g/l to 5.4 g/l, also a 75% gain. These results suggest that the *glyA* gene is substantially induced in these new strains by disruption of the *purR* gene. Alternatively, the improvements in pantothenate production in PA1060 and PA1061 may be due to more complex pleiotropic effects. In either case, deregulation of the *purR* regulon has a positive effect on pantothenate production.

In other embodiments, the *purR* disruption can be installed in other pantothenate production strains, for example those that have an integrated *P26serA* allele or more than one copy of the *P₂₆panBCD* operon. The *purR* gene can also be used as a site for addition of desired expression cassettes, such as *P₂₆panB*. One can also use resistance to the guanine analogs, such as 8-azaguanine, as a selection for a *purR* mutation.

**Table 10. Production of pantothenate and fantothenate by derivatives of PA824 and PA668-24 containing disrupted purR, in test tube cultures grown in SVY glucose plus 5 g/l β-alanine.**

| Strain | inoculum* | parent | new feature | OD₆₀₀ | [fan] g/l | [pan] g/l |
|---|---|---|---|---|---|---|
| PA668-24 | cam 5, tet 7.5 | PA824 | - | 9 | b.d. | 3.0 |
| " | " | " | - | 12 | b.d. | 3.0 |
| PA1060-1 | cam 5, tet 7.5 | PA668-24 | *purR::kan* | 14 | 0.14 | 4.5 |
| PA1060-2 | " | " | " | 12 | b.d. | 5.3 |
| PA1060-3 | " | " | " | 12 | b.d. | 4.5 |
| PA1060-4 | " | " | " | 16 | 0.11 | 5.1 |
| PA824 | tet 30 | PA377 | - | 9 | 0.25 | 3.2 |
| " | " | " | - | 11 | 0.22 | 3.0 |
| PA1061-1 | tet 15 | PA824 | *purR::kan* | 13 | 0.45 | 5.4 |
| PA1061-3 | " | " | " | 14 | 0.39 | 5.4 |
| PA10614 | " | " | " | 11 | 0.40 | 4.7 |

| | | | | | | |
|---|---|---|---|---|---|---|
| b.d. = below detection * Concentration of antibiotics in the petri plate from which the inoculum colony was taken. | | | | | | |

### Example VII. Overexpression of the serA gene from a non-amplifiable cassette.

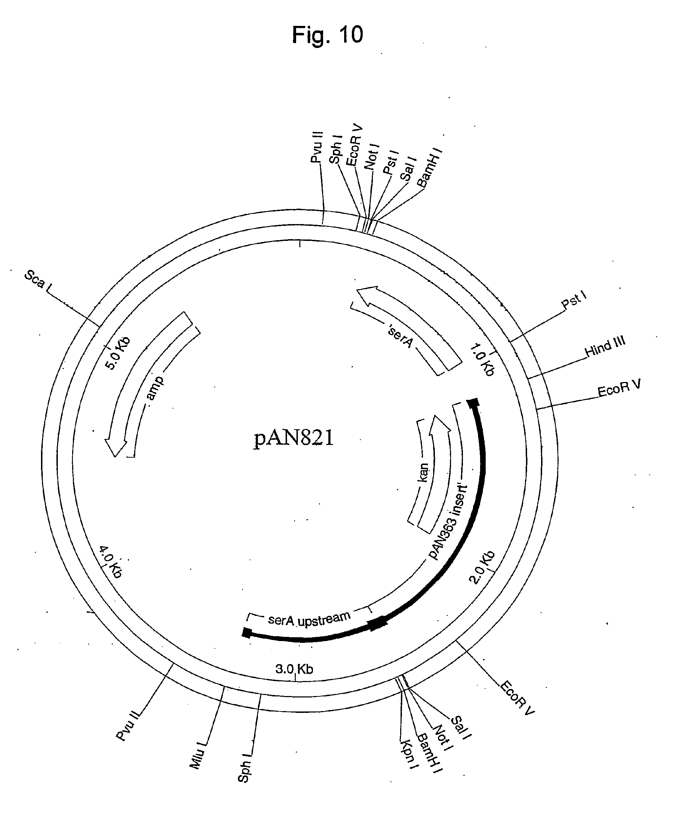

This Example describes another method to increase serine production, in which a two step procedure deposits a strong, constitutive promoter (*P₂₆*) in front of the chromosomal *serA* gene. Two plasmids were constructed, each containing about 700 base pairs of DNA sequence from the region immediately upstream of the native *serA* gene. The first plasmid, pAN821, also contains the 3' half of the *serA* coding region, and in between the two aforementioned sequences, a kanamycin resistance gene (SEQ ID NO:30, Figure 10). When transformed into *B. subtilis,* selecting for kanamycin resistance, pAN821 will give a disruption of the *serA* gene, leading to serine auxotrophy. This creates a genetic sequence termed the *ΔserA::kan* allele.

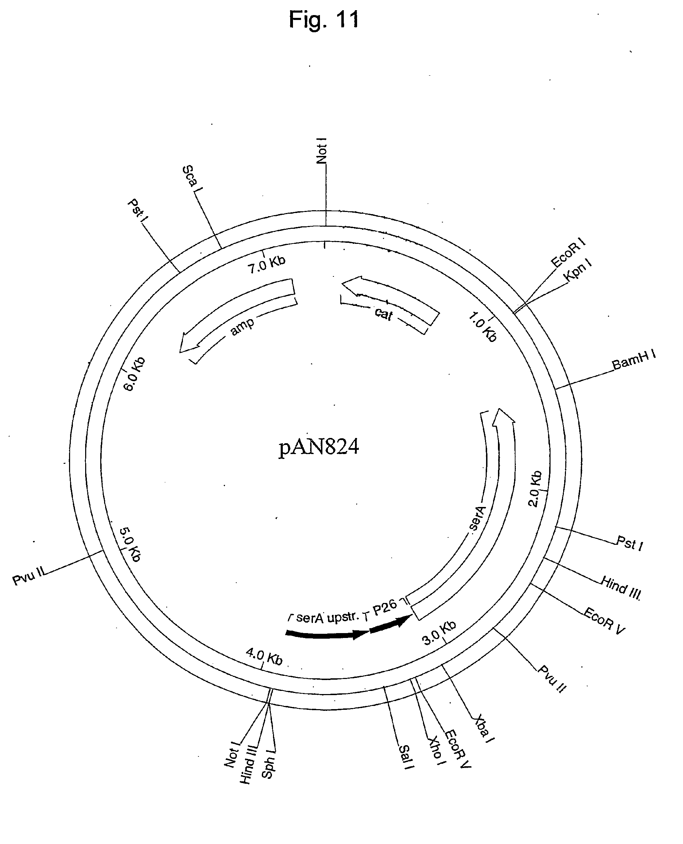

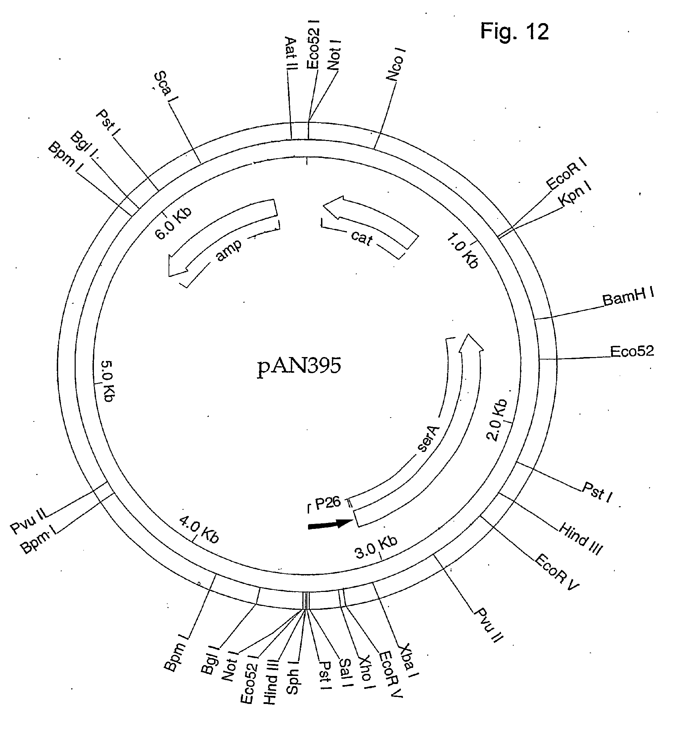

The second plasmid, designed to introduce the *P₂₆ serA* structure, was constructed by inserting the *serA* upstream sequence at the 5' end of the *P₂₆* promoter in pAN395. The resulting plasmid, pAN824, is shown in Figure 11 (SEQ ID NO:31). The plasmid pAN395 is similar to pAN393 described in Example IV. The open reading frame of the *serA gene* was synthesized by PCR using *B. subtilis* PY79 DNA as the template. The upstream primer contains an *X*baI site and a moderately strong synthetic ribosome binding site, RBS2. The downstream primer contains a *BamHI* site. This *serA* open reading frame was used to replace the *panBCD* genes in the medium copy plasmid, pAN006, to give pAN395 (SEQ ID NO:29, Figure 12). This plasmid contains the *serA* gene expressed from the *P₂₆* promoter and the RBS2 ribosome binding site.

The *ΔserA::kan* allele from pAN821 was introduced into strain PA824 to give PA 1026. As expected, PA 1026 did not grow on minimal medium. In the second step, the *P₂₆ serA* cassette from plasmid pAN824 was introduced into PA 1026, selecting for serine prototrophy, to give strain PA1028. Several PA1028 isolates were confirmed to have the expected chromosomal structure *(P₂₆ serA)* by diagnostic PCR. These isolates were then tested for pantothenate production in test tube cultures grown for 48 hours in SVY plus 5 g/l β-alanine (Table 11). The PA1028 isolates (derived from PA824) gave increases from 10% to 25% in pantothenate production. As shown in Table 12, in shake flask experiments, PA824 produced about 7 g/l pantothenate, whereas PA1028 produced 11 g/l.

### Example VIII. Construction of pantothenate producing strains that contain both an integrated non-amplifiable P₂₆ serA cassette and an amplifiable P₂₆ glyA cassette.

Since a non-amplifiable *P₂₆ serA* cassette integrated at *serA* led to higher pantothenate synthesis (see, *e.g*., Table 12), and since a chloramphenicol amplifiable *P₂₆ glyA* cassette *at glyA* led to much higher pantothenate synthesis (see, *e.g.,* PA1014-3, Table 8), it was proposed that a combination of the two might be synergistic. Strain PA1028-4, which is the derivative of PA824 that contains the non-amplifiable *P₂₆ serA* cassette integrated at *serA,* was transformed to chloramphenicol resistance at 5 mg/l using chromosomal DNA from PA 1014-3, to give a set of strains named PA1038, which now contain the chloramphenicol amplifiable *P₂₆ glyA* cassette. PA1038 isolates were tested for pantothenate production using standard test tube cultures grown in SVY plus β-alanine (Table 13). As expected, PA1038 showed a dramatic increase in pantothenate production from about 4.2 g/l by PA824 to 6.6 to 7.5 g/l by the PA1038 set. Isolates PA1038-3 and PA1038-12 were further tested in shake flasks as shown in Table 12. Both produced an average of 13.6 g/l pantothenate, as compared to the 7.4 g/l pantothenate produced by PA824.

**Table 11. Production of pantothenate and fantothenate by derivatives of PA824 that contain a single copy of P₂₆ serA at the serA locus, in 48 hour test tube cultures grown in SVY plus 5 g/l β-alanine.**

| **Strain** | **parent** | **OD₆₀₀** | **[fan] g/l** | **[pan] g/l** |
|---|---|---|---|---|
| PA824 | | 17 | 0.44 | 4.0 |
| PA824 | | 15 | 0.45 | 4.0 |
| PA1028-1 | PA824 | 13 | 0.46 | 4.4 |
| PA1028-2 | " | 18 | 0.49 | 4.9 |
| PA 1028-3 | " | 15 | 0.44 | 4.4 |
| PA 1028-4 | " | 13 | 0.43 | 4.5 |
| PA1028-5 | " | 14 | 0.45 | 4.4 |
| PA1028-6 | " | 11 | 0.43 | 4.8 |
| PA1028-8 | " | 15 | 0.51 | 5.0 |

| | | | | |
|---|---|---|---|---|
| b.d. = below detection | | | | |

**Table 12. Shake flask evaluation of pantothenate production strains overexpressing serA and/or glyA.**

| **Strain** | **Parent** | ***glyA* cassette** | ***serA* cassette** | **Fantothenate (g/l)** | **Pantothenate (g/l)** |
|---|---|---|---|---|---|
| PA824 | | | | 0.6 | 7.4 |
| PA1014-3 | PA824 | N x P₂₆*glyA* | | 0.7 | 12.0 |
| PA1028-4 | PA824 | | *P₂₆serA@serA* | 0.8 | 11.1 |
| PA1038-3 | PA1028-4 | N x P₂₆*glyA* | *P_{w26} serA @ serA* | 0.5 | 13.6 |
| PA1038-12 | PA1028-4 | N x P₂₆*glyA* | *P₂₆serA @ serA* | 0.6 | 13.6 |
| | | | | | |

| | | | | | |
|---|---|---|---|---|---|
| All data are the average of duplicate shake flasks after 48 hours. Conditions: 40 ml medium / 200 ml baffled shake flask, 4X Bioshield covers, 300 rpm, 2.5% inoculum and 43°C. Inoculum: SVY base w/maltose 24 hours at 43°C. Medium: 20 g/l Cargill 200/20 soy flour, 8 g/l (NH₄)₂SO₄, 5g/l glutamate and 1xPSTE. Buffer: 0.1M phosphate pH 7.2 and 0.3M MOPS pH 7.2. Carbon Source (Sterilized separately as 20X stock): 30 g/l maltose, 5 mM MgCl₂ and 0.7 mM CaCl₂. | | | | | |

**Table 13. Pantothenate production by PA1038, a derivative of PA824 that contains a non-amplifiable P₂₆ serA cassette at serA and an amplifiable P₂₆ glyA cassette at glyA.**

| Strain | Inoculum Medium | OD₆₀₀ | [Fan] g/L | [Pan] g/L |
|---|---|---|---|---|
| PA824 | tet 15 | 16 | 0.56 | 4.4 |
| PA824 | " | 14 | 0.59 | 4.3 |
| PA824 | tet 30 | 12 | 0.57 | 4.3 |
| PA824 | " | 14 | 0.58 | 4.2 |
| PA1038-3 | cam 5, tet 15 | 16 | 0.47 | 7.2 |
| PA1038-4 | " | 14 | 0.49 | 7.0 |
| PA1038-5 | " | 15 | 0.52 | 7.0 |
| PA1038-6 | " | 15 | 0.51 | 7.2 |
| PA1038-9 | " | 14 | 0.56 | 7.2 |
| PA1038-11 | " | 13 | 0.49 | 6.6 |
| PA1038-12 | " | 16 | 0.58 | 7.5 |

| | | | | |
|---|---|---|---|---|
| Test tube cultures were grown with SVY glucose plus 5 g/l β-alanine at 43°C for 48 hours. | | | | |

### Example IX. Increasing the production of MTF by altering the glycine cleavage pathway.

As demonstrated with the above examples, increasing MTF production in bacteria increases the production of pantothenate in strains that have been engineered to produce more pantothenate by manipulation of the *panBCD and*/*or panE* genes. It has been demonstrated that pantothenate production can be increased by increasing the expression of the *glyA* or the *serA* gene. Stronger promoters or ribosome binding sites can be used to increase *glyA* or *serA* expression as demonstrated in Examples III through V and VII through VIII. Alternatively, the expression of the *glyA* gene can be deregulated in *Bacillus* by disrupting the *purR* repressor gene as illustrated in Example VI.

Another method to increase MTF production is to enhance the expression of enzymes of the glycine cleavage pathway. For example, enzymes encoded by the *gcvT, gcvPA, gcvPB, gcvH , and pdhD* genes catalyze the breakdown of glycine to MTF, CO₂, and NH₃. A strong, constitutive promoter, such as the SP01 phage *P₂₆* promoter described previously, can be cloned in front of the *gcvT-gcvPA-gcvPB* operon or in front of the *gcvH or pdhD* gene to enhance their expression. In addition to the above mentioned approaches, additional glycine, which is inexpensive, can be added to the medium to further enhance MTF production by any strain engineered as described herein.

Equivalents Those skilled in the art will recognize, or be able to ascertain using no more than routine experimentation, many equivalents to the specific embodiments of the invention described herein. Such equivalents are intended to be encompassed by the following claims.

## Claims

1. A process for the enhanced production of pantothenate as compared to pantothenate production in said microorganism prior to deregulation, comprising culturing a microorganism having a deregulated methylenetetrahydrofolate (MTF) biosynthetic pathway, under conditions such that pantothenate production is enhanced, wherein the microorganism has a deregulated serine hydroxymethyltransferase gene and a mutated, deleted or disrupted purine repressor gene modulating repression of the serine hydroxymethyltransferase gene.

2. A process for the enhanced production of pantothenate as compared to pantothenate production in said microorganism prior to deregulation, comprising culturing a microorganism having (i) a deregulated pantothenate biosynthetic pathway, and (ii) a deregulated methylenetetrahydrofolate (MTF) biosynthetic pathway, under conditions such that pantothenate production is enhanced as compared to pantothenate production in said microorganism prior to deregulation, wherein the microorganism has a deregulated serine hydroxymethyltransferase gene and a mutated, deleted or disrupted purine repressor gene modulating repression of the serine hydroxymethyltransferase gene.

3. The process of claim 2, wherein said microorganism has at least two pantothenate biosynthetic enzymes deregulated.

4. The process of claim 2, wherein said microorganism has at least three pantothenate biosynthetic enzymes deregulated.

5. The process of claim 2, wherein said microorganism has at least four pantothenate biosynthetic enzymes deregulated.

6. The process of claim 5, wherein said microorganism has a deregulated ketopantoate hydroxymethyltransferase, a deregulated ketopantoate reductase, a deregulated pantothenate synthetase and a deregulated aspartate-alpha-decarboxylase.

7. The process of any one of claims 1 to 6, wherein said microorganism further has a deregulated isoleucine-valine (ilv) biosynthetic pathway.

8. The process of claim 7, wherein said microorganism has at least two isoleucine-valine (ilv) biosynthetic enzymes deregulated.

9. The process of claim 7, wherein said microorganism has at least three isoleucine-valine (ilv) biosynthetic enzymes deregulated.

10. The process of claim 9, wherein said microorganism has a deregulated acetohydroxyacid acid synthetase, a deregulated acetohydroxyacid isomeroreductase, and a deregulated dihydroxyacid dehydratase.

11. The process of any one of claims 1 to 10, wherein the microorganism has at least one MTF biosynthetic enzyme deregulated.

12. The process of claim 11, wherein the microorganism has a deregulated serA gene.

13. The process of claim 11, wherein the microorganism has a deregulated serine hydroxymethyltransferase gene and a deregulated serA gene.

14. The process of any one of claims 1 to 13 for the enhanced production of pantothenate as compared to pantothenate production in said microorganism prior to deregulation, comprising culturing a microorganism having a deregulated pantothenate biosynthetic pathway, a deregulated isoleucine-valine (ilv) biosynthetic pathway, and a deregulated methylenetetrahydrofolate (MTF) biosynthetic pathway deregulated, such that production of pantothenate is enhanced as compared to pantothenate production in said microorganism prior to deregulation.

15. The process for the production of pantothenate of any one of claims 1 to 14, comprising culturing a microorganism having a deregulated pantothenate biosynthetic pathway, a deregulated isoleucine-valine (ilv) biosynthetic pathway, and a deregulated methylenetetrahydrofolate (MTF) biosynthetic pathway, such that at least 50 g/L pantothenate is produced after 36 hours of culturing the microorganism, wherein the microorganism has a deregulated serine hydroxymethyltransferase gene and a mutated, deleted or disrupted purine repressor gene.

16. The process of claim 15, comprising culturing the microorganism such that at least 60 g/L pantothenate is produced after 36 hours of culturing the microorganism.

17. The process of claim 15, comprising culturing the microorganism such that at least 70 g/L pantothenate is produced after 36 hours of culturing the microorganism.

18. The process for the production of pantothenate of any one of claims 1 to 14, comprising culturing a microorganism having a deregulated pantothenate biosynthetic pathway, a deregulated isoleucine-valine (ilv) biosynthetic pathway, and a deregulated methylenetetrahydrofolate (MTF) biosynthetic pathway deregulated, such that at least 60 g/L pantothenate is produced after 48 hours of culturing the microorganism, wherein the microorganism has a deregulated serine hydroxymethyltransferase gene and a mutated, deleted or disrupted purine repressor gene.

19. The process of claim 18, comprising culturing the microorganism such that at least 70 g/L pantothenate is produced after 48 hours of culturing the microorganism.

20. The process of claim 18, comprising culturing the microorganism such that at least 80 g/L pantothenate is produced after 48 hours of culturing the microorganism.

21. The process of any one of the preceding claims, wherein pantothenate production is further enhanced by regulating pantothenate kinase activity.

22. The process of claim 21, wherein pantothenate kinase activity is decreased.

23. The process of claim 22, wherein CoaA is deleted and CoaX is downregulated.

24. The process of claim 22, wherein CoaX is deleted and CoaA is downregulated.

25. The process of claim 22, wherein CoaX and CoaA are downregulated.

26. The process of any one of the above claims, wherein said microorganism is cultured under conditions of excess serine.

27. A process for producing pantothenate comprising culturing a microorganism having a deregulated pantothenate biosynthetic pathway under conditions of excess serine, such that pantothenate in produced, wherein the microorganism has a deregulated serine hydroxymethyltransferase gene and a mutated, deleted or disrupted purine repressor gene.

28. The process of any one of the above claims, wherein said microorganism has the pantothenate biosynthetic pathway deregulated such that pantothenate production is independent of beta-alanine feed.

29. The process of any one of the above claims wherein the microorganism is a Gram positive microorganism.

30. The process of any one of the above claims wherein the microorganism belongs to the genus Bacillus.

31. The process of any one of the above claims, wherein the microorganism is Bacillus subtilis.

32. A recombinant microorganism for the enhanced production of pantothenate as compared to pantothenate production in said microorganism prior to deregulation, said microorganism having a deregulated pantothenate biosynthetic pathway, and a deregulated methylenetetrahydrofolate (MTF) biosynthetic pathway, wherein the microorganism has a deregulated serine hydroxymethyltransferase gene and a mutated, deleted or disrupted purine repressor gene.

33. A recombinant microorganism for the enhanced production of pantothenate as compared to pantothenate production in said microorganism prior to deregulation, said microorganism having a deregulated pantothenate biosynthetic pathway, a deregulated methylenetetrahydrofolate (MTF) biosynthetic pathway, and a deregulated isoleucine-valine (ilv) pathway, wherein the microorganism has a deregulated serine hydroxymethyltransferase gene and a mutated, deleted or disrupted purine repressor gene.

34. The microorganism of claim 32 or 33, further having reduced pantothenate kinase activity.

35. The microorganism of any one of claims 32-34 which is a Gram positive microorganism.

36. The microorganism of any one of claims 32-35 belonging to the genus Bacillus.

37. The microorganism of any one of claims 32-38 which is Bacillus subtilis.

38. A process for producing pantothenate comprising culturing a recombinant microorganism having: (a) a deregulated panB gene; (b) a deregulated panD gene ; and (c) at least one deregulated isoleucine-valine (ilv) biosynthetic enzyme-encoding gene; under conditions such that at least 30 g/l pantothenate is produced after 36 hours of culturing the microorganism, wherein the microorganism has a deregulated serine hydroxymethyltransferase gene and a mutated, deleted or disrupted purine repressor gene.

39. The process of claim 38, wherein said microorganism further has a deregulated methylenetetrahydrofolate (MTF) biosynthetic pathway and said microorganism is cultured under conditions such that at least 50 g/l pantothenate is produced after 36 hours of culturing the microorganism.

40. A process for producing pantothenate comprising culturing a recombinant microorganism having: (a) a deregulated panB gene; and (b) a deregulated panD gene; under conditions of excess serine, such that at least 50 g/l pantothenate is produced after 36 hours of culturing the microorganism, wherein the microorganism has a deregulated serine hydroxymethyltransferase gene and a mutated, deleted or disrupted purine repressor gene.

41. A process for producing pantothenate comprising culturing a recombinant microorganism having: (a) a deregulated panB gene; (b) a deregulated panD gene; and (c) a deregulated methylenetetrahydrofolate (MTF) biosynthetic pathway; under conditions of excess valine, such that at least 50 g/l pantothenate is produced after 36 hours of culturing the microorganism, wherein the microorganism has a deregulated serine hydroxymethyltransferase gene and a mutated, deleted or disrupted purine repressor gene.

42. A process for producing pantothenate comprising culturing a recombinant microorganism having: (a) a deregulated panB gene; (b) a deregulated panD gene; and (c) a deregulated serine hydroxymethyltransferase gene, wherein the microorganism has a mutated, deleted or disrupted purine repressor gene; under conditions of excess valine, such that at least 50 g/I pantothenate is produced after 36 hours of culturing the microorganism.

43. A process for producing pantothenate comprising culturing a recombinant microorganism having: (a) a deregulated panB gene; (b) a deregulated panD gene; and (c) a mutated, deleted or disrupted purine repressor gene; under conditions of excess valine, such that at least 50 g/I pantothenate is produced after 36 hours of culturing the microorganism.

44. A process for producing pantothenate comprising culturing a recombinant microorganism having: (a) a deregulated panB gene ; (b) a deregulated panD gene; and (c) a deregulated serA gene; under conditions of excess valine, such that at least 50 g/I pantothenate is produced after 36 hours of culturing the microorganism,
wherein the microorganism has a deregulated serine hydroxymethyltransferase gene and a mutated, deleted or disrupted purine repressor gene.

45. A process for producing pantothenate comprising culturing a recombinant microorganism having: (a) a deregulated panB gene; (b) a deregulated panD gene; (c) a deregulated serA gene; (d) a deregulated serine hydroxymethyltransferase gene; and under conditions of excess valine, such that at least 50 g/l pantothenate is produced after 36 hours of culturing the microorganism, wherein the microorganism has a mutated, deleted or disrupted purine repressor gene.

## Patentansprüche

1. Verfahren zur verglichen mit der Pantothenatproduktion in dem genannten Mikroorganismus vor der Deregulation verbesserten Produktion von Pantothenat, bei dem man einen Mikroorganismus mit einem deregulierten Methylentetrahydrofolat-(MTF-)Biosyntheseweg unter solchen Bedingungen kultiviert, dass die Pantothenatproduktion verbessert wird, wobei der Mikroorganismus ein dereguliertes Serin-Hydroxymethyltransferase-Gen und ein mutiertes, deletiertes oder unterbrochenes Purinrepressor-Gen, das die Repression des Serin-Hydroxymethyltransferase-Gens moduliert, aufweist.

2. Verfahren zur verglichen mit der Pantothenatproduktion in dem genannten Mikroorganismus vor der Deregulation verbesserten Produktion von Pantothenat, bei dem man einen Mikroorganismus mit (i) einem deregulierten Pantothenat-Biosyntheseweg und (ii) einem deregulierten Methylentetrahydrofolat-(MTF-)Biosyntheseweg unter solchen Bedingungen kultiviert, dass die Pantothenatproduktion verglichen mit der Pantothenatproduktion in dem genannten Mikroorganismus vor der Deregulation verbessert wird, wobei der Mikroorganismus ein dereguliertes Serin-Hydroxymethyltransferase-Gen und ein mutiertes, deletiertes oder unterbrochenes Purinrepressor-Gen, das die Repression des Serin-Hydroxymethyltransferase-Gens moduliert, aufweist.

3. Verfahren nach Anspruch 2, wobei in dem Mikroorganismus wenigstens zwei Enzyme der Pantothenat-Biosynthese dereguliert vorliegen.

4. Verfahren nach Anspruch 2, wobei in dem Mikroorganismus wenigstens drei Enzyme der Pantothenat-Biosynthese dereguliert vorliegen.

5. Verfahren nach Anspruch 2, wobei in dem Mikroorganismus wenigstens vier Enzyme der Pantothenat-Biosynthese dereguliert vorliegen.

6. Verfahren nach Anspruch 5, wobei der Mikroorganismus eine deregulierte Ketopantoat-Hydroxymethyltransferase, eine deregulierte Ketopantoat-Reduktase, eine deregulierte Pantothenat-Synthetase und eine deregulierte Aspartat-alpha-Decarboxylase aufweist.

7. Verfahren nach einem der Ansprüche 1 bis 6, wobei der Mikroorganismus ferner einen deregulierten Isoleucin-Valin-(ilv-)Biosyntheseweg aufweist.

8. Verfahren nach Anspruch 7, wobei in dem Mikroorganismus wenigstens zwei Enzyme der Isoleucin-Valin-(ilv-)Biosynthese dereguliert vorliegen.

9. Verfahren nach Anspruch 7, wobei in dem Mikroorganismus wenigstens drei Enzyme der Isoleucin-Valin-(ilv-)Biosynthese dereguliert vorliegen.

10. Verfahren nach Anspruch 9, wobei der Mikroorganismus eine deregulierte Acetohydroxysäure-Synthetase, eine deregulierte Acetohydroxysäure-Reductoisomerase und eine deregulierte Dihydroxysäure-Dehydratase aufweist.

11. Verfahren nach einem der Ansprüche 1 bis 10, wobei in dem Mikroorganismus wenigstens ein Enzym der MTF-Biosynthese dereguliert vorliegt.

12. Verfahren nach Anspruch 11, wobei der Mikroorganismus ein dereguliertes serA-Gen aufweist.

13. Verfahren nach Anspruch 11, wobei der Mikroorganismus ein dereguliertes Serin-Hydroxymethyltransferase-Gen und ein dereguliertes serA-Gen aufweist.

14. Verfahren nach einem der Ansprüche 1 bis 13 zur verglichen mit der Pantothenatproduktion in dem genannten Mikroorganismus vor der Deregulation verbesserten Produktion von Pantothenat, bei dem man einen Mikroorganismus mit einem deregulierten Pantothenat-Biosyntheseweg, einem deregulierten Isoleucin-Valin-(ilv-)Biosyntheseweg und einem deregulierten Methylentetrahydrofolat-(MTF-)Biosyntheseweg so kultiviert, dass die Produktion von Pantothenat verglichen mit der Pantothenatproduktion in dem genannten Mikroorganismus vor der Deregulation verbessert wird.

15. Verfahren zur Produktion von Pantothenat nach einem der Ansprüche 1 bis 14, bei dem man einen Mikroorganismus mit einem deregulierten Pantothenat-Biosyntheseweg, einem deregulierten Isoleucin-Valin-(ilv-)Biosyntheseweg und einem deregulierten Methylentetrahydrofolat-(MTF-)Biosyntheseweg so kultiviert, dass nach 36 Stunden Kultivierung des Mikroorganismus wenigstens 50 g/l Pantothenat produziert werden, wobei der Mikroorganismus ein dereguliertes Serin-Hydroxymethyltransferase-Gen und ein mutiertes, deletiertes oder unterbrochenes Purinrepressor-Gen aufweist.

16. Verfahren nach Anspruch 15, bei dem man den Mikroorganismus so kultiviert, dass nach 36 Stunden Kultivierung des Mikroorganismus wenigstens 60 g/l Pantothenat produziert werden.

17. Verfahren nach Anspruch 15, bei dem man den Mikroorganismus so kultiviert, dass nach 36 Stunden Kultivierung des Mikroorganismus wenigstens 70 g/l Pantothenat produziert werden.

18. Verfahren zur Produktion von Pantothenat nach einem der Ansprüche 1 bis 14, bei dem man einen Mikroorganismus mit einem deregulierten Pantothenat-Biosyntheseweg, einem deregulierten Isoleucin-Valin-(ilv-)Biosyntheseweg und einem deregulierten Methylentetrahydrofolat-(MTF-)Biosyntheseweg so kultiviert, dass nach 48 Stunden Kultivierung des Mikroorganismus wenigstens 60 g/l Pantothenat produziert werden, wobei der Mikroorganismus ein dereguliertes Serin-Hydroxymethyltransferase-Gen und ein mutiertes, deletiertes oder unterbrochenes Purinrepressor-Gen aufweist.

19. Verfahren nach Anspruch 18, bei dem man den Mikroorganismus so kultiviert, dass nach 48 Stunden Kultivierung des Mikroorganismus wenigstens 70 g/l Pantothenat produziert werden.

20. Verfahren nach Anspruch 18, bei dem man den Mikroorganismus so kultiviert, dass nach 48 Stunden Kultivierung des Mikroorganismus wenigstens 80 g/l Pantothenat produziert werden.

21. Verfahren nach einem der vorhergehenden Ansprüche, wobei die Pantothenatproduktion durch Regulieren der Pantothenatkinase-Aktivität weiter verbessert wird.

22. Verfahren nach Anspruch 21, wobei Pantothenatkinase-Aktivität verringert wird.

23. Verfahren nach Anspruch 22, wobei CoaA deletiert und CoaX herunterreguliert wird.

24. Verfahren nach Anspruch 22, wobei CoaX deletiert und CoaA herunterreguliert wird.

25. Verfahren nach Anspruch 22, wobei CoaX und CoaA herunterreguliert werden.

26. Verfahren nach einem der obigen Ansprüche, wobei der Mikroorganismus unter Serinüberschuss-Bedingungen kultiviert wird.

27. Verfahren zur Produktion von Pantothenat, bei dem man einen Mikroorganismus mit einem deregulierten Pantothenat-Biosyntheseweg unter Serinüberschuss-Bedingungen kultiviert, so dass Pantothenat produziert wird, wobei der Mikroorganismus ein dereguliertes Serin-Hydroxymethyltransferase-Gen und ein mutiertes, deletiertes oder unterbrochenes Purinrepressor-Gen aufweist.

28. Verfahren nach einem der obigen Ansprüche, wobei in dem Mikroorganismus der Pantothenat-Biosyntheseweg dereguliert vorliegt, so dass die Pantothenatproduktion von der Zufütterung von Beta-Alanin unabhängig ist.

29. Verfahren nach einem der obigen Ansprüche, wobei es sich bei dem Mikroorganismus um einen grampositiven Mikroorganismus handelt.

30. Verfahren nach einem der obigen Ansprüche, wobei der Mikroorganismus zur Gattung Bacillus gehört.

31. Verfahren nach einem der obigen Ansprüche, wobei es sich bei dem Mikroorganismus um Bacillus subtilis handelt.

32. Rekombinanter Mikroorganismus zur verglichen mit der Pantothenatproduktion in dem Mikroorganismus vor der Deregulation verbesserten Produktion von Pantothenat, mit einem deregulierten Pantothenat-Biosyntheseweg und einem deregulierten Methylen-tetrahydrofolat-(MTF-)Biosyntheseweg, wobei der Mikroorganismus ein dereguliertes Serin-Hydroxymethyltransferase-Gen und ein mutiertes, deletiertes oder unterbrochenes Purinrepressor-Gen aufweist.

33. Rekombinanter Mikroorganismus zur verglichen mit der Pantothenatproduktion in dem Mikroorganismus vor der Deregulation verbesserten Produktion von Pantothenat, mit einem deregulierten Pantothenat-Biosyntheseweg, einem deregulierten Methylen-tetrahydrofolat-(MTF-)Biosyntheseweg und einem deregulierten Isoleucin-Valin-(ilv-)Biosyntheseweg, wobei der Mikroorganismus ein dereguliertes Serin-Hydroxymethyltransferase-Gen und ein mutiertes, deletiertes oder unterbrochenes Purinrepressor-Gen aufweist.

34. Mikroorganismus nach Anspruch 32 oder 33, ferner mit reduzierter Pantothenatkinase-Aktivität.

35. Mikroorganismus nach einem der Ansprüche 32-34, bei dem es sich um einen grampositiven Mikroorganismus handelt.

36. Mikroorganismus nach einem der Ansprüche 32-35, zur Gattung Bacillus gehörend.

37. Mikroorganismus nach einem der Ansprüche 32-36,
bei dem es sich um Bacillus subtilis handelt.

38. Verfahren zur Produktion von Pantothenat, bei dem man einen rekombinanten Mikroorganismus mit: (a) einem deregulierten panB-Gen; (b) einem deregulierten panD-Gen; und (c) wenigstens einem deregulierten, ein Enzym der Isoleucin-Valin-(ilv-)Biosynthese codierenden Gen; unter solchen Bedingungen kultiviert, dass nach 36 Stunden Kultivierung des Mikroorganismus wenigstens 30 g/l Pantothenat produziert werden, wobei der Mikroorganismus ein dereguliertes Serin-Hydroxymethyltransferase-Gen und ein mutiertes, deletiertes oder unterbrochenes Purinrepressor-Gen aufweist.

39. Verfahren nach Anspruch 38, wobei der Mikroorganismus ferner einen deregulierten Methylen-tetrahydrofolat-(MTF-)Biosyntheseweg aufweist und der Mikroorganismus unter solchen Bedingungen kultiviert wird, dass nach 36 Stunden Kultivierung des Mikroorganismus wenigstens 50 g/l Pantothenat produziert werden.

40. Verfahren zur Produktion von Pantothenat, bei dem man einen rekombinanten Mikroorganismus mit: (a) einem deregulierten panB-Gen; und (b) einem deregulierten panD-Gen; unter Serinüberschuss-Bedingungen kultiviert, so dass nach 36 Stunden Kultivierung des Mikroorganismus wenigstens 50 g/l Pantothenat produziert werden, wobei der Mikroorganismus ein dereguliertes Serin-Hydroxymethyltransferase-Gen und ein mutiertes, deletiertes oder unterbrochenes Purinrepressor-Gen aufweist.

41. Verfahren zur Produktion von Pantothenat, bei dem man einen rekombinanten Mikroorganismus mit: (a) einem deregulierten panB-Gen; (b) einem deregulierten panD-Gen; und (c) einem deregulierten Methylentetrahydrofolat-(MTF-)Biosyntheseweg; unter Valinüberschuss-Bedingungen kultiviert, so dass nach 36 Stunden Kultivierung des Mikroorganismus wenigstens 50 g/l Pantothenat produziert werden, wobei der Mikroorganismus ein dereguliertes Serin-Hydroxymethyltransferase-Gen und ein mutiertes, deletiertes oder unterbrochenes Purinrepressor-Gen aufweist.

42. Verfahren zur Produktion von Pantothenat, bei dem man einen rekombinanten Mikroorganismus mit: (a) einem deregulierten panB-Gen; (b) einem deregulierten panD-Gen; und (c) einem deregulierten Serin-Hydroxymethyltransferase-Gen, wobei der Mikroorganismus ein mutiertes, deletiertes oder unterbrochenes Purinrepressor-Gen aufweist; unter Valinüberschuss-Bedingungen kultiviert, so dass nach 36 Stunden Kultivierung des Mikroorganismus wenigstens 50 g/l Pantothenat produziert werden.

43. Verfahren zur Produktion von Pantothenat, bei dem man einen rekombinanten Mikroorganismus mit: (a) einem deregulierten panB-Gen; (b) einem deregulierten panD-Gen; und (c) einem mutierten, deletierten oder unterbrochenen Purinrepressor-Gen; unter Valinüberschuss-Bedingungen kultiviert, so dass nach 36 Stunden Kultivierung des Mikroorganismus wenigstens 50 g/l Pantothenat produziert werden.

44. Verfahren zur Produktion von Pantothenat, bei dem man einen rekombinanten Mikroorganismus mit: (a) einem deregulierten panB-Gen; (b) einem deregulierten panD-Gen; und (c) einem deregulierten serA-Gen; unter Valinüberschuss-Bedingungen kultiviert, so dass nach 36 Stunden Kultivierung des Mikroorganismus wenigstens 50 g/l Pantothenat produziert werden,
wobei der Mikroorganismus ein dereguliertes Serin-Hydroxymethyltransferase-Gen und ein mutiertes, deletiertes oder unterbrochenes Purinrepressor-Gen aufweist.

45. Verfahren zur Produktion von Pantothenat, bei dem man einen rekombinanten Mikroorganismus mit: (a) einem deregulierten panB-Gen; (b) einem deregulierten panD-Gen; (c) einem deregulierten serA-Gen; (d) einem deregulierten Serin-Hydroxymethyltransferase-Gen; kultiviert, und zwar unter Valinüberschuss-Bedingungen, so dass nach 36 Stunden Kultivierung des Mikroorganismus wenigstens 50 g/l Pantothenat produziert werden, wobei der Mikroorganismus ein mutiertes, deletiertes oder unterbrochenes Purinrepressor-Gen aufweist.

## Revendications

1. Procédé pour la production de pantothénate renforcée par comparaison avec une production de pantothénate dans ledit micro-organisme avant la dérégulation, comprenant la culture d'un micro-organisme ayant une voie dérégulée de biosynthèse du méthylènetétrahydrofolate (MTF), dans des conditions dans lesquelles la production du pantothénate soit renforcée, le micro-organisme ayant un gène dérégulé de la sérine hydroxyméthyltransférase et un gène répresseur de purine muté, délété ou rompu, modulant la répression du gène de la sérine hydroxyméthyltransférase.

2. Procédé pour la production de pantothénate renforcée par comparaison avec la production de pantothénate dans ledit micro-organisme avant la dérégulation, comprenant la culture d'un micro-organisme ayant (i) une voie dérégulée de biosynthèse du pantothénate, et (ii) une voie dérégulée de biosynthèse du méthylènetétrahydrofolate (MTF), dans des conditions telles que la production de pantothénate soit renforcée par comparaison avec une production de pantothénate dans ledit micro-organisme avant la dérégulation, le micro-organisme ayant un gène dérégulé de la sérine hydroxyméthyltransférase et un gène répresseur de la purine, muté, délété ou rompu, modulant la répression du gène de la sérine hydroxyméthyltransférase.

3. Procédé de la revendication 2, dans lequel ledit micro-organisme a au moins deux enzymes de biosynthèse du pantothénate qui sont dérégulées.

4. Procédé de la revendication 2, dans lequel ledit micro-organisme a au moins trois enzymes de biosynthèse du pantothénate qui sont dérégulées.

5. Procédé de la revendication 2, dans lequel ledit micro-organisme a au moins quatre enzymes de biosynthèse du pantothénate qui sont dérégulées.

6. Procédé de la revendication 5, dans lequel ledit micro-organisme a une cétopantoate hydroxyméthyltransférase dérégulée, une cétopantoate réductase dérégulée, une pantothénate synthétase dérégulée et une aspartate-alpha-décarboxylase dérégulée.

7. Procédé de l'une quelconque des revendications 1 à 6, dans lequel ledit micro-organisme a en outre une voie dérégulée de biosynthèse de l'isoleucine-valine (ilv).

8. Procédé de la revendication 7, dans lequel ledit micro-organisme a au moins deux enzymes de biosynthèse de l'isoleucine-valine (ilv) qui sont dérégulées.

9. Procédé de la revendication 7, dans lequel ledit micro-organisme a au moins trois enzymes de biosynthèse de l'isoleucine-valine (ilv) qui sont dérégulées.

10. Procédé de la revendication 9, dans lequel ledit micro-organisme a une acétohydroxyacide synthétase dérégulée, une acétohydroxyacide isoméroréductase dérégulée et une dihydroxyacide déshydratase dérégulée.

11. Procédé de l'une quelconque des revendications 1 à 10, dans lequel le micro-organisme a au moins une enzyme de biosynthèse du MTF qui est dérégulée.

12. Procédé de la revendication 11, dans lequel le micro-organisme a un gène serA dérégulé.

13. Procédé de la revendication 11, dans lequel le micro-organisme a un gène dérégulé de la sérine hydroxyméthyltransférase et un gène serA dérégulé.

14. Procédé de l'une quelconque des revendications 1 à 13 pour la production de pantothénate renforcée par comparaison avec la production de pantothénate dans ledit micro-organisme avant la dérégulation, comprenant la culture d'un micro-organisme ayant une voie dérégulée de biosynthèse du pantothénate, une voie dérégulée de biosynthèse de l'isoleucine-valine (ilv) et une voie dérégulée de biosynthèse du méthylènetétrahydrofolate (MTF), de telle sorte que la production du pantothénate soit renforcée par comparaison avec la production du pantothénate dans ledit micro-organisme avant la dérégulation.

15. Procédé pour la production de pantothénate de l'une quelconque des revendications 1 à 14, comprenant la culture d'un micro-organisme ayant une voie dérégulée de biosynthèse du pantothénate, une voie dérégulée de biosynthèse de l'isoleucine-valine (ilv) et une voie dérégulée de biosynthèse du méthylènetétrahydrofolate (MTF), de telle sorte qu'au moins 50 g/l de pantothénate soient produits après 36 heures de culture du micro-organisme, le micro-organisme ayant un gène dérégulé de la sérine hydroxyméthyltransférase et un gène répresseur de purine muté, délété ou rompu.

16. Procédé de la revendication 15, comprenant la culture du micro-organisme de telle sorte qu'au moins 60 g/l de pantothénate soient produits après 36 heures de culture du micro-organisme.

17. Procédé de la revendication 15, comprenant la culture du micro-organisme de telle sorte qu'au moins 70 g/l de pantothénate soient produits après 36 heures de culture du micro-organisme.

18. Procédé pour la production de pantothénate de l'une quelconque des revendications 1 à 14, comprenant la culture d'un micro-organisme ayant une voie dérégulée de biosynthèse du pantothénate, une voie dérégulée de biosynthèse de l'isoleucine-valine (ilv) et une voie dérégulée de biosynthèse du méthylènetétrahydrofolate (MTF), de telle sorte qu'au moins 60 g/l de pantothénate soient produits après 48 heures de culture du micro-organisme, le micro-organisme ayant un gène dérégulé de la sérine hydroxyméthyltransférase et un gène répresseur de purine muté, délété ou rompu.

19. Procédé de la revendication 18, comprenant la culture du micro-organisme de telle sorte qu'au moins 70 g/l de pantothénate soient produits après 48 heures de culture du micro-organisme.

20. Procédé de la revendication 18, comprenant la culture du micro-organisme de telle sorte qu'au moins 80 g/l de pantothénate soient produits après 48 heures de culture du micro-organisme.

21. Procédé de l'une quelconque des revendications précédentes, dans lequel la production du pantothénate est encore renforcée par régulation de l'activité de pantothénate kinase.

22. Procédé de la revendication 21, dans lequel on a une diminution de l'activité de pantothénate kinase.

23. Procédé de la revendication 22, dans lequel le CoaA est délété et le CoaX est régulé à la baisse.

24. Procédé de la revendication 22, dans lequel le CoaX est délété et le CoaA est régulé à la baisse.

25. Procédé de la revendication 22, dans lequel le CoaX et le CoaA sont régulés à la baisse.

26. Procédé de l'une quelconque des revendications ci-dessus, dans lequel ledit micro-organisme est cultivé dans les conditions correspondant à un excès de sérine.

27. Procédé pour la production de pantothénate, comprenant la culture d'un micro-organisme ayant une voie dérégulée de biosynthèse du pantothénate dans des conditions correspondant à un excès de sérine, de telle sorte qu'il y ait production de pantothénate, le micro-organisme ayant un gène dérégulé de la sérine hydroxyméthyltransférase et un gène répresseur de purine muté, délété ou rompu.

28. Procédé de l'une quelconque des revendications ci-dessus, dans lequel ledit micro-organisme a sa voie de biosynthèse du pantothénate dérégulée, de telle sorte que la production de pantothénate soit indépendante de la bêta-alanine de charge.

29. Procédé de l'une quelconque des revendications ci-dessus, dans lequel le micro-organisme est un micro-organisme Gram-positif.

30. Procédé de l'une quelconque des revendications ci-dessus, dans lequel le micro-organisme appartient au genre Bacillus.

31. Procédé de l'une quelconque des revendications ci-dessus, dans lequel le micro-organisme est Bacillus subtilis.

32. Micro-organisme recombinant pour la production de pantothénate renforcée par comparaison avec la production de pantothénate dans ledit micro-organisme avant dérégulation, ledit micro-organisme ayant une voie dérégulée de biosynthèse du pantothénate et une voie dérégulée de biosynthèse du méthylènetétrahydrofolate (MTF), le micro-organisme ayant un gène dérégulé de la sérine hydroxyméthyltransférase et un gène répresseur de purine muté, délété ou rompu.

33. Micro-organisme recombinant pour la production renforcée de pantothénate par comparaison avec la production de pantothénate dans ledit micro-organisme avant dérégulation, ledit micro-organisme ayant une voie dérégulée de biosynthèse du pantothénate, une voie dérégulée de biosynthèse du méthylènetétrahydrofolate (MTF) et une voie dérégulée de biosynthèse de l'isoleucine-valine (ilv), le micro-organisme ayant un gène dérégulé de la sérine hydroxyméthyltransférase et un gène répresseur de purine muté, délété ou rompu.

34. Micro-organisme de la revendication 32 ou 33, ayant en outre une activité réduite de pantothénate kinase.

35. Micro-organisme de l'une quelconque des revendications 32 à 34, qui est un micro-organisme Gram-positif.

36. Micro-organisme de l'une quelconque des revendications 32 à 35, appartenant au genre Bacillus.

37. Micro-organisme de l'une quelconque des revendications 32 à 36, qui est Bacillus subtilis.

38. Procédé de production de pantothénate, comprenant la culture d'un micro-organisme recombinant ayant : (a) un gène panB dérégulé ; (b) un gène panD dérégulé ; et (c) au moins un gène dérégulé codant pour une enzyme de biosynthèse de l'isoleucine-valine (ilv), dans des conditions telles qu'au moins 30 g/l de pantothénate soient produits après 36 heures de culture du micro-organisme, le micro-organisme ayant un gène dérégulé de la sérine hydroxyméthyltransférase et un gène répresseur de purine muté, délété ou rompu.

39. Procédé de la revendication 38, dans lequel ledit micro-organisme a en outre une voie dérégulée de biosynthèse du méthylènetétrahydrofolate (MTF), et ledit micro-organisme est cultivé dans des conditions telles qu'au moins 50 g/l de pantothénate soient produits après 36 heures de culture du micro-organisme.

40. Procédé de production de pantothénate, comprenant la culture d'un micro-organisme recombinant ayant : (a) un gène panB dérégulé ; et (b) un gène panD dérégulé ; dans des conditions correspondant à un excès de sérine, de telle sorte qu'au moins 50 g/l de pantothénate soient produits après 36 heures de culture du micro-organisme, le micro-organisme ayant un gène dérégulé de la sérine hydroxyméthyltransférase et un gène répresseur de purine muté, délété ou rompu.

41. Procédé de production de pantothénate, comprenant la culture d'un micro-organisme recombinant ayant : (a) un gène panB dérégulé ; (b) un gène panD dérégulé ; et (c) une voie dérégulée de biosynthèse du méthylènetétrahydrofolate (MTF) ; dans des conditions correspondant à un excès de valine, de telle sorte qu'au moins 50 g/l de pantothénate soient produits après 36 heures de culture du micro-organisme, le micro-organisme ayant un gène dérégulé de la sérine hydroxyméthyltransférase et un gène répresseur de purine muté, délété ou rompu.

42. Procédé de production de pantothénate, comprenant la culture d'un micro-organisme recombinant ayant : (a) un gène panB dérégulé ; (b) un gène panD dérégulé ; et (c) un gène dérégulé de la sérine hydroxyméthyltransférase ; le micro-organisme ayant un gène répresseur de purine muté, délété ou rompu ; dans des conditions correspondant à un excès de valine, de telle sorte qu'au moins 50 g/l de pantothénate soient produits après 36 heures de culture du micro-organisme.

43. Procédé de production de pantothénate, comprenant la culture d'un micro-organisme recombinant ayant : (a) un gène panB dérégulé ; (b) un gène panD dérégulé ; et (c) un gène répresseur de purine muté, délété ou rompu ; dans des conditions correspondant à un excès de valine, de telle sorte qu'au moins 50 g/l de pantothénate soient produits après 36 heures de culture du micro-organisme.

44. Procédé de production de pantothénate, comprenant la culture d'un micro-organisme recombinant ayant : (a) un gène panB dérégulé ; (b) un gène panD dérégulé ; et (c) un gène serA dérégulé ; dans des conditions correspondant à un excès de valine, de telle sorte qu'au moins 50 g/l de pantothénate soient produits après 36 heures de culture du micro-organisme, le micro-organisme ayant un gène dérégulé de la sérine hydroxyméthyltransférase et un gène répresseur de purine muté, délété ou rompu.

45. Procédé de production de pantothénate, comprenant la culture d'un micro-organisme recombinant ayant : (a) un gène panB déréglé ; (b) un gène panD dérégulé ; (c) un gène serA dérégulé ; (d) un gène dérégulé de la sérine hydroxyméthyltransférase ; et dans des conditions correspondant à un excès de valine, de telle sorte qu'au moins 50 g/l de pantothénate soient produits après 36 heures de culture du micro-organisme, le micro-organisme ayant un gène répresseur de purine muté, délété ou rompu.
